# EUROPEAN PATENT SPECIFICATION

(11) **EP 3 906 233 B1**
(45) Date of publication and mention of the grant of the patent: **31.01.2024**
(21) Application number: 19907044.2
(22) Date of filing: 31.12.2019
(51) Int. Cl.: C07D 401/04, C07D 403/02, C07D 403/14, A61K 31/444, A61P 25/00

(54) **KINASE INHIBITOR COMPOUNDS AND COMPOSITIONS AND METHODS OF USE**
KINASEINHIBITORVERBINDUNGEN UND ZUSAMMENSETZUNGEN UND VERFAHREN ZUR VERWENDUNG
COMPOSÉS INHIBITEURS DE KINASE, COMPOSITIONS ET PROCÉDÉS D'UTILISATION

(30) Priority: 31.12.2018 US 201862786966 P
(43) Date of publication of application: 10.11.2021
(73) Proprietor: Icahn School of Medicine at Mount Sinai, New York, NY 10029 (US)
(72) Inventor: DEVITA, Robert, J., New York, NY 10029 (US); STEWART, Andrew, F., New York, NY 10029 (US); SUEBSUWONG, Chalada, New York, NY 10029 (US); KUMAR, Kunal, New York, NY 10029 (US); WANG, Peng, New York, NY 10029 (US); SANCHEZ, Roberto, J., New York, NY 10029 (US); WANG, Hui, New York, NY 10029 (US)
(74) Representative: Adamson Jones
(86) International application number: PCT/US2019/069057
(87) International publication number: WO 2020/142485

(56) References cited:
- WO-A1-2014/203217
- WO-A1-2017/106630
- US-A1- 2007 060 619
- US-A1- 2007 208 053
- US-A1- 2009 196 912
- US-A1- 2010 197 562
- US-A1- 2012 071 512
- US-A1- 2015 266 878
- US-A1- 2015 297 573
- US-A1- 2017 280 720
- US-A1- 2018 216 076
- US-B2- 9 951 050
- COOMBS THOMAS C ET AL: "Small-molecule pyrimidine inhibitors of the cdc2-like (Clk) and dual specificity tyrosine phosphorylation-regulated (Dyrk) kinases: Development of chemical probe ML315", BIOORGANIC & MEDICINAL CHEMISTRY LETTERS, ELSEVIER, AMSTERDAM NL, vol. 23, no. 12, 30 March 2013 (2013-03-30), pages 3654-3661, XP028543373, ISSN: 0960-894X, DOI: 10.1016/J.BMCL.2013.02.096
- PU CHUNLAN ET AL: "Design, synthesis and biological evaluation of indole derivatives as Vif inhibitors", BIOORGANIC & MEDICINAL CHEMISTRY LETTERS, vol. 27, no. 17, 1 September 2017 (2017-09-01), pages 4150-4155, XP055948998, Amsterdam NL ISSN: 0960-894X, DOI: 10.1016/j.bmcl.2017.07.026
- KUMAR, K et al.: "Novel Selective Thiadiazine DYRK1A Inhibitor Lead Scaffold with Human Pancreatic beta- cell Proliferation Activity", European Journal of Medical Chemistry, vol. 157, 5 September 2018 (2018-09-05), pages 1005-1016, XP085491737, DOI: 10.1016/j.ejmech.2018.08.007
- MULTHOFF, G et al.: "Chronic inflammation in cancer development", Frontiers in Immunology, vol. 2, 12 January 2012 (2012-01-12), pages 1-17, XP055710204, DOI: 10.3389/fimmu.2011.00098

## Description

This application claims the priority benefit of U.S. Provisional Patent Application Serial No. 62/786,966, filed December 31, 2018.

This invention was made with government support under grant number DK015015 and DK116904 awarded by the National Institutes of Health. The government has certain rights in the invention.

### FIELD OF THE INVENTION

The present invention relates to kinase inhibitor compounds and compositions and methods of use thereof.

### BACKGROUND OF THE INVENTION

The Dual-Specificity Tyrosine-Regulated kinases ("DYRKs") belong to the CMCG family of eukaryotic protein kinases which include the CDK-like kinases (CLKs), Glycogen Synthase Kinase 3 (GSK3), Cyclin Dependent Kinases (CDKs), and Mitogen-Activated Protein Kinases (MAPKs). DYRK family proteins self-activate by autophosphorylation of the conserved tyrosine residue in the activation loop, then subsequently phosphorylate substrates only on serine and threonine residues (Lochhead et al., "Activation-Loop Autophosphorylation is Mediated by a Novel Transitional Intermediate Form of DYRKs," Cell 121(6):925-936 (2005); Walte et al., "Mechanism of Dual Specificity Kinase Activity of DYRK1A," FEBS J. 280(18):4495-4511 (2013); and Becker et al., "Activation, Regulation, and Inhibition of DYRK1A," FEBS J. 278(2):246-256 (2011)). The DYRK family consists of five subtypes, including 1A, 1B, 2, 3 and 4. Among them, DYRK1A is the most extensively studied subtype. It is ubiquitously expressed and has been shown to play an important role in brain development and function (Becker et al., "DYRK1A: A Potential Drug Target for Multiple Down Syndrome Neuropathologies," CNS Neurol. Disord.: Drug Targets 13(1):26-33 (2014)), neurodegenerative diseases (Wegiel et al., "The Role of DYRK1A in Neurodegenerative Diseases," FEBS J. 278(2):236-245 (2011) and Smith et al., "Recent Advances in the Design, Synthesis, and Biological Evaluation of Selective DYRK1A Inhibitors: A New Avenue for a Disease Modifying Treatment of Alzheimer's?," ACS Chem. Neurosci. 3(11):857-872 (2012)), tumorigenesis, apoptosis (Ionescu et al., "DYRK1A Kinase Inhibitors With Emphasis on Cancer," Mini-Rev. Med. Chem. 12(13):1315-1329 (2012) and Fernandez-Martinez et al., "DYRK1A: The Double-Edged Kinase as a Protagonist in Cell Growth and Tumorigenesis," Mol. Cell. Oncol. 2(1):e970048 (2015)), and human pancreatic β-cell proliferation (Wang et al., "A High-Throughput Chemical Screen Reveals That Harmine-Mediated Inhibition of DYRK1A Increases Human Pancreatic Beta Cell Replication," Nat. Med. 21(4):383-388 (2015); Shen et al., "Inhibition of DYRK1A and GSK3B Induces Human β-cell Proliferation," Nat. Commun. 6:8372 (2015); Rachdi et al., "Dyrk1A Induces Pancreatic β Cell Mass Expansion and Improves Glucose Tolerance," Cell Cycle 13(14):2221-2229 (2014); and Dirice et al., "Inhibition of DYRK1A Stimulates Human Beta-Cell Proliferation," Diabetes 65:(6):1660-1671 (2016)).

Regulated expression of DYRK1A during fetal, postnatal life, as well as in adults, is essential for normal neuronal development and brain function. DYRK1A is located in the Down Syndrome Critical region ("DSCR") on human chromosome 21, a genomic region that has an important role in pathogenesis of Down Syndrome ("DS"), one of the most common and frequent human genetic disorders (Becker et al., "Activation, Regulation, and Inhibition of DYRK1A," FEBS J. 278(2):246-256 (2011) and Becker et al., "Structural and Functional Characteristics of Dyrk, a Novel Subfamily of Protein Kinases With Dual Specificity," Prog. Nucleic Acid Res. Mol. Biol. 62:1-17 (1999)). Overexpression of DYRK1A in mouse and drosophila models mimics the neurodevelopmental abnormalities associated with DS (Becker et al., "DYRK1A: A Potential Drug Target for Multiple Down Syndrome Neuropathologies," CNS Neurol. Disord.: Drug Targets 13(1):26-33 (2014); Wegiel et al., "The Role of DYRK1A in Neurodegenerative Diseases," FEBS J. 278(2):236-245 (2011); Park et al., "Function and Regulation of Dyrk1A: Towards Understanding Down Syndrome," Cell. Mol. Life Sci. 66(20):3235-3240 (2009); and Ogawa et al., "Development of a Novel Selective Inhibitor of the Down Syndrome-Related Kinase Dyrk1A," Nat. Commun. 1: Article Number 86 (2010)). Recent evidences has also implicated DYRK1A in the *tau* dysfunction and *tau* pathology of Alzheimer's disease ("AD"), dementia with Lewy bodies, and Parkinson's disease (Wegiel et al., "The Role of DYRK1A in Neurodegenerative Diseases," FEBS J. 278(2):236-245 (2011); Smith et al., "Recent Advances in the Design, Synthesis, and Biological Evaluation of Selective DYRK1A Inhibitors: A New Avenue for a Disease Modifying Treatment of Alzheimer's?," ACS Chem. Neurosci. 3(11):857-872 (2012); and Stotani et al., "DYRK1A Inhibition as Potential Treatment for Alzheimer's Disease," Future Med. Chem. 8(6):681-696 (2016)). It has been reported that DYRK1A is overexpressed in various tumors such as, ovarian cancer, colon cancer, lung cancer, and pancreatic cancer, signifying its role in tumorigenesis and uncontrolled cell proliferation (Ionescu et al., "DYRK1A Kinase Inhibitors With Emphasis on Cancer," Mini-Rev. Med. Chem. 12(13):1315-1329 (2012) and Fernandez-Martinez et al., "DYRK1A: The Double-Edged Kinase as a Protagonist in Cell Growth and Tumorigenesis," Mol. Cell. Oncol. 2(1):e970048 (2015)). Inhibition of DYRK1A leads to destabilized EGFR and reduced EGFR-dependent tumor growth in glioblastoma (Pozo et al., "Inhibition of DYRK1A Destabilizes EGFR and Reduces EGFR-Dependent Glioblastoma Growth," J. Clin. Invest. 123(6):2475-2487 (2013)). Also, DYRK1A inhibition induces activation of caspase-9 which leads to massive apoptosis in specific cancer cell types (Seifert et al., "DYRK1A Phosphorylates Caspase 9 at an Inhibitory Site and is Potently Inhibited in Human Cells by Harmine," FEBS J. 275(24):6268-6280 (2008)). Recently, DYRK1A has been shown to be involved in molecular pathways relevant to human β-cell proliferation, making it a potential therapeutic target for β-cell regeneration in Type 1 and Type 2 diabetes (Wang et al., "A High-throughput Chemical Screen Reveals That Harmine-Mediated Inhibition of DYRK1A Increases Human Pancreatic Beta Cell Replication," Nat. Med. 21(4):383-388 (2015); Shen et al., "Inhibition of DYRK1A and GSK3B Induces Human β-cell Proliferation," Nat. Commun. 6:8372 (2015); Rachdi et al., "Dyrk1A Induces Pancreatic β Cell Mass Expansion and Improves Glucose Tolerance," Cell Cycle 13(14):2221-2229 (2014); and Dirice et al., "Inhibition of DYRK1A Stimulates Human Beta-cell Proliferation," Diabetes 65:(6):1660-1671 (2016)). DYRK1A inhibition has been proposed to drive β-cell proliferation by inducing translocation of the nuclear factor of activated T cells ("NFAT") family of transcription factors to the nucleus, allowing access to the promoters of genes which subsequently activate human β-cell proliferation (Wang et al., "A High-throughput Chemical Screen Reveals That Harmine-Mediated Inhibition of DYRK1A Increases Human Pancreatic Beta Cell Replication," Nat. Med. 21(4):383-388 (2015) and Rachdi et al., "Dyrk1A Induces Pancreatic β Cell Mass Expansion and Improves Glucose Tolerance," Cell Cycle 13(14):2221-2229 (2014)).

Because of its involvement in neurodegenerative disease, cancer, and diabetes, DYRK1A has attracted increasing interest as a potential therapeutic target. A significant amount of work has been carried out to not only understand its underlying role in diseases, but also in identifying novel DYRK1A inhibitors (Becker et al., "Activation, Regulation, and Inhibition of DYRK1A," FEBS J. 278(2):246-256 (2011); Becker et al., "DYRK1A: A Potential Drug Target for Multiple Down Syndrome Neuropathologies," CNS Neurol. Disord.: Drug Targets 13(1):26-33 (2014); Wegiel et al., "The Role of DYRK1A in Neurodegenerative Diseases," FEBS J. 278(2):236-245 (2011); Smith et al., "Recent Advances in the Design, Synthesis, and Biological Evaluation of Selective DYRK1A Inhibitors: A New Avenue for a Disease Modifying Treatment of Alzheimer's?," ACS Chem. Neurosci. 3(11):857-872 (2012); Ionescu et al., "DYRK1A Kinase Inhibitors with Emphasis on Cancer," Mini-Rev. Med. Chem. 12(13):1315-1329 (2012); Fernandez-Martinez et al., "DYRK1A: The Double-Edged Kinase as a Protagonist in Cell Growth and Tumorigenesis," Mol. Cell. Oncol. 2(1):e970048 (2015); Wang et al., "A High-throughput Chemical Screen Reveals That Harmine-Mediated Inhibition of DYRK1A Increases Human Pancreatic Beta Cell Replication," Nat. Med. 21(4):383-388 (2015); Shen et al., "Inhibition of DYRK1A and GSK3B Induces Human β-cell Proliferation," Nat. Commun. 6:8372 (2015); and Dirice et al., "Inhibition of DYRK1A Stimulates Human Beta-cell Proliferation," Diabetes 65:(6):1660-1671 (2016)).

Several DYRK1A inhibitors from natural sources as well as small molecule drug discovery programs have been identified and characterized. Among all the DYRK1A inhibitors, harmine and its analogues (β-carbolines) are the most commonly studied and remain the most potent and orally bioavailable class of inhibitors covered to date (Becker et al., "Activation, Regulation, and Inhibition of DYRK1A," FEBS J. 278(2):246-256 (2011) and Smith et al., "Recent Advances in the Design, Synthesis, and Biological Evaluation of Selective DYRK1A Inhibitors: A New Avenue for a Disease Modifying Treatment of Alzheimer's?," ACS Chem. Neurosci. 3(11):857-872 (2012)).

Apart from harmine, EGCg and other flavan-3-ols (Guedj et al., "Green Tea Polyphenols Rescue of Brain Defects Induced by Overexpression of DYRK1A," PLoS One 4(2):e4606 (2009) and Bain et al., "The Specificities of Protein Kinase Inhibitors: An Update," Biochem. J. 371(1):199-204 (2003)), leucettines (Tahtouh et al., "Selectivity, Cocrystal Structures, and Neuroprotective Properties of Leucettines, a Family of Protein Kinase Inhibitors Derived from the Marine Sponge Alkaloid Leucettamine B," J. Med. Chem. 55(21):9312-9330 (2012) and Naert et al., "Leucettine L41, a DYRK1A-preferential DYRKs/CLKs Inhibitor, Prevents Memory Impairments and Neurotoxicity Induced by Oligomeric Aβ25-35 Peptide Administration in Mice," Eur. Neuropsychopharmacol. 25(11):2170-2182 (2015)), quinalizarine (Cozza et al., "Quinalizarin as a Potent, Selective and Cell-permeable Inhibitor of Protein Kinase CK2," Biochem. J. 421(3):387-395 (2009)), peltogynoids Acanilol A and B (Ahmadu et al, "Two New Peltogynoids from Acacia nilotica Delile with Kinase Inhibitory Activity," Planta Med. 76(5):458-460 (2010)), benzocoumarins (dNBC) (Sarno et al., "Structural Features Underlying the Selectivity of the Kinase Inhibitors NBC and dNBC: Role of a Nitro Group that Discriminates Between CK2 and DYRK1A," Cell. Mol. Life Sci. 69(3):449-460 (2012)), and indolocarbazoles (Starosporine, rebeccamycin and their analogues) (Sanchez et al., "Generation of Potent and Selective Kinase Inhibitors by Combinatorial Biosynthesis of Glycosylated Indolocarbazoles," Chem. Commun. 27:4118-4120 (2009), are other natural products that have been shown to inhibit DYRK1A and other kinases.

Among the other scaffolds identified from small molecule drug discovery attempts, INDY (Ogawa et al., "Development of a Novel Selective Inhibitor of the Down Syndrome-Related Kinase Dyrk1A," Nat. Commun. 1: Article Number 86 (2010)), DANDY (Gourdain et al., "Development of DANDYs, New 3,5-Diaryl-7-Azaindoles Demonstrating Potent DYRK1A Kinase Inhibitory Activity," J. Med. Chem. 56(23):9569-9585 (2013)), and FINDY (Kii et al., "Selective Inhibition of the Kinase DYRK1A by Targeting its Folding Process," Nat. Commun. 7:11391 (2016)), pyrazolidine-diones (Koo et al., "QSAR Analysis of Pyrazolidine-3,5-Diones Derivatives as DyrklA Inhibitors," Bioorg. Med. Chem. Lett. 19(8):2324-2328 (2009); Kim et al., "Putative Therapeutic Agents for the Learning and Memory Deficits of People with Down Syndrome," Bioorg. Med. Chem. Lett. 16(14):3772-3776 (2006)), amino-quinazolines (Rosenthal et al., "Potent and Selective Small Molecule Inhibitors of Specific Isoforms of Cdc2-Like Kinases (Clk) and Dual Specificity Tyrosine-Phosphorylation-Regulated Kinases (Dyrk)," Bioorg. Med. Chem. Lett. 21(10):3152-3158 (2011)), meriolins (Giraud et al., "Synthesis, Protein Kinase Inhibitory Potencies, and In Vitro Antiproliferative Activities of Meridianin Derivatives," J. Med. Chem. 54(13):4474-4489 (2011); Echalier et al., "Meriolins (3-(Pyrimidin-4-yl)-7-Azaindoles): Synthesis, Kinase Inhibitory Activity, Cellular Effects, and Structure of a CDK2/Cyclin A/Meriolin Complex," J. Med. Chem. 51(4):737-751 (2008); and Akue-Gedu et al., "Synthesis and Biological Activities of Aminopyrimidyl-Indoles Structurally Related to Meridianins," Bioorg. Med. Chem. 17(13):4420-4424 (2009)), pyridine and pyrazines (Kassis et al., "Synthesis and Biological Evaluation of New 3-(6-hydroxyindol-2-yl)-5-(Phenyl) Pyridine or Pyrazine V-Shaped Molecules as Kinase Inhibitors and Cytotoxic Agents," Eur. J. Med. Chem. 46(11):5416-5434 (2011)), chromenoidoles (Neagoie et al., "Synthesis of Chromeno[3,4-b]indoles as Lamellarin D Analogues: A Novel DYRK1A Inhibitor Class," Eur. J. Med. Chem. 49:379-396 (2012)), 11H-indolo[3,2-c]quinoline-6-carboxylic acids,37 thiazolo[5,4-f]quinazolines (EHT 5372) (Foucourt et al., "Design and Synthesis of Thiazolo[5,4-f]quinazolines as DYRK1A Inhibitors, Part I.," Molecules 19(10):15546-15571 (2014) and Coutadeur et al., "A Novel DYRK1A (Dual Specificity Tyrosine Phosphorylation-Regulated Kinase 1A) Inhibitor for the Treatment of Alzheimer's Disease: Effect on Tau and Amyloid Pathologies In Vitro," J. Neurochem. 133(3):440-451 (2015)), and 5-iodotubercidin (Dirice et al., "Inhibition of DYRK1A Stimulates Human Beta-cell Proliferation," Diabetes 65:(6):1660-1671 (2016) and Annes et al., "Adenosine Kinase Inhibition Selectively Promotes Rodent and Porcine Islet β-cell Replication," Proc. Natl. Acad. Sci. 109(10):3915-3920 (2012)) showed potent DYRK1A activity with varying degrees of kinase selectivity.

Most of these compounds are non-selective inhibitors of DYRK1A and exhibit pharmacological side effects, such as CNS activity or apoptosis, thereby limiting their therapeutic utility and potential for pharmaceutical development. This non-selectivity may be attributed to the fact that all these DYRK1A inhibitors are Type I kinase inhibitors, which bind to a highly conserved ATP binding pocket.

The present invention is directed to overcoming deficiencies in the art.

### SUMMARY OF THE INVENTION

One aspect of the present invention relates to a compound of formula (I) having the following structure: 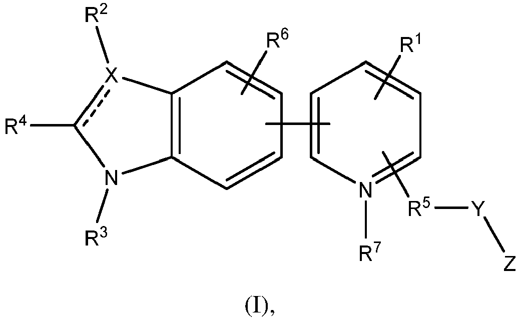 or a stereoisomer, pharmaceutically acceptable salt, oxide, or solvate thereof, wherein
R¹ and R⁶ are independently optionally present, and when present, each is independently a substituted or unsubstituted C₁-C₆ alkyl, halogen, -CF₃, or -OCF₃;
R² is H;
R³ is H;
R⁴ an oxygen that forms a carbonyl;
R⁵ is NH;
R⁷ is optionally present, and when present is an oxygen forming a pyridine N-oxide;
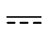 is a single bond;
Xis N;
Y is selected from a bond, CH₂, CH(CH₃), CH₂CH₂, CH₂CH(CH₃), and CH(CH₃)CH₂; and
Z is an unsubstituted phenyl ring or a phenyl substituted with a hydroxyl, - OCF₃, halogen, nitrile, a benzene ring, C₁-C₆ alkoxy, or -CONH₂.

Also disclosed is a method of inhibiting activity of a kinase in a cell. This method involves contacting the cell with a compound of formula (I) of the present invention under conditions effective to inhibit activity of the kinase in the cell.

Also disclosed is a method of increasing cell proliferation in a population of pancreatic beta cells. This method involves contacting a population of pancreatic beta cells with a compound of formula (I) according to the present invention under conditions effective to increase cell proliferation in the population of pancreatic beta cells.

Another aspect of the present invention relates to a composition comprising a compound of formula (I) according to the present invention and a carrier.

An additional aspect of the present invention relates to a compound of the invention for use in the treatment of a condition associated with insufficient insulin secretion. This involves administering to a subject in need of treatment for a condition associated with an insufficient level of insulin secretion a compound or composition of the present invention.

A further aspect of the present invention relates to a compound of the invention for use in the treatment of a neurological disorder. This involves administering to a subject in need of treatment for a neurological disorder a compound of formula (I) according to the present invention under conditions effective to treat the subject for the condition.

Although efforts have been made toward the discovery of potent and selective DYRK1A inhibitors, most of them are still in early stages of lead identification.

Described herein *infra* is the identification and evaluation of a highly potent and novel class of kinase inhibitor compounds.

### BRIEF DESCRIPTION OF THE DRAWINGS

Figure 1 is a schematic illustration showing the synthesis of N-benzyl-haloanilines intermediate compounds.
Figure 2 is a schematic illustration showing the synthesis of Indole-, Oxindole- and Benzimidazolone-boronic acid pinacol ester intermediate compounds.
Figure 3 is a schematic illustration showing the synthesis of Indole-, Benzimidazole- and Benzimidazolone benzyl heterocyclic amine compounds.
Figure 4 is a schematic illustration showing the synthesis of N-benzylpyridin-2-amine or N-(naphthalenylmethyl)pyridin-2-amine intermediate compounds.
Figure 5 is a schematic illustration showing the synthesis of Benzimidazolone benzyl or naphthyl heterocyclic amine compounds.
Figure 6 is a schematic illustration showing the synthesis of Benzimidazole pyridin-3-yl amino methylbenzamide compounds.
Figure 7 is a schematic illustration showing the synthesis of *N*-((5-Bromopyridin-3-yl)methyl)aniline and 5-Bromo-N-phenylnicotinamide compounds.
Figure 8 is a schematic illustration showing the synthesis of 5-(5-((Phenylamino)methyl)pyridin-3-yl)-1*H*-benzo[*d*]imidazol-2(3*H*)-one and 5-(2-Oxo-2,3-dihydro-1*H*-benzo[*d*]imidazol-5-yl)-*N*-phenylnicotinamide compounds.
Figure 9 is a schematic illustration showing the synthesis of 5-(5-(Benzylamino)pyridin-3-yl)-methyl-1*H*-benzo[*d*]imidazol-2(3*H*)-one compounds.
Figure 10 is a schematic illustration showing the synthesis of 5-(5-((3-Alkoxybenzyl)amino)pyridin-3-yl)-1*H*-benzo[*d*]imidazol-2(3*H*)-one compounds.
Figure 11 is a schematic illustration showing the synthesis of 5-(5-(((Heterocyclic)methyl)amino)pyridin-3-yl)-1*H*-benzo[*d*]imidazol-2(3*H*)-one compounds.
Figure 12 is a schematic illustration showing the synthesis of 5-(5-((Phenylamino)methyl)pyridin-3-yl)-1*H*-benzo[*d*]imidazol-2(3*H*)-one derivatives
Figure 13 is a schematic illustration showing the synthesis of substituted Benzo[*d*]imidazol-2(3*H*)-one pyridyl(benzylamine) compounds.
Figure 14 is a schematic illustration showing the synthesis of 5-(5-(1-Amino-2-phenylethyl)pyridin-3-yl)-1*H*-benzo[*d*]imidazol-2(3*H*)-one compounds.
Figure 15 is a schematic illustration showing the synthesis of 5-(5-((1-phenylethyl) amino)pyridin-3-y-yl)-1*H*-benzo[*d*]imidazol-2(3*H*)-one compounds.
Figure 16 is a schematic illustration showing the synthesis of 3-(Benzylamino)-5-(2-oxo-2,3-dihydro-1*H*-benzo[*d*]imidazol-5-yl)pyridine 1-oxide compounds.
Figure 17 is a schematic illustration showing the synthesis of Benzylamino pyridinyl-1*H*-benzo[*d*]imidazol-2(3*H*)-one compounds.

### DETAILED DESCRIPTION OF THE INVENTION

One aspect of the present invention relates to a compound of formula (I) having the following structure: 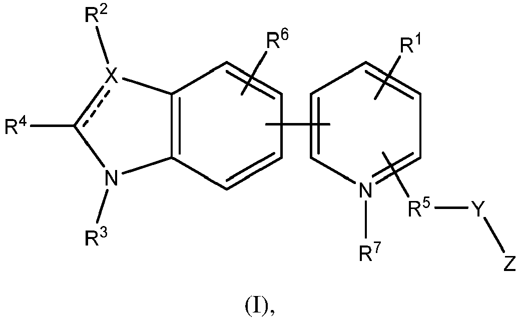 or a stereoisomer, pharmaceutically acceptable salt, oxide, or solvate thereof, wherein
R¹ and R⁶ are independently optionally present, and when present, each is independently a substituted or unsubstituted C₁-C₆ alkyl, halogen, -CF₃, or -OCF₃;
R² is H;
R³ is H;
R⁴ is an oxygen that forms a carbonyl;
R⁵ is NH;
R⁷ is optionally present, and when present is an oxygen forming a pyridine N-oxide;
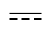 is a single bond;
Xis N;
Y is selected from a bond, CH₂, CH(CH₃), CH₂CH₂, CH₂CH(CH₃), and CH(CH₃)CH₂; and
Z is an unsubstituted phenyl ring or a phenyl ring substituted with a hydroxyl,-OCF₃, halogen, nitrile, a benzene ring, C₁-C₆ alkoxy, or -CO₂NH.

As used above, and throughout the description herein, the following terms, unless otherwise indicated, shall be understood to have the following meanings. If not defined otherwise herein, all technical and scientific terms used herein have the same meaning as is commonly understood by one of ordinary skill in the art to which this technology belongs.

As used herein, the term "halogen" means fluoro, chloro, bromo, or iodo.

The term "alkyl" means an aliphatic hydrocarbon group which may be straight or branched having about 1 to about 6 carbon atoms in the chain (or the number of carbons designated by "Cₙ₋Cₙ", where *n* is the numerical range of carbon atoms). Branched means that one or more lower alkyl groups such as methyl, ethyl, or propyl are attached to a linear alkyl chain. Exemplary alkyl groups include methyl, ethyl, n-propyl, i-propyl, n-butyl, t-butyl, n-pentyl, and 3-pentyl.

The term "alkoxy" means groups of from 1 to 6 carbon atoms of a straight, branched, or cyclic configuration and combinations thereof attached to the parent structure through an oxygen. Examples include methoxy, ethoxy, propoxy, isopropoxy, butoxy, cyclopropyloxy, cyclohexyloxy, and the like. Alkoxy also includes methylenedioxy and ethylenedioxy in which each oxygen atom is bonded to the atom, chain, or ring from which the methylenedioxy or ethylenedioxy group is pendant so as to form a ring. Thus, for example, phenyl substituted by alkoxy may be, for example,

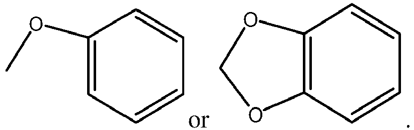

The term "cycloalkyl" means a non-aromatic, saturated or unsaturated, mono- or multi-cyclic ring system of about 3 to about 7 carbon atoms, or of about 5 to about 7 carbon atoms, and which may include at least one double bond. Exemplary cycloalkyl groups include, without limitation, cyclopropyl, cyclobutyl, cyclopentyl, cyclohexyl, cycloheptyl, cyclopropenyl, cyclobutenyl, cyclopentenyl, cyclophenyl, anti-bicyclopropane, and syn-tricyclopropane.

The term "aryl" means an aromatic monocyclic or multi-cyclic (polycyclic) ring system of 6 to about 19 carbon atoms, or of 6 to about 10 carbon atoms, and includes arylalkyl groups. The ring system of the aryl group may be optionally substituted. Representative aryl groups of the present invention include, but are not limited to, groups such as phenyl, naphthyl, azulenyl, phenanthrenyl, anthracenyl, fluorenyl, pyrenyl, triphenylenyl, chrysenyl, and naphthacenyl.

As used herein the term "biaryl" includes not only such traditional biaryl groups as biphenyl, but fused variants thereof, naphthyl-containing and heteroatom-containing variants thereof, and benzhydryl variants thereof.

The term "heteroaryl" means an aromatic monocyclic or multi-cyclic ring system of about 5 to about 19 ring atoms, or about 5 to about 10 ring atoms, in which one or more of the atoms in the ring system is/are element(s) other than carbon, for example, nitrogen, oxygen, or sulfur. In the case of multi-cyclic ring system, only one of the rings needs to be aromatic for the ring system to be defined as "heteroaryl." Preferred heteroaryls contain about 5 to 6 ring atoms. The prefix aza, oxa, thia, or thio before heteroaryl means that at least a nitrogen, oxygen, or sulfur atom, respectively, is present as a ring atom. A nitrogen, carbon, or sulfur atom in the heteroaryl ring may be optionally oxidized; the nitrogen may optionally be quaternized. Representative heteroaryls include pyridyl, 2-oxo-pyridinyl, pyrimidinyl, pyridazinyl, pyrazinyl, triazinyl, furanyl, pyrrolyl, thiophenyl, pyrazolyl, imidazolyl, oxazolyl, isoxazolyl, thiazolyl, isothiazolyl, triazolyl, oxadiazolyl, thiadiazolyl, tetrazolyl, indolyl, isoindolyl, benzofuranyl, benzothiophenyl, indolinyl, 2-oxoindolinyl, dihydrobenzofuranyl, dihydrobenzothiophenyl, indazolyl, benzimidazolyl, benzooxazolyl, benzothiazolyl, benzoisoxazolyl, benzoisothiazolyl, benzotriazolyl, benzo[1,3]dioxolyl, quinolinyl, isoquinolinyl, quinazolinyl, cinnolinyl, pthalazinyl, quinoxalinyl, 2,3-dihydro-benzo[1,4]dioxinyl, benzo[1,2,3]triazinyl, benzo[1,2,4]triazinyl, 4*H*-chromenyl, indolizinyl, quinolizinyl, 6*aH*-thieno[2,3-d]imidazolyl, 1*H*-pyrrolo[2,3-*b*]pyridinyl, imidazo[1,2-*a*]pyridinyl, pyrazolo[1,5-*a*]pyridinyl, [1,2,4]triazolo[4,3-*a*]pyridinyl, [1,2,4]triazolo[1,5-*a*]pyridinyl, thieno[2,3-*b*]furanyl, thieno[2,3-*b*]pyridinyl, thieno[3,2-*b*]pyridinyl, furo[2,3-*b*]pyridinyl, furo[3,2-*b*]pyridinyl, thieno[3,2-*d*]pyrimidinyl, furo[3,2-*d*]pyrimidinyl, thieno[2,3-*b*]pyrazinyl, imidazo[1,2-*a*]pyrazinyl, 5,6,7,8-tetrahydroimidazo[1,2-*a*]pyrazinyl, 6,7-dihydro-4*H*-pyrazolo[5,1-*c*][1,4]oxazinyl, 2-oxo-2,3-dihydrobenzo[*d*]oxazolyl, 3,3-dimethyl-2-oxoindolinyl, 2-oxo-2,3-dihydro-1*H*-pyrrolo[2,3-*b*]pyridinyl, benzo[*c*][1,2,5]oxadiazolyl, benzo[*c*][1,2,5]thiadiazolyl, 3,4-dihydro-2H-benzo[*b*][1,4]oxazinyl, 5,6,7,8-tetrahydro-[1,2,4]triazolo[4,3-*a*]pyrazinyl, [1,2,4]triazolo[4,3-*a*]pyrazinyl, 3-oxo-[1,2,4]triazolo[4,3-*a*]pyridin-2(3*H*)-yl, and the like.

As used herein, "heterocycle" refers to a stable 3- to 18-membered ring (radical) of carbon atoms and from one to five heteroatoms selected from nitrogen, oxygen, and sulfur. The heterocycle may be a monocyclic or a polycyclic ring system, which may include fused, bridged, or spiro ring systems; and the nitrogen, carbon, or sulfur atoms in the heterocycle may be optionally oxidized; the nitrogen atom may be optionally quaternized; and the ring may be partially or fully saturated. Examples of such heterocycles include, without limitation, azepinyl, azocanyl, pyranyl dioxanyl, dithianyl, 1,3-dioxolanyl, tetrahydrofuryl, dihydropyrrolidinyl, decahydroisoquinolyl, imidazolidinyl, isothiazolidinyl, isoxazolidinyl, morpholinyl, octahydroindolyl, octahydroisoindolyl, 2-oxopiperazinyl, 2-oxopiperidinyl, 2-oxopyrrolidinyl, 2-oxoazepinyl, oxazolidinyl, oxiranyl, piperidinyl, piperazinyl, 4-piperidonyl, pyrrolidinyl, pyrazolidinyl, thiazolidinyl, tetrahydropyranyl, thiamorpholinyl, thiamorpholinyl sulfoxide, and thiamorpholinyl sulfone.

Further heterocycles and heteroaryls are described in Katritzky et al., eds., Comprehensive Heterocyclic Chemistry: The Structure, Reactions, Synthesis and Use of Heterocyclic Compounds, Vol. 1-8, Pergamon Press, N.Y. (1984).

The terms "carboxamide" or "amide" as used herein refer to C(O)NRₐR_{b} wherein Rₐ and R_{b} are each independently hydrogen, alkyl or any other suitable substituent.

The term "aminocarboxamide" means NH₂XC(O)NRₐR_{b} where X is phenyl or heterocycle and Rₐ and R_{b} are each independently hydrogen, alkyl, or any other chemically suitable substituent.

The phrases "substituted or unsubstituted" and "optionally substituted" mean a group may (but does not necessarily) have a substituent at each substitutable atom of the group (including more than one substituent on a single atom), and the identity of each substituent is independent of the others.

The term "substituted" means that one or more hydrogen on a designated atom is replaced with a selection from the indicated group, provided that the designated atom's normal valency is not exceeded. "Unsubstituted" atoms bear all of the hydrogen atoms dictated by their valency. When a substituent is oxo (*i.e*., =O), then 2 hydrogens on the atom are replaced. Combinations of substituents and/or variables are permissible only if such combinations result in stable compounds. By "stable compound" it is meant a compound that is sufficiently robust to survive isolation to a useful degree of purity from a reaction mixture and formulation into an efficacious therapeutic agent.

By "compound(s) of the invention" and equivalent expressions, it is meant compounds herein described, which expression includes the prodrugs, the pharmaceutically acceptable salts, the oxides, and the solvates, *e.g*. hydrates, where the context so permits.

Compounds described herein may contain one or more asymmetric centers and may thus give rise to enantiomers, diastereomers, and other stereoisomeric forms. Each chiral center may be defined, in terms of absolute stereochemistry, as (R)- or (S)-. The present invention is meant to include all such possible isomers, as well as mixtures thereof, including racemic and optically pure forms. Optically active (R)- and (S)-, (-)- and (+)-, or (D)- and (L)-isomers may be prepared using chiral synthons or chiral reagents, or resolved using conventional techniques. All tautomeric forms are also intended to be included.

As would be understood by a person of ordinary skill in the art, the recitation of "a compound" is intended to include salts, solvates, oxides, and inclusion complexes of that compound as well as any stereoisomeric form, or a mixture of any such forms of that compound in any ratio. Thus, in accordance with some embodiments of the invention, a compound as described herein, including in the contexts of pharmaceutical compositions, methods of treatment, and compounds *per se,* is provided as the salt form.

The term "solvate" refers to a compound in the solid state, where molecules of a suitable solvent are incorporated in the crystal lattice. A suitable solvent for therapeutic administration is physiologically tolerable at the dosage administered. Examples of suitable solvents for therapeutic administration are ethanol and water. When water is the solvent, the solvate is referred to as a hydrate. In general, solvates are formed by dissolving the compound in the appropriate solvent and isolating the solvate by cooling or using an antisolvent. The solvate is typically dried or azeotroped under ambient conditions.

Inclusion complexes are described in Remington, The Science and Practice of Pharmacy, 19th Ed. 1:176-177 (1995). The most commonly employed inclusion complexes are those with cyclodextrins, and all cyclodextrin complexes, natural and synthetic, are specifically encompassed by the present invention.

The term "pharmaceutically acceptable salt" refers to salts prepared from pharmaceutically acceptable non-toxic acids or bases including inorganic acids and bases and organic acids and bases.

The term "pharmaceutically acceptable" means it is, within the scope of sound medical judgment, suitable for use in contact with the cells of humans and lower animals without undue toxicity, irritation, allergic response and the like, and are commensurate with a reasonable benefit/risk ratio.

### BLANK PARAGRAPH

Z may be hydroxyl, -OCF₃, an unsubstituted phenyl ring or a phenyl ring substituted with a halogen, a nitrile, a benzene ring, C₁-C₆ alkoxy, or -CONH₂. Compounds include, without limitation:

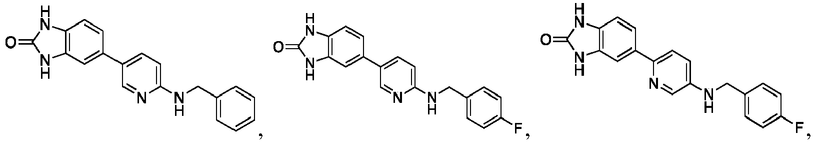

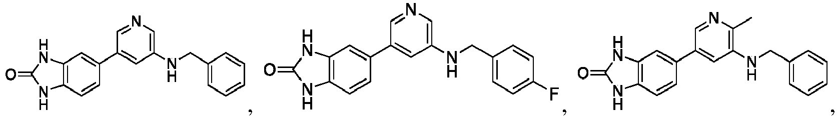

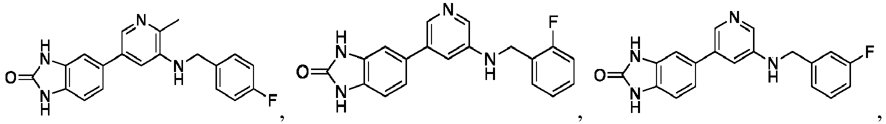

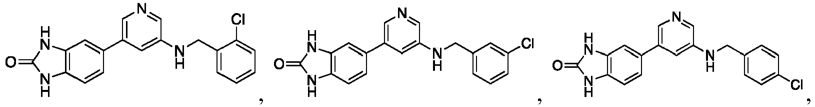

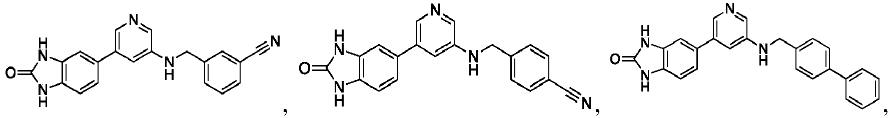

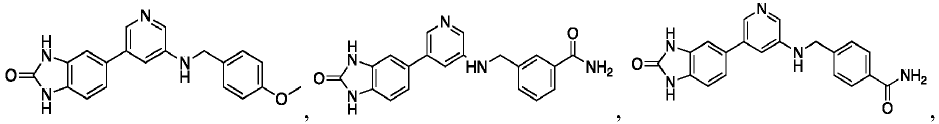

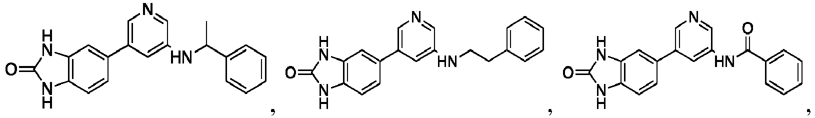

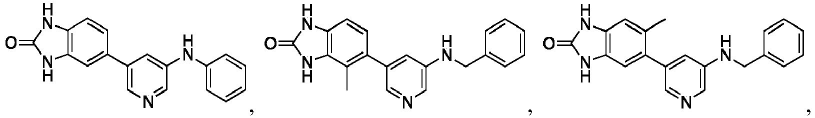

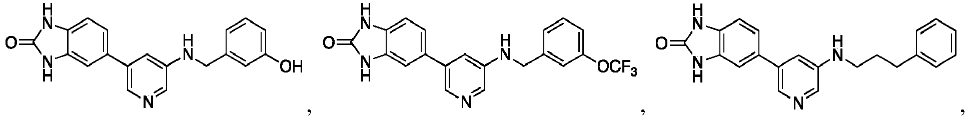

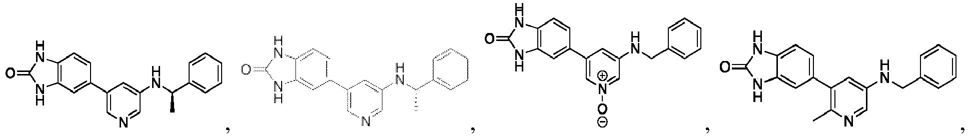

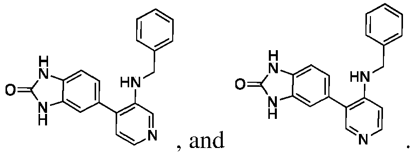

Also disclosed are compounds in which Z is selected from pyridinyl and naphthalene. Compounds include, without limitation: 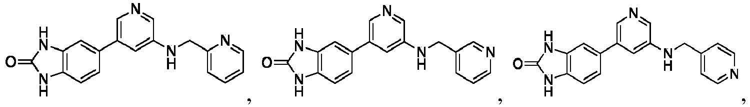 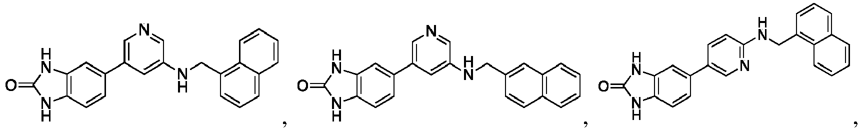 and

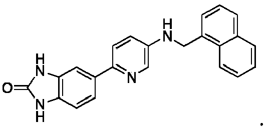

In another embodiment of the compound of formula (I),
R² and R³ are H;
R⁵ is NH;
Y is CH₂ or CH(CH₃);
X is N; and
Z is an unsubstituted phenyl ring or a phenyl ring substituted with a halogen.

The following compounds are also disclosed herein 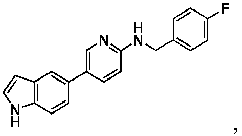 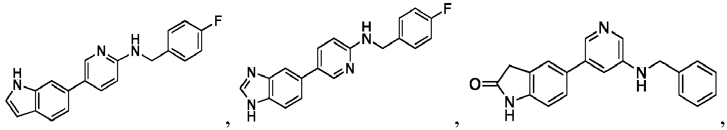 and

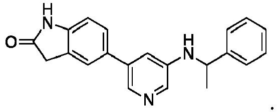

Also disclosed herein are compounds in which
R² is CH₃
R³ is H;
R⁵ is NH;
Y is CH₂ or CH(CH₃); and
XisN.

Exemplary compounds include, without limitation: 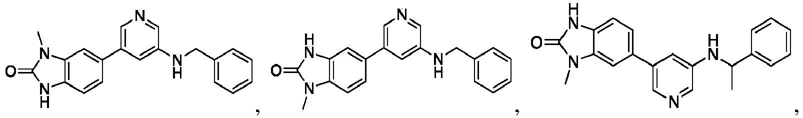 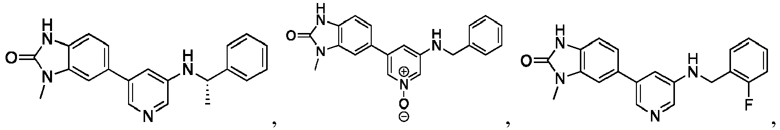 and

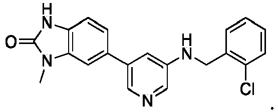

Also disclosed herein are compounds in which
R² is H;
R³ is H;
R⁴ is a carbonyl;
R⁵ is NH;
Y is CH₂; and
Z is a heteroaryl.

Exemplary compounds include

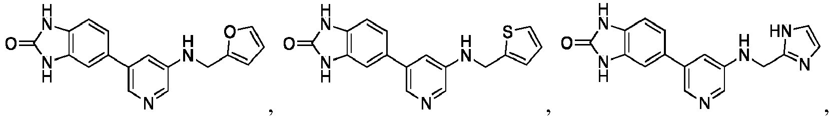

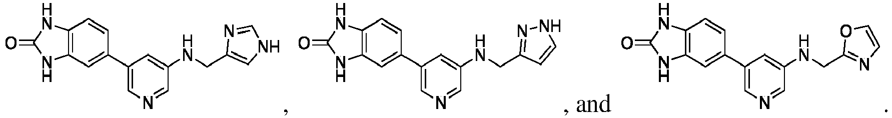

Also disclosed herein are compounds in which
R² is H;
R³ is H;
R⁴ is a carbonyl;
R⁵ is substituted or unsubstituted C₁-C₂ alkyl; and
Z is a unsubstituted phenyl ring or a phenyl ring substituted with a halogen or methoxy.

Exemplary compounds include 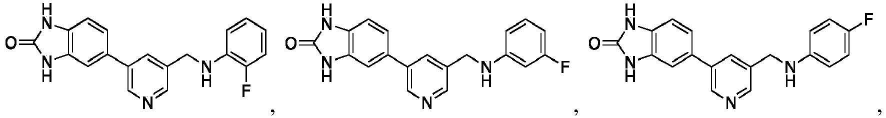 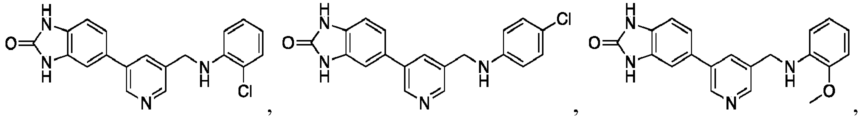 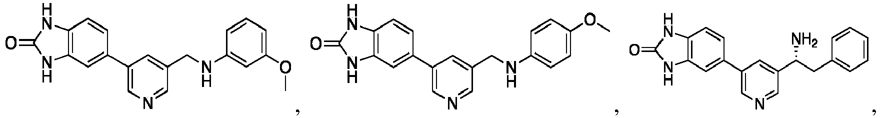 and

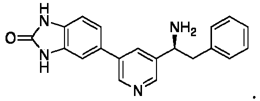

In yet another embodiment of the compound of formula (I),
R² is H;
R⁴ is a carbonyl;
R⁵ is NH;
Y is CH₂; and
Z is a phenyl ring.

The following compounds are also disclosed

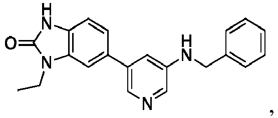

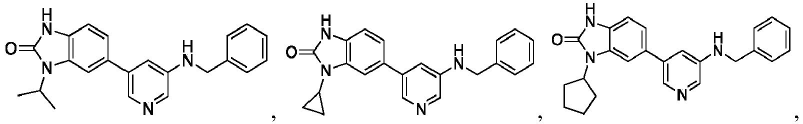

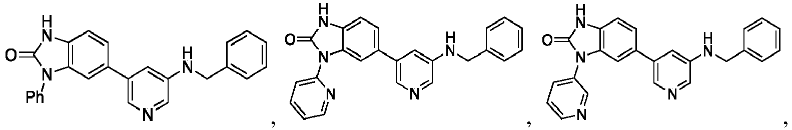

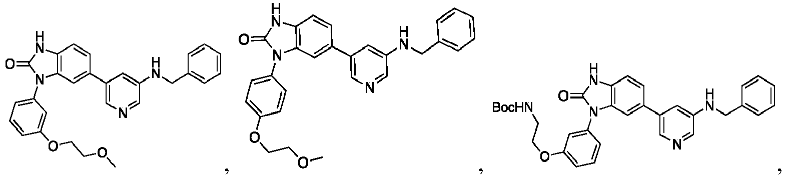

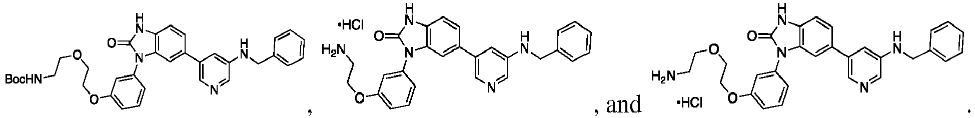

Also disclosed is a method of inhibiting activity of a kinase in a cell. This method involves contacting the cell with a compound of formula (I) under conditions effective to inhibit activity of the kinase in the cell.

The kinase may be a dual-specificity tyrosine phosphorylation-regulated kinase ("DYRK"). The kinase may be a dual-specificity tyrosine phosphorylation-regulated kinase 1A ("DYRK1A").

The cell may be a mammalian cell. Mammalian cells include cells from, for example, mice, hamsters, rats, cows, sheep, pigs, goats, and horses, monkeys, dogs (*e.g., Canis familiaris*)*,* cats, rabbits, guinea pigs, and primates, including humans. For example, the cell may be a human cell.

The cell may be a pancreatic beta cell. If needed, methods for determining whether a cell has a pancreatic beta cell phenotype are known in the art and include, without limitation, incubating the cell with glucose and testing whether insulin expression in the cell is increased or induced. Other methods include testing whether beta cell specific transcription factors are expressed, the detection of beta cell specific gene products with the help of RNA quantitative PCR, the transplantation of a candidate cell in diabetic mice, and subsequent testing of the physiologic response following said transplantation as well analyzing the cells with electron microscopy.

The cell may be a cancer cell.

The cell may be a neural cell.

Methods disclosed herein may be carried out *ex vivo* or *in vivo.* When carried out *ex vivo,* a population of cells may be, according to one embodiment, provided by obtaining cells from a pancreas and culturing the cells in a liquid medium suitable for the *in vitro* or *ex vivo* culture of mammalian cells, in particular human cells. For example, and without limitation, a suitable and non-limiting culture medium may be based on a commercially available medium such as RPMI1640 from Invitrogen.

Also disclosed herein is a method of increasing cell proliferation in a population of pancreatic beta cells. This method involves contacting a population of pancreatic beta cells with a compound of formula (I) under conditions effective to increase cell proliferation in the population of pancreatic beta cells.

Contacting may be carried out with a composition (*i.e*., a single composition) comprising the compound.

The method may further involve contacting the population of pancreatic beta cells with a transforming growth factor beta (TGFβ) superfamily signaling pathway inhibitor. The method may be carried out with a composition comprising the compound and the TGFβ superfamily signaling pathway inhibitor. The compound of formula (I) and the TGFβ superfamily signaling pathway inhibitor may separately contact a population of pancreatic beta cells simultaneously or in sequence.

TGFβ superfamily signaling pathway inhibitors include small molecules and other (*e.g*., neutralizing monoclonal antibodies, synthetic/recombinant peptide inhibitors, and siRNA) inhibitors of the BMP family of receptors, activing and inhibin receptors, GDF11 receptors and related receptors.

TGFβ superfamily signaling pathway inhibitors are also known in the art and include, without limitation, SB431542, SB505124, A-83-01, Decorin, soluble TGF-β receptor, Ierdelimumab, metelimumab, AP-12009, Follistatin, FLRG, GAST-1, GDF8 propeptide, MYO-029, Noggin, chordin, Cer/Dan, ectodin, and Sclerostin (*see* Tsuchida et al., "Inhibitors of the TGF-beta Superfamily and their Clinical Applications," Mini Rev. Med. Chem. 6(11):1255-61 (2006).

Other inhibitors of TGF-β signaling include, without limitation, 2-(3-(6-Methylpyridin-2-yl)-1H-pyrazol-4-yl)-1,5 napththyridine; [3-(Pyridin-2-yl)-4-(4-quinoyl)]-1H-pyrazole; 3-(6-Methylpyridin-2-yl)-4-(4-quinolyl)-1-phenylthiocarbamoyl-1*H*-pyrazole; SB-431542; SM16; SB-505124; and 2-(3-(6-Methylpyridin-2-yl)-1*H*-pyrazol-4-yl)-1,5 napththyridine (ALK5 Inhibitor II) (*see* U.S. Patent No. 8,298,825).

Inhibitors of TGF-β signaling are described in Callahan et al., J. Med. Chem. 45:999-1001 (2002); Sawyer et al., J. Med. Chem. 46:3953-3956 (2003); Gellibert et al., J. Med. Chem. 47:4494-4506 (2004); Tojo et al., Cancer Sci. 96:791-800 (2005); Valdimarsdottir et al., APMIS 113:773-389 (2005); Petersen et al., Kidney International 73:705-715 (2008); Yingling et al., Nature Rev. Drug Disc. 3:1011-1022 (2004); Byfield et al., Mol. Pharmacol. 65:744-752 (2004); Dumont et al., Cancer Cell 3:531-536 (2003); PCT Publication No. WO 2002/094833; PCT Publication No. WO 2004/026865; PCT Publication No. WO 2004/067530; PCT Publication No. WO 2009/032667; PCT Publication No. WO 2004/013135; PCT Publication No. WO 2003/097639; PCT Publication No. WO 2007/048857; PCT Publication No. WO 2007/018818; PCT Publication No. WO 2006/018967; PCT Publication No. WO 2005/039570; PCT Publication No. WO 2000/031135; PCT Publication No. WO 1999/058128; U.S. Patent No. 6,509,318; U.S. Patent No. 6,090,383; U.S. Patent No. 6,419,928; U.S. Patent No. 9,927,738; U.S. Patent No. 7,223,766; U.S. Patent No. 6,476,031; U.S. Patent No. 6,419,928; U.S. Patent No. 7,030,125; U.S. Patent No. 6,943,191; U.S. Patent Application Publication No. 2005/0245520; U.S. Patent Application Publication No. 2004/0147574; U.S. Patent Application Publication No. 2007/0066632; U.S. Patent Application Publication No. 2003/0028905; U.S. Patent Application Publication No. 2005/0032835; U.S. Patent Application Publication No. 2008/0108656; U.S. Patent Application Publication No. 2004/015781; U.S. Patent Application Publication No. 2004/0204431; U.S. Patent Application Publication No. 2006/0003929; U.S. Patent Application Publication No. 2007/0155722; U.S. Patent Application Publication No. 2004/0138188 and U.S. Patent Application Publication No. 2009/0036382.

Exemplary inhibitors of TGF-β signaling include, but are not limited to, AP-12009 (TGF-β Receptor type II antisense oligonucleotide), Lerdelimumab (CAT 152, antibody against TGF-β Receptor type II) GC-1008 (antibody to all isoforms of human TGF-β), ID11 (antibody to all isoforms of murine TGF-β), soluble TGF-β, soluble TGF-β Receptor type II, dihydropyrroloimidazole analogs (*e.g.,* SKF-104365), triarylimidazole analogs (*e.g.,* SB-202620 (4-(4-(4-fluorophenyl)-5-(pyridin-4-yl)-1H-imidazol-2-yl)benzoic acid) and SB-203580 (4-(4-Fluorophenyl)-2-(4-methylsulfinyl phenyl)-5-(4-pyridyl)-1H-imidazole)), RL-0061425, 1,5-naphthyridine aminothiazole and pyrazole derivatives (*e.g*., 4-(6-methyl-pyridin-2-yl)-5-(1,5-naphthyridin-2-yl)-1,3-thiazole-2-amine and 2-[3-(6-methyl-pyridin-2-yl)-1H-pyrazole-4-yl]-1,5-naphthyridine), SB-431542 (4-(5-Benzol[1,3]dioxol-5-yl-4-pyridin-2-yl-1H-imidazol-2-yl)-benzamide), GW788388 (4-(4-(3-(pyridin-2-yl)-1H-pyrazol-4-yl)pyridin-2-yl)-N-(tetrahydro-2H-pyran-4-yl)benzamide), A-83-01 (3-(6-Methyl-2-pyridinyl)-N-phenyl-4-(4-quinolinyl)-1H-pyrazole-1-carbothioamide), Decorin, Lefty 1, Lefty 2, Follistatin, Noggin, Chordin, Cerberus, Gremlin, Inhibin, BIO (6-bromo-indirubin-3'-oxime), Smad proteins (*e.g*., Smad6, Smad7), and Cystatin C.

Inhibitors of TGF-β signaling also include molecules which inhibit TGF-β Receptor type I. Inhibitors of TGF-β Receptor type I include, but are not limited to, soluble TGF-β Receptor type I; AP-11014 (TGF-β Receptor type I antisense oligonucleotide); Metelimumab (CAT 152, TGF-β Receptor type I antibody); LY550410; LY580276 (3-(4-fluorophenyl)-5,6-dihydro-2-(6-methylpyridin-2-yl)-4H-pyrrolo[1,2-b]pyrazole); LY364947 (4-[3-(2-Pyridinyl)-1H-pyrazol-4-yl]-quinoline); LY2109761; LY573636 (N-((5-bromo-2-thienyl)sulfonyl)-2,4-dichlorobenzamide); SB-505124 (2-(5-Benzo[1,3]dioxol-5-yl-2-tert-butyl-3H-imidazol-4-yl)-6-methylpyridine); SD-208 (2-(5-Chloro-2-fluorophenyl)-4-[(4-pyridyl)amino]pteridine); SD-093; KI2689; SM16; FKBP12 protein; and 3-(4-(2-(6-methylpyridin-2-yl)H-imidazo[1,2-a]pyridin-3-yl)quinolin-7-yloxy)-N,N-dimethylpropan-1-amine.

Inhibitors of TGF-β Receptor type I are described in Byfield and Roberts, Trends Cell Biol. 14:107-111 (2004); Sawyer et al., Bioorg. Med. Chem. Lett. 14:3581-3584 (2004); Sawyer et al., J. Med. Chem. 46:3953-3956 (2003); Byfield et al., Mol. Pharmacol. 65:744-752 (2004); Gellibert et al., J. Med. Chem. 47:4494-4506 (2004); Yingling et al., Nature Rev. Drug Disc. 3:1011-1022 (2004); Dumont et al., Cancer Cell 3:531-536 (2003); Tojo et al., Cancer Sci. 96:791-800 (2005); PCT Publication No. WO 2004/026871; PCT Publication No. WO 2004/021989; PCT Publication No. WO 2004/026307; PCT Publication No. WO 2000/012497; U.S. Patent No. 5,731,424; U.S. Patent No. 5,731,144; U.S. Patent No. 7,151,169; U.S. Patent Application Publication No. 2004/00038856 and U.S. Patent Application Publication No. 2005/0245508, all of which are herein incorporated in their entirety.

In one embodiment, the TGFβ superfamily signaling pathway inhibitor includes compounds that interfere with TGFβ superfamily ligands, receptors, and/or downstream signaling molecules (*e.g*., SMADs) or nuclear targets (*e.g*., chromatin modifying complexes and transcription factors).

In one embodiment, the TGFβ superfamily signaling pathway inhibitor may be antisera that neutralize, *e.g*., TGFβ ligand.

In another embodiment, the TGFβ superfamily signaling pathway inhibitor is selected from the group consisting of an inhibitor of TOPPβ/TGFβ receptor binding, activin or inhibin/activin receptor binding, and bone morphogenetic protein (BMP)/BMP receptor binding.

The TGFβ superfamily signaling pathway inhibitor may be an inhibitor of TGFβ/TGFβ receptor binding selected from the group consisting of LY364947 and GW788388.

The TGFP superfamily signaling pathway inhibitor may be an inhibitor of activin or inhibin/activin receptor binding selected from the group consisting of SB431542 and Alk5 inhibitor II. Additional exemplary inhibitors of activin or inhibin/activin receptor binding may be selected from the group consisting of SB-505124, BYM388, follistatin, follistatin-related protein (FSRP), follistatin domains (*i.e.,* Fs2, Fs12, Fs123), A-83-01, Cripto, GW788388, BAMBI, and Sotatercept (*see* Byfield et al., "SB-505124 is a Selective Inhibitor of Transforming Growth Factor-Beta Type I Receptors ALK4, ALK5, and ALK7," Mol. Pharmacol. 65(3):744-52 (2004); Lach-Trifilieffa et al., "An Antibody Blocking Activin Type II Receptors Induces Strong Skeletal Muscle Hypertrophy and Protects from Atrophy," Mol. Cell. Biol. 34(4):606-18 (2014); Zhang et al., "Inhibition of Activin Signaling Induces Pancreatic Epithelial Cell Expansion and Diminishes Terminal Differentiation of Pancreatic β-Cells," Diabetes 53(8):2024-33 (2004); Harrington et al., "Structural Basis for the Inhibition of Activin Signalling by Follistatin," EMBO J. 25(5):1035-45 (2006); Tojo et al., "The ALK-5 Inhibitor A-83-01 Inhibits Smad Signaling and Epithelial-to-Mesenchymal Transition by Transforming Growth Factor-Beta," Cancer Sci. 96(11):790-800 (2005); Yan et al., "Human BAMBI Cooperates with Smad7 to Inhibit Transforming Growth Factor-Beta Signaling," J. Biol. Chem. 284(44):30097-104 (2009); Tan et al., "Targeted Inhibition of Activin Receptor-Like Kinase 5 Signaling Attenuates Cardiac Dysfunction Following Myocardial Infarction," Am. J. Physiol. Heart Circ. Physiol. 298(5):H1415-25 (2010); and Gokoffski et al., "Activin and GDF11 Collaborate in Feedback Control of Neuroepithelial Stem Cell Proliferation and Fate," Develop. 138(19):4131-42 (2011)).

The TGFβ superfamily signaling pathway inhibitor may be an inhibitor of BMP/BMP receptor binding. An exemplary inhibitor of BMP/BMP receptor binding is LDN193189. Additional exemplary BMP inhibitors may be selected from the group consisting of noggin, sclerostin, chordin, CTGF, follistatin, gremlin, inhibin, DMH1, DMH2, Dorsomorphin, K02288, LDN212854, DM 3189, BMP-3, and BAMBI (*see* WO 2014018691 A1 and Mohedas et al., "Development of an ALK2-Biased BMP Type I Receptor Kinase Inhibitor," ACS Chem. Biol. 8(6):1291-302 (2013); Yan et al., "Human BAMBI Cooperates with Smad7 to Inhibit Transforming Growth Factor-Beta Signaling," J. Biol. Chem. 284(44):30097-104 (2009)).

The TGFβ superfamily signaling pathway inhibitor may be a SMAD signaling pathway inhibitor. Exemplary SMAD signaling pathway inhibitors may be selected from the group including, without limitation, SMAD3 siRNA, SMAD 2/3 siRNA, PD169316, SB203580, SB202474, specific inhibitor of Smad3 (SIS3), HSc025, and SB525334 (*see* Qureshi et al., "Smad Signaling Pathway is a Pivotal Component of Tissue Inhibitor of Metalloproteinases-3 Regulation by Transforming Growth Factor Beta in Human Chondrocytes," BBA Mol. Cell Res. 1783(9):1605-12 (2008); Hasegawa et al., "A Novel Inhibitor of Smad-Dependent Transcriptional Activation Suppresses Tissue Fibrosis in Mouse Models of Systemic Sclerosis, Arthritis Rheum. 60(11):3465-75 (2009); and Ramdas et al., "Canonical Transforming Growth Factor-β Signaling Regulates Disintegrin Metalloprotease Expression in Experimental Renal Fibrosis via miR-29," Am. J. Pathol. 183(6):1885-96 (2013)).

Additional exemplary SMAD signaling pathway inhibitors include, without limitation, miR-100, LDN 193189, SMAD-binding peptide aptamers (*e.g*., Trx-FoxH1, Trx-Le1, Trx-CBP, Trx-SARA), pirfenidone, and LDN193189 (*see* Fu et al., "MicroRNA-100 Inhibits Bone Morphogenetic Protein-Induced Osteoblast Differentiation by Targeting Smad," Eur. Rev. Med. Pharmacol. Sci. 20(18):3911-19 (2016); Boergermann et al., "Dorsomorphin and LDN-193189 Inhibit BMP-Mediated Smad, p38 and Akt signalling in C2C12 Cells," Int. J. Biochem. Cell Biol. 42(11):1802-7 (2010); Cui et al., "Selective Inhibition of TGF-Responsive Genes by Smad-Interacting Peptide Aptamers from FoxH1, Lef1 and CBP," Oncogene 24:3864-74 (2005); Zhao et al., "Inhibition of Transforming Growth Factor-Beta1-Induced Signaling and Epithelial-to-Mesenchymal Transition by the Smad-Binding Peptide Aptamer Trx-SARA," Mol. Biol. Cell 17:3819-31 (2006); Li et al., "Oral Pirfenidone Protects Against Fibrosis by Inhibiting Fibroblast Proliferation and TGF-β Signaling in a Murine Colitis Model," Biochem. Pharmacol. 117:57-67 (2016); and Cook et al., "BMP Signaling Balances Murine Myeloid Potential Through SMAD-Independent p38MAPK and NOTCH Pathways," Blood 124(3):393-402 (2014)).

The TGFβ superfamily signaling pathway inhibitor may be an inhibitor of the trithorax complex. Exemplary trithorax complex inhibitors include, without limitation, WDR5-0103, MI-1, MI-2, MI-2-2, MLS001171971-01, ML227, MCP-1, RBB5 siRNA, and MLL1 siRNA (*see* Senisterra et al., "Small-Molecule Inhibition of MLL Activity by Disruption of its Interaction with WDR5," Biochem. J. 449(1):151-9 (2013); Cierpicki et al., "Challenges and Opportunities in Targeting the Menin-MLL Interaction," Future Med. Chem. 6(4):447-62 (2014); Lee et al., "Roles of DPY30 in the Proliferation and Motility of Gastric Cancer Cells," PLOS One 10(7):e0131863 (2015); and Zhou et al., "Combined Modulation of Polycomb and Trithorax Genes Rejuvenates β Cell Replication," J. Clin. Invest. 123(11):4849-4858 (2013)).

The TGFβ superfamily signaling pathway inhibitor may be an inhibitor of the polycomb repressive complex 2 ("PRC2"). Exemplary PRC2 inhibitors include GSK926, EPZ005687, GSK126, GSK343, E11, UNC1999, EPZ6438, Constellation Compound 3, EZH2 siRNA, and 3-deazaneplanocin A (*see* Verma et al., "Identification of Potent, Selective, Cell-Active Inhibitors of the Histone Lysine Methyltransferase EZH2," ACS Med. Chem. Lett. 3:1091-6 (2012); Xu et al., "Targeting EZH2 and PRC2 Dependence as Novel Anticancer Therapy," Exp. Hematol. 43:698-712 (2015); Knutson et al., "A Selective Inhibitor of EZH2 Blocks H3K27 Methylation and Kills Mutant Lymphoma Cells," Nat. Chem. Biol. 8:890-6 (2012); Qi et al., "Selective Inhibition of Ezh2 by a Small Molecule Inhibitor Blocks Tumor Cells Proliferation," Proc. Natl Acad. Sci. USA 109:21360-65 (2012); McCabe et al., "EZH2 Inhibition as a Therapeutic Strategy for Lymphoma with EZH2-Activating Mutations," Nature 492:108-12 (2012); Nasveschuk et al., "Discovery and Optimization of Tetramethylpiperidinyl Benzamides as Inhibitors of EZH2," ACS Med. Chem. Lett. 5:378-83 (2014); Brooun et al., "Polycomb Repressive Complex 2 Structure with Inhibitor Reveals a Mechanism of Activation and Drug Resistance," Nature Comm. 7:11384 (2016); Fiskus et al., "Histone Deacetylase Inhibitors Deplete Enhancer of Zeste 2 and Associated Polycomb Repressive Complex 2 Proteins in Human Acute Leukemia Cells," Mol. Cancer Ther. 5(12):3096-104 (2006); and Fiskus et al., "Combined Epigenetic Therapy with the Histone Methyltransferase EZH2 Inhibitor 3-Deazaneplanocin A and the Histone Deacetylase Inhibitor Panobinostat Against Human AML Cells," Blood 114(13):2733-43 (2009).)

The method may further involve contacting the population of pancreatic beta cells with a glucagon-like peptide-1 receptor (GLP1R) agonist and/or a Dipeptidyl Peptidase IV ("DDP4") inhibitor. The method may be carried out with a composition comprising a compound according to formula (I) and the glucagon-like peptide-1 receptor (GLP1R) agonist and/or the DDP4 inhibitor, and, optionally, the TGFβ superfamily signaling pathway inhibitor. The compound of formula (I), the GLP1R agonist and/or the DDP4 inhibitor, and, optionally, the TGFβ superfamily signaling pathway inhibitor may each contact the population of pancreatic beta cells simultaneously or in sequence.

Glucagon-like peptide-1 receptor agonists mimic the effects of the incretin hormone GLP-1, which is released from the intestine in response to food intake. Their effects include increasing insulin secretion, decreasing glucagon release, increasing satiety, and slowing gastric emptying. An alternate approach to enhancing GLP1 concentrations in blood is prevention of its degradation by the enzyme DPP4. The GLP1 receptor agonists and the DDP4 inhibitors are among the most widely used drugs for the treatment of Type 2 diabetes (Campbell et al., "Pharmacology, Physiology and Mechanisms of Incretin Hormone Action," Cell Metab. 17:819-37 (2013); Guo X-H., "The Value of Short- and Long-Acting Glucagon-Like Peptide Agonists in the Management of Type 2 Diabetes Mellitus: Experience with Exenatide," Curr. Med. Res. Opinion 32(1):61-76 (2016); Deacon et al., "Dipeptidyl Peptidase-4 Inhibitors for the Treatment of Type 2 Diabetes: Comparison, Efficacy and Safety," Expert Opinion on Pharmacotherapy 14:2047-58 (2013); Lovshin, "Glucagon-Like Peptide-1 Receptor Agonists: A Class Update for Treating Type 2 Diabetes," Can. J. Diabetes 41:524-35 (2017); and Yang et al., "Lixisenatide Accelerates Restoration of Normoglycemia and Improves Human Beta Cell Function and Survival in Diabetic Immunodeficient NOD-scid IL2rg(null) RIP-DTR Mice Engrafted With Human Islets," Diabetes Metab. Syndr. Obes. 8:387-98 (2015)).

Suitable GLP1R agonists include, *e.g*. and without limitation, exenatide, liraglutide, exenatide LAR, taspoglutide, lixisenatide, albiglutide, dulaglutide, and semaglutide. Exenatide and Exenatide LAR are synthetic exendin-4 analogues obtained from the saliva of the *Heloderma suspectum* (lizard). Liraglutide is an acylated analogue of GLP-1 that self-associates into a heptameric structure that delays absorption from the subcutaneous injection site. Taspoglutide shares 3% homology with the native GLP-1 and is fully resistant to DPP-4 degradation. Lixisenatide is a human GLP1R agonist. Albiglutide is a long-acting GLP-1 mimetic, resistant to DPP-4 degradation. Dulaglutide is a long-acting GLP1 analogue. Semaglutide is a GLP1R agonist approved for the use of T2D. Clinically available GLP1R agonists include, *e.g*., exenatide, liraglutide, albiglutide, dulaglutide, lixisenatide, semaglutide.

In some embodiments of the methods and compositions of the present invention, the GLP1R agonist is selected from the group consisting of GLP1(7-36), extendin-4, liraglutide, lixisenatide, semaglutide, and combinations thereof.

Additional suitable GLP1 agonists include, without limitation, disubstituted-7-aryl-5,5-bis(trifluoromethyl)-5,8-dihydropyrimido[4,5-d]pyrimidine-2,4(1H,3H)-dione compounds and derivatives thereof, *e.g*., 7-(4-Chlorophenyl)-1,3-dimethyl-5,5-bis(trifluoromethyl)-5,8-dihydropyrimido[4,5-d]pyrimidine-2,4(1H,3H)-dione (*see, e.g.,* Nance et al., "Discovery of a Novel Series of Orally Bioavailable and CNS Penetrant Glucagon-like Peptide-1 Receptor (GLP-1R) Noncompetitive Antagonists Based on a 1,3-Disubstituted-7-aryl-5,5-bis(trifluoromethyl)-5,8-dihydropyrimido[4,5-d]pyrimidine-2,4(1H,3H)-dione Core," J. Med. Chem. 60:1611-1616 (2017)).

Further suitable GLP1 agonists include positive allosteric modulators ("PAMS") of GLP1R, *e.g.*, (S)-2-cyclopentyl-N-((1-isopropylpyrrolidin-2-yl)methyl)-10-methyl-1-oxo-1,2-dihydropyrazino[1,2-a]indole-4-carboxamide; (R)-2-cyclopentyl-N-((1-isopropylpyrrolidin-2-yl)methyl)-10-methyl-1-oxo-1,2-dihydropyrazino[1,2-a]indole-4-carboxamide; 2-cyclopentyl-N-(((S)-1-isopropylpyrrolidin-2-yl)methyl)-10-methyl-1-oxo-1,2,3,4-tetrahydropyrazino[1,2-a]indole-4-carboxamide; N-(((S)-1-isopropylpyrrolidin-2-yl)methyl)-10-methyl-1-oxo-2-((S)-tetrahydrofuran-3-yl)-1,2-dihydropyrazino[1,2-a]indole-4-carboxamide; N-(((R)-1-isopropylpyrrolidin-2-yl)methyl)-10-methyl-1-oxo-2-((S)-tetrahydrofuran-3-yl)-1,2-dihydropyrazino[1,2-a]indole-4-carboxamide; (S)-2-cyclopentyl-8-fluoro-N-((1-isopropylpyrrolidin-2-yl)methyl)-10-methyl-1-oxo-1,2-dihydropyrazino[1,2-a]indole-4-carboxamide; (R)-2-cyclopentyl-8-fluoro-N-((1-isopropylpyrrolidin-2-yl)methyl)-10-methyl-1-oxo-1,2-dihydropyrazino[1,2-a]indole-4-carboxamide; (R)-2-cyclopentyl-N-(((S)-1-isopropylpyrrolidin-2-yl)methyl)-10-methyl-1-oxo-1,2,3,4-tetrahydropyrazino[1,2-a]indole-4-carboxamide; (S)-2-cyclopentyl-N-(((S)-1-isopropylpyrrolidin-2-yl)methyl)-10-methyl-1-oxo-1,2,3,4-tetrahydropyrazino[1,2-a]indole-4-carboxamide; (S)-10-chloro-2-cyclopentyl-N-((1-isopropylpyrrolidin-2-yl)methyl)-1-oxo-1,2-dihydropyrazino[1,2-a]indole-4-carboxamide; (R)-10-chloro-2-cyclopentyl-N-((1-isopropylpyrrolidin-2-yl)methyl)-1-oxo-1,2-dihydropyrazino[1,2-a]indole-4-carboxamide; (S)-10-bromo-2-cyclopentyl-N-((1-isopropylpyrrolidin-2-yl)methyl)-1-oxo-1,2-dihydropyrazino[1,2-a]indole-4-carboxamide; (R)-10-bromo-2-cyclopentyl-N-((1-isopropylpyrrolidin-2-yl)methyl)-1-oxo-1,2-dihydropyrazino[1,2-a]indole-4-carboxamide; (R)-N-((1-isopropylpyrrolidin-2-yl)methyl)-10-methyl-1-oxo-2-phenyl-1,2-dihydropyrazino[1,2-a]indole-4-carboxamide; (S)-10-cyano-2-cyclopentyl-N-((1-isopropylpyrrolidin-2-yl)methyl)-1-oxo-1,2-dihydropyrazino[1,2-a]indole-4-carboxamide; (S)-2-cyclopentyl-N-((1-isopropylpyrrolidin-2-yl)methyl)-1-oxo-10-vinyl-1,2-dihydropyrazino[1,2-a]indole-4-carboxamide; (S)-N-((1-isopropylpyrrolidin-2-yl)methyl)-10-methyl-2-(1-methyl-1H-pyrazol-4-yl)-1-oxo-1,2-dihydropyrazino[1,2-a]indole-4-carboxamide; (R)-N-((1-isopropylpyrrolidin-2-yl)methyl)-10-methyl-2-(1-methyl-1H-pyrazol-4-yl)-1-oxo-1,2-dihydropyrazino[1,2-a]indole-4-carboxamide; (S)-N-((1-isopropylpyrrolidin-2-yl)methyl)-10-methyl-1-oxo-2-(pyridin-3-yl)-1,2-dihydropyrazino[1,2-a]indole-4-carboxamide; (R)-N-((1-isopropylpyrrolidin-2-yl)methyl)-10-methyl-1-oxo-2-(pyridin-3-yl)-1,2-dihydropyrazino[1,2-a]indole-4-carboxamide; N-(azetidin-2-ylmethyl)-2-cyclopentyl-10-methyl-1-oxo-1,2-dihydropyrazino[1,2-a]indole-4-carboxamide; and 2-cyclopentyl-N-((1-isopropylazetidin-2-yl)methyl)-10-methyl-1-oxo-1,2-dihydropyrazino[1,2-a]indole-4-carboxamide; or pharmaceutically acceptable salts thereof (*see* PCT Publication No. WO 2017/117556).

Suitable DDP4 inhibitors include, without limitation, sitagliptin, vildagliptin, saxagliptin, alogliptin, teneligliptin, and anagliptin.

"Pancreatic beta cells" may be primary human pancreatic beta cells.

Contacting may not induce beta cell death or DNA damage. Moreover, contacting may induce beta cell differentiation and increase glucose-stimulated insulin secretion.

The method may be carried out to enhance cell survival. For example, the method may be carried out to enhance cell survival of a treated population of cells relative to an untreated population of cells. Alternatively, the method may be carried out to decrease cell death or apoptosis of a treated population of cells relative to an untreated population of cells.

A further aspect of the present invention relates to a composition comprising a compound of formula (I) described herein and a carrier.

The composition may further comprise a transforming growth factor beta (TGFβ) superfamily signaling pathway inhibitor.

In another embodiment, the composition may further comprise a glucagon-like peptide-1 receptor (GLP1R) agonist or a Dipeptidyl Peptidase IV (DDP4) inhibitor.

The carrier may be a pharmaceutically-acceptable carrier.

While it may be possible for compounds of formula (I) to be administered as the raw chemical, they may also be administered as a pharmaceutical composition. In accordance with an embodiment of the present invention, there is provided a pharmaceutical composition comprising a compound of formula (I) or a pharmaceutically acceptable salt or solvate thereof, together with one or more pharmaceutically carriers thereof and optionally one or more other therapeutic ingredients.

The carrier(s) must be "acceptable" in the sense of being compatible with the other ingredients of the formulation and not deleterious to the recipient thereof. Furthermore, notwithstanding the statements herein regarding the term "compound" including salts thereof as well, so that independent claims reciting "a compound" will be understood as referring to salts thereof as well, if in an independent claim reference is made to a compound or a pharmaceutically acceptable salt thereof, it will be understood that claims which depend from that independent claim which refer to such a compound also include pharmaceutically acceptable salts of the compound, even if explicit reference is not made to the salts in the dependent claim.

Formulations include those suitable for oral, parenteral (including subcutaneous, intradermal, intramuscular, intravenous, and intraarticular), rectal and topical (including dermal, buccal, sublingual, and intraocular) administration. The most suitable route may depend upon the condition and disorder of the recipient. The formulations may conveniently be presented in unit dosage form and may be prepared by any of the methods well known in the art of pharmacy. Such methods include the step of bringing into association a compound of formula (I) or a pharmaceutically acceptable salt or solvate thereof ("active ingredient") with the carrier, which constitutes one or more accessory ingredients. In general, the formulations are prepared by uniformly and intimately bringing into association the active ingredient with liquid carriers or finely divided solid carriers or both and then, if necessary, shaping the product into the desired formulation.

Formulations suitable for oral administration may be presented as discrete units such as capsules, cachets, or tablets each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary, or paste.

A tablet may be made by compression or molding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent. Molded tablets may be made by molding in a suitable machine a mixture of the powdered compound moistened with an inert liquid diluent. The tablets may optionally be coated or scored and may be formulated so as to provide sustained, delayed or controlled release of the active ingredient therein.

The pharmaceutical compositions may include a "pharmaceutically acceptable inert carrier," and this expression is intended to include one or more inert excipients, which include, for example and without limitation, starches, polyols, granulating agents, microcrystalline cellulose, diluents, lubricants, binders, disintegrating agents, and the like. If desired, tablet dosages of the disclosed compositions may be coated by standard aqueous or nonaqueous techniques. "Pharmaceutically acceptable carrier" also encompasses controlled release means.

Pharmaceutical compositions may also optionally include other therapeutic ingredients, anti-caking agents, preservatives, sweetening agents, colorants, flavors, desiccants, plasticizers, dyes, and the like. Any such optional ingredient must be compatible with the compound of formula (I) to insure the stability of the formulation. The composition may contain other additives as needed including, for example, lactose, glucose, fructose, galactose, trehalose, sucrose, maltose, raffinose, maltitol, melezitose, stachyose, lactitol, palatinite, starch, xylitol, mannitol, myoinositol, and the like, and hydrates thereof, and amino acids, for example alanine, glycine and betaine, and peptides and proteins, for example albumen.

Examples of excipients for use as the pharmaceutically acceptable carriers and the pharmaceutically acceptable inert carriers and the aforementioned additional ingredients include, but are not limited to, binders, fillers, disintegrants, lubricants, anti-microbial agents, and coating agents.

Dose ranges for adult humans vary, but may generally be from about 0.005 mg to 10 g/day orally. Tablets or other forms of presentation provided in discrete units may conveniently contain an amount of compound of formula (I) which is effective at such dosage or as a multiple of the same, for instance, units containing 5 mg to 500 mg, or around 10 mg to 200 mg. The precise amount of compound administered to a patient will be the responsibility of the attendant physician. However, the dose employed will depend on a number of factors, including the age and sex of the patient, the precise disorder being treated, and its severity.

A dosage unit (*e.g*., an oral dosage unit) can include from, for example, 1 to 30 mg, 1 to 40 mg, 1 to 100 mg, 1 to 300 mg, 1 to 500 mg, 2 to 500 mg, 3 to 100 mg, 5 to 20 mg, 5 to 100 mg (*e.g.,* 1 mg, 2 mg, 3 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 150 mg, 200 mg, 250 mg, 300 mg, 350 mg, 400 mg, 450 mg, 500 mg) of a compound described herein.

Additional information about pharmaceutical compositions and their formulation is described in Remington: The Science and Practice of Pharmacy, 20th Edition, 2000.

The agents can be administered, *e.g*., by intravenous injection, intramuscular injection, subcutaneous injection, intraperitoneal injection, topical, sublingual, intraarticular (in the joints), intradermal, buccal, ophthalmic (including intraocular), intranasaly (including using a cannula), or by other routes. The agents can be administered orally, *e.g.,* as a tablet or cachet containing a predetermined amount of the active ingredient, gel, pellet, paste, syrup, bolus, electuary, slurry, capsule, powder, granules, as a solution or a suspension in an aqueous liquid or a non-aqueous liquid, as an oil-in-water liquid emulsion or a water-in-oil liquid emulsion, via a micellar formulation (*see, e.g.,* PCT Publication No. WO 97/11682) via a liposomal formulation (*see, e.g.,* EP Patent No. 736299, PCT Publication No. WO 99/59550, and PCT Publication No. WO 97/13500), via formulations described in PCT Publication No. WO 03/094886 () or in some other form. The agents can also be administered transdermally (*i.e.,* via reservoir-type or matrix-type patches, microneedles, thermal poration, hypodermic needles, iontophoresis, electroporation, ultrasound, or other forms of sonophoresis, jet injection, or a combination of any of the preceding methods (Prausnitz et al. Nature Reviews Drug Discovery 3:115 (2004)). The agents can be administered locally.

The agents can be administered in the form a suppository or by other vaginal or rectal means. The agents can be administered in a transmembrane formulation as described in PCT Publication No. WO 90/07923. The agents can be administered non-invasively via the dehydrated particles described in U.S. Patent No. 6,485,706. The agents can be administered in an enteric-coated drug formulation as described in PCT Publication No. WO 02/49621. The agents can be administered intranasaly using the formulation described in U.S. Patent No. 5,179,079. Formulations suitable for parenteral injection are described in PCT Publication No. WO 00/62759. The agents can be administered using the casein formulation described in U.S. Patent Application Publication No. 2003/0206939 and PCT Publication No. WO 00/06108. The agents can be administered using the particulate formulations described in U.S. Patent Application Publication No. 20020034536.

The agents, alone or in combination with other suitable components, can be administered by pulmonary route utilizing several techniques including, but not limited to, intratracheal instillation (delivery of solution into the lungs by syringe), intratracheal delivery of liposomes, insufflation (administration of powder formulation by syringe or any other similar device into the lungs), and aerosol inhalation. Aerosols (*e.g*., jet or ultrasonic nebulizers, metered-dose inhalers ("MDIs"), and dry-Powder inhalers ("DPIs")) can also be used in intranasal applications. Aerosol formulations are stable dispersions or suspensions of solid material and liquid droplets in a gaseous medium and can be placed into pressurized acceptable propellants, such as hydrofluoroalkanes (HFAs, *i.e.,* HFA-134a and HFA-227, or a mixture thereof), dichlorodifluoromethane (or other chlorofluorocarbon propellants such as a mixture of Propellants 11, 12, and/or 114), propane, nitrogen, and the like. Pulmonary formulations may include permeation enhancers such as fatty acids, and saccharides, chelating agents, enzyme inhibitors (*e.g.,* protease inhibitors), adjuvants (*e.g.,* glycocholate, surfactin, span 85, and nafamostat), preservatives (*e.g*., benzalkonium chloride or chlorobutanol), and ethanol (normally up to 5% but possibly up to 20%, by weight). Ethanol is commonly included in aerosol compositions as it can improve the function of the metering valve and in some cases also improve the stability of the dispersion.

Pulmonary formulations may also include surfactants which include, but are not limited to, bile salts and those described in U.S. Patent No. 6,524,557 and references therein. The surfactants described in U.S. Patent No. 6,524,557, *e.g.,* a C₈-C₁₆ fatty acid salt, a bile salt, a phospholipid, or alkyl saccharide are advantageous in that some of them also reportedly enhance absorption of the compound in the formulation.

Also suitable in the invention are dry powder formulations comprising a therapeutically effective amount of active compound blended with an appropriate carrier and adapted for use in connection with a dry-powder inhaler. Absorption enhancers that can be added to dry powder formulations include those described in U.S. Patent No. 6,632,456. PCT Publication No. WO 02/080884, describes new methods for the surface modification of powders. Aerosol formulations may include those described in U.S. Patent Nos. 5,230,884 and 5,292,499; PCT Publication Nos. WO 017/8694 and 01/78696; and U.S. Patent Application Publication No. 2003/019437, 2003/0165436; and PCT Publication No. WO 96/40089 (which includes vegetable oil). Sustained release formulations suitable for inhalation are described in U.S. Patent Application Publication Nos. 2001/0036481, 2003/0232019, and 2004/0018243 as well as in PCT Publication Nos. WO 01/13891, 02/067902, 03/072080, and 03/079885.

Pulmonary formulations containing microparticles are described in PCT Publication No. WO 03/015750, U.S. Patent Application Publication No. 2003/0008013, and PCT Publication No. WO 00/00176. Pulmonary formulations containing stable glassy state powder are described in U.S. Patent Application Publication No. 2002/0141945 and U.S. Patent No. 6,309,671. Other aerosol formulations are described in EP Patent No. 1338272, PCT Publication No. WO 90/09781, U.S. Patent Nos. 5,348,730 and 6,436,367, PCT Publication No. WO 91/04011, and U.S. Patent Nos. 6,294,153 and 6,290,987, which describe a liposomal based formulation that can be administered via aerosol or other means.

Powder formulations for inhalation are described in U.S. Patent Application Publication No. 2003/0053960 and PCT Publication No. WO 01/60341. The agents can be administered intranasally as described in U.S. Patent Application Publication No. 2001/0038824.

Solutions of medicament in buffered saline and similar vehicles are commonly employed to generate an aerosol in a nebulizer. Simple nebulizers operate on Bernoulli's principle and employ a stream of air or oxygen to generate the spray particles. More complex nebulizers employ ultrasound to create the spray particles. Both types are well known in the art and are described in standard textbooks of pharmacy such as Sprowls' American Pharmacy and Remington's The Science and Practice of Pharmacy.

Other devices for generating aerosols employ compressed gases, usually hydrofluorocarbons and chlorofluorocarbons, which are mixed with the medicament and any necessary excipients in a pressurized container. These devices are likewise described in standard textbooks such as Sprowls and Remington.

The agent can be incorporated into a liposome to improve half-life. The agent can also be conjugated to polyethylene glycol ("PEG") chains. Methods for pegylation and additional formulations containing PEG-conjugates (*i.e*., PEG-based hydrogels, PEG modified liposomes) can be found in Harris and Chess, Nature Reviews Drug Discovery 2:214-221, and the references therein. The agent can be administered via a nanocochleate or cochleate delivery vehicle (BioDelivery Sciences International). The agents can be delivered transmucosally (i.e., across a mucosal surface such as the vagina, eye, or nose) using formulations such as that described in U.S. Patent No. 5,204,108. The agents can be formulated in microcapsules as described in PCT Publication No. WO 88/01165. The agent can be administered intra-orally using the formulations described in U.S. Patent Application Publication No. 2002/0055496, PCT Publication No. WO 00/47203, and U.S. Patent No. 6,495,120. The agent can be delivered using nanoemulsion formulations described in PCT Publication No. WO 01/91728.

Another aspect of the present invention relates to a compound or composition of the invention for use in the treatment of a condition associated with an insufficient level of insulin secretion. This involves administering to a subject in need of treatment for a condition associated with an insufficient level of insulin secretion a compound or composition of the present invention.

In one embodiment, the treatment methods of the present invention are carried out under conditions effective to increase pancreatic beta cell mass in the subject to treat the subject for an insufficient level of insulin secretion.

In one embodiment, the compound or composition may be administered with or coincident with a TGFβ superfamily signaling pathway inhibitor. Suitable transforming growth factor beta (TGFβ) superfamily signaling pathway inhibitors are described in detail above.

In another embodiment, the compound or composition may be administered with or coincident with a glucagon-like peptide-1 receptor (GLP1R) agonist or a Dipeptidyl Peptidase IV (DDP4) inhibitor. Suitable glucagon-like peptide-1 receptor (GLP1R) agonists or a Dipeptidyl Peptidase IV (DDP4) inhibitors are described in detail above. In accordance with this embodiment, the administering is carried out under conditions effective to cause a synergistic increase in pancreatic beta cell mass in the subject to treat the subject for an insufficient level of insulin secretion.

As used herein, a condition associated with an insufficient level of insulin secretion means a condition where a subject produces a lower plasma level of insulin than is required to maintain normal glucose levels in the blood such that the subject with the condition associated with insufficient insulin secretion becomes hyperglycemic. In such a condition, the pancreatic beta cells of the afflicted subject secrete an insufficient level of insulin to maintain the presence of a normal concentration of glucose in the blood (*i.e*., normoglycemica).

According to one embodiment, one of the conditions associated with an insufficient level of insulin secretion is insulin resistance. Insulin resistance is a condition in which a subject's cells become less sensitive to the glucose-lowering effects of insulin. Insulin resistance in muscle and fat cells reduces glucose uptake (and, therefore, local storage of glucose as glycogen and triglycerides), whereas insulin resistance in liver cells results in reduced glycogen synthesis and storage and a failure to suppress glucose production and release into the blood. Insulin resistance normally refers to reduced glucose-lowering effects of insulin. However, other functions of insulin can also be affected. For example, insulin resistance in fat cells reduces the normal effects of insulin on lipids and results in reduced uptake of circulating lipids and increased hydrolysis of stored triglycerides. Increased mobilization of stored lipids in these cells elevates free fatty acids in the blood plasma. Elevated blood fatty-acid concentrations, reduced muscle glucose uptake, and increased liver glucose production all contribute to elevated blood glucose levels. If insulin resistance exists, more insulin needs to be secreted by the pancreas. If this compensatory increase does not occur, blood glucose concentrations increase and type II diabetes occurs.

According to another embodiment, one of the conditions associated with an insufficient level of insulin secretion is diabetes. Diabetes can be divided into two broad types of diseases: type I (T1D) and type II (T2D). The term "diabetes" also refers herein to a group of metabolic diseases in which patients have high blood glucose levels, including type I diabetes (T1D), type II diabetes (T2D), gestational diabetes, congenital diabetes, maturity onset diabetes (MODY), cystic fibrosis-related diabetes, hemochromatosis-related diabetes, drug-induced diabetes (*e.g*., steroid diabetes), and several forms of monogenic diabetes.

Thus, in one embodiment, the subject has been diagnosed as having one or more of type I diabetes (T1D), type II diabetes (T2D), gestational diabetes, congenital diabetes, maturity onset diabetes (MODY), cystic fibrosis-related diabetes, hemochromatosis-related diabetes, drug-induced diabetes, or monogenic diabetes.

According to another embodiment, a condition associated with an insufficient level of insulin secretion is metabolic syndrome. Metabolic syndrome is generally used to define a constellation of abnormalities that is associated with increased risk for the development of type II diabetes and atherosclerotic vascular disease. Related conditions and symptoms include, but are not limited to, fasting hyperglycemia (diabetes mellitus type II or impaired fasting glucose, impaired glucose tolerance, or insulin resistance), high blood pressure; central obesity (also known as visceral, male-pattern or apple-shaped adiposity), meaning overweight with fat deposits mainly around the waist; decreased HDL cholesterol; and elevated triglycerides.

In one embodiment, the subject has been diagnosed as having metabolic syndrome or insulin resistance.

Other conditions that may be associated with an insufficient level of insulin secretion include, without limitation, hyperuricemia, fatty liver (especially in concurrent obesity) progressing to non-alcoholic fatty liver disease, polycystic ovarian syndrome (in women), and acanthosis nigricans.

Related disorders may also be treated pursuant to the treatment methods of the present invention including, without limitation, any disease associated with a blood or plasma glucose level outside the normal range, preferably hyperglycemia. Consequently, the term "related disorders" includes impaired glucose tolerance (IGT), impaired fasting glucose (IFG), insulin resistance, metabolic syndrome, postprandial hyperglycemia, and overweight/obesity. Such related disorders can also be characterized by an abnormal blood and/or plasma insulin level.

The methods described herein may be carried out to treat a subject with conditions associated with beta cell failure or deficiency. Such conditions include, without limitation, type I diabetes (T1D), type II diabetes (T2D), gestational diabetes, congenital diabetes, maturity onset diabetes (MODY), cystic fibrosis-related diabetes, hemochromatosis-related diabetes, drug-induced diabetes, or monogenic diabetes. Drug induced diabetes relates to a condition that is caused through the use of drugs that are toxic to beta cells (e.g., steroids, antidepressants, second generation antipsychotics, and immunosuppressive. Exemplary immunosuppressive drugs include, but are not limited to, members of the cortisone family (*e.g.,* prednisone and dexamethasome), rapamycin/sirolimus, everolimus, and calciuneurin inhibitors (*e.g*., FK-506/tacrolimus).

Additional conditions associated with beta cell deficiency include, without limitation, pancreatectomy, partial pancreatectomy, pancreas transplantation, and pancreatic islet transplantation.

The methods described herein may be carried out to treat a subject at risk of developing Type II Diabetes. For example, a patient at risk of developing Type II Diabetes has pre-diabetes/metabolic syndrome. The patient at risk of developing Type II Diabetes may have been treated with a psychoactive drug, including but not limited to a selective serotonin reuptake inhibitors ("SSRI") for depression, obsessive compulsive disorder ("OCD"), etc.

In carrying out treatment, a compound of Formula (I) or composition containing such compound and a TGFβ superfamily signaling pathway inhibitor are administered under conditions effective to increase pancreatic beta cell mass in the subject to treat the subject for a condition associated with an insufficient level of insulin secretion.

A compound or composition described herein and/or TGFβ superfamily signaling pathway inhibitor may be administered to increase pancreatic beta cell mass in the subject, which will result in an increased level of insulin secretion in the subject.

The compound and/or composition and TGFβ superfamily signaling pathway inhibitor may be formulated as separate pharmaceutical compositions or a single pharmaceutical composition comprising both the compound of formula (I) and TGFβ superfamily signaling pathway inhibitor. Such pharmaceutical composition(s) may comprise a therapeutically effective amount of the compound of formula (I) and/or TGFβ superfamily signaling pathway inhibitor.

Thus, a combination or combinatorial therapy or treatment of a compound of formula (I) and TGFβ superfamily signaling pathway inhibitor may be administered. The terms "combination" or "combinatorial therapy" or "combinatory treatment" mean a treatment where at least two compounds are co-administered to a subject to cause a biological effect, in this case a synergistic effect. In a combinatorial therapy, the at least two drugs may be administered together or separately, at the same time or sequentially. Simultaneous administration is not required, as long as the drugs produce a synergistic effect in the subject to improve the subject's conditions. Also, the at least two drugs may be administered through different routes and protocols. As a result, although they may be formulated together, the drugs of a combination may also be formulated separately.

A further aspect relates to a compound of formula (I) for use in the treatment of a neurological disorder. This method involves administering to a subject in need of treatment for a neurological disorder a compound of formula (I) under conditions effective to treat the subject for the condition.

The subject may have diabetes and/or has been diagnosed as having one or more of Down's Syndrome and a neurodegenerative disease.

In carrying out the treatment methods, administering of compounds to a subject may involve administering pharmaceutical compositions containing the compound(s) (*i.e*., a DYRK1A inhibitor of formula (I) and TGFβ superfamily signaling pathway inhibitor) in therapeutically effective amounts, which means an amount of compound effective in treating the stated conditions and/or disorders in the subject. Such amounts generally vary according to a number of factors well within the purview of ordinarily skilled artisans. These include, without limitation: the particular subject, as well as its age, weight, height, general physical condition, and medical history, the particular compound used, as well as the carrier in which it is formulated and the route of administration selected for it; the length or duration of treatment; and the nature and severity of the condition being treated.

Administering typically involves administering pharmaceutically acceptable dosage forms, which means dosage forms of compounds described herein and includes, for example, tablets, dragees, powders, elixirs, syrups, liquid preparations, including suspensions, sprays, inhalants tablets, lozenges, emulsions, solutions, granules, capsules, and suppositories, as well as liquid preparations for injections, including liposome preparations. Techniques and formulations generally may be found in Remington's Pharmaceutical Sciences, Mack Publishing Co., Easton, Pa., latest edition.

In carrying out treatment methods, the drug (*i.e.,* a compound of formula (I) and, optionally, a TGFβ superfamily signaling pathway inhibitor) may be contained, in any appropriate amount, in any suitable carrier substance. The drug may be present in an amount of up to 99% by weight of the total weight of the composition. The composition may be provided in a dosage form that is suitable for the oral, parenteral (*e.g*., intravenously, intramuscularly), rectal, cutaneous, nasal, vaginal, inhalant, skin (patch), or ocular administration route. Thus, the composition may be in the form of, *e.g.,* tablets, capsules, pills, powders, granulates, suspensions, emulsions, solutions, gels including hydrogels, pastes, ointments, creams, plasters, drenches, osmotic delivery devices, suppositories, enemas, injectables, implants, sprays, or aerosols.

Pharmaceutical compositions may be formulated to release the active drug substantially immediately upon administration or at any predetermined time or time period after administration.

Controlled release formulations include (i) formulations that create a substantially constant concentration of the drug(s) within the body over an extended period of time; (ii) formulations that after a predetermined lag time create a substantially constant concentration of the drug(s) within the body over an extended period of time; (iii) formulations that sustain drug(s) action during a predetermined time period by maintaining a relatively, constant, effective drug level in the body with concomitant minimization of undesirable side effects associated with fluctuations in the plasma level of the active drug substance; (iv) formulations that localize drug(s) action by, *e.g*., spatial placement of a controlled release composition adjacent to or in the diseased tissue or organ; and (v) formulations that target drug(s) action by using carriers or chemical derivatives to deliver the drug to a particular target cell type.

Administration of drugs in the form of a controlled release formulation is especially preferred in cases in which the drug has (i) a narrow therapeutic index (*i.e.,* the difference between the plasma concentration leading to harmful side effects or toxic reactions and the plasma concentration leading to a therapeutic effect is small; in general, the therapeutic index ("TI") is defined as the ratio of median lethal dose (LD₅₀) to median effective dose (ED₅₀)); (ii) a narrow absorption window in the gastro-intestinal tract; or (iii) a very short biological half-life so that frequent dosing during a day is required in order to sustain the plasma level at a therapeutic level.

Any of a number of strategies can be pursued to obtain controlled release in which the rate of release outweighs the rate of metabolism of the drug in question. Controlled release may be obtained by appropriate selection of various formulation parameters and ingredients, including, *e.g*., various types of controlled release compositions and coatings. Thus, the drug is formulated with appropriate excipients into a pharmaceutical composition that, upon administration, releases the drug in a controlled manner (single or multiple unit tablet or capsule compositions, oil solutions, suspensions, emulsions, microcapsules, microspheres, nanoparticles, patches, and liposomes).

Thus, administering may be carried out orally, topically, transdermally, parenterally, subcutaneously, intravenously, intramuscularly, intraperitoneally, by intranasal instillation, by intracavitary or intravesical instillation, intraocularly, intraarterially, intralesionally, or by application to mucous membranes. Compounds may be administered alone or with suitable pharmaceutical carriers, and can be in solid or liquid form, such as tablets, capsules, powders, solutions, suspensions, or emulsions.

The subject may be a mammalian subject. In one embodiment, the subject is a human subject. Suitable human subjects include, without limitation, children, adults, and elderly subjects having a beta-cell and/or insulin deficiency.

The subject may be bovine, ovine, porcine, feline, equine, murine, canine, lapine, etc.

The administering step may increase the number of proliferating pancreatic beta cells in the subject by at least about 5%, 6%, 7%, or more.

Administering may increase glucose-stimulated insulin secretion in pancreatic beta cells of the subject.

The designation of a compound is meant to designate the compound per se, as well as any pharmaceutically acceptable salt, hydrate, isomer, racemate, ester, or ether thereof. The designation of a compound is meant to designate the compound as specifically designated per se, as well as any pharmaceutically acceptable salt thereof.

Within the context of the present disclosure, by "treating" it is meant preventive or curative treatment.

Treatment may designate, in particular, the correction, decrease in the rate of change, or reduction of an impaired glucose homeostasis. The level of glucose in blood fluctuates throughout the day. Glucose levels are usually lower in the morning, before the first meal of the day and rise after meals for some hours. Consequently, the term treatment includes the control of blood glucose level by increasing or decreasing blood glucose level depending on the condition of the subject and the daytime in order to reach normal glucose levels. The term treatment more particularly includes a temporary or persistent reduction of blood glucose level in a subject having diabetes or a related disorder. The term "treatment" or "treating" also designates an improvement in insulin release (*e.g.,* by pancreatic beta cells).

As used herein, the phrase "control of blood glucose level" refers to the normalization or the regulation of the blood or plasma glucose level in a subject having abnormal levels (*i.e*., levels that are below or above a known reference, median, or average value for a corresponding subject with a normal glucose homeostasis).

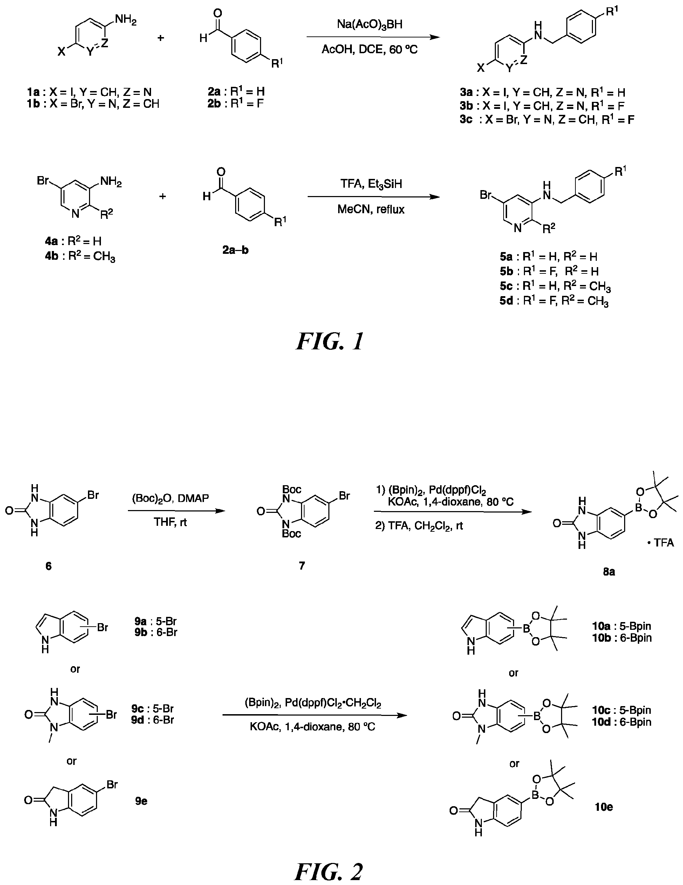

Compounds referred to herein in the examples are referenced by names and also numbers (*e.g*., **1a**). Structures corresponding to the numbers are identified in Figures 1-6. For example, compound (**1a**) is shown in Figure 1.

### EXAMPLES

### Example 1 - Synthesis of Pyridine Based DYRK1A Inhibitors

*General Experimental Conditions.* All reactions involving air-sensitive reagents were carried out with magnetic stirring and in oven-dried glassware with rubber septa under argon unless otherwise stated. All commercially available chemicals and reagent grade solvents were used without further purification unless otherwise specified. Thin-layer chromatography (TLC) was performed on Baker-flex^{®} silica gel plates (IB2-F) using UV-light (254 and 365 nm) detection or visualizing agents (ninhydrin or phosphomolybdic acid stain) and flash chromatography was carried out on silica gel (230-400 mesh) using Teledyne Isco CombiFlash^{®} Rf. NMR spectra were acquired at room temperature using a Bruker DRX-600 spectrometer at 600 MHz for ¹H and 150 MHz for ¹³C. Chemical shifts (δ) are given in parts per million (ppm) with reference to solvent signals [¹H-NMR: CDCl₃ (7.26 ppm), CD₃OD (3.30 ppm), DMSO-*d₆* (2.49 ppm); ¹³C-NMR: CDCl₃ (77.0 ppm), CD₃OD (49.0 ppm), DMSO-*d₆* (39.5 ppm)]. Signal patterns are reported as s (singlet), d (doublet), t (triplet), q (quartet), quin (quintet), sex (sextet), sep (septet), m (multiplet), br (broad), dd (doublet of doublets), dt (doublet of triplets), td (triplet of doublets), and tt (triplet of triplets). Coupling constants (*J*) are given in Hz. LCMS analysis was conducted on an Agilent Technologies G1969A high-resolution API-TOF mass spectrometer attached to an Agilent Technologies 1200 HPLC system. Samples were ionized by electrospray ionization (ESI) in positive mode and reported as *m*/*z* (relative intensity) for the molecular ion [M].

### Example 2 - General Procedure for the Preparation of N-Benzylhalopyridinamine-Method A

### N-Benzyl-5-iodopyridin-2-amine (3a)

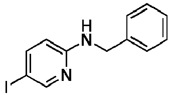

To a solution of 5-iodopyridin-2-amine (**1a**) (200.0 mg, 0.91 mmol) in anhydrous 1,2-dichloroethane (8 mL) was added benzaldehyde (**2a**) (0.09 mL, 0.91 mmol), sodium triacetoxyborohydride (250.5 mg, 1.18 mmol) and acetic acid (0.16 mL, 2.73 mmol) under nitrogen. The resulting mixture was stirred at 60 °C for 6.5 h. After being quenched with H₂O (5 mL), the aqueous layer was extracted with EtOAc (2 × 20 mL). The combined organic extracts were washed with brine, dried over anhydrous Na₂SO₄, filtered, and concentrated. The residue was purified by column chromatography on silica gel (EtOAc/hexane, 15:85 to 30:70) to afford **3a** (103.8 mg, 37%) as a white solid; **¹H NMR** (CDCl₃, 600 MHz) 8.19 (1 H, s), 7.58 (1 H, d, *J* = 9.8 Hz), 7.34 (4 H, s), 7.30-7.29 (1 H, m), 6.22 (1 H, d, *J* = 8.5 Hz), 5.19 (1 H, br), 4.47 (2 H, d, *J* = 4.9 Hz); HRMS (ESI-TOF) *m*/*z:* [M + H]⁺ calculated for C₁₂H₁₂IN₂ 311.0040; found 311.0018.

### N-(4-Fluorobenzyl)-5-iodopyridin-2-amine (3b)

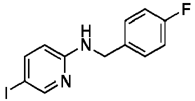

The above compound (**3b**) was prepared from **1a** and **2b** following Method A and purified by column chromatography on silica gel (EtOAc/hexane, 15:85 to 30:70) to give **3b** (43%) as a yellow solid; **¹H NMR** (CDCl₃, 600 MHz) 8.18 (1 H, s), 7.58 (1 H, d, *J* = 8.5 Hz), 7.29 (2 H, t, *J* = 8.5 Hz), 7.01 (2 H, t, *J* = 8.5 Hz), 6.21 (1 H, d, *J* = 8.5 Hz), 5.18 (1 H, br), 4.43 (2 H, d, *J* = 4.9 Hz); HRMS (ESI-TOF) *m*/*z:* [M + H]⁺ calculated for C₁₂H₁₁FIN₂ 328.9945; found 329.0146.

### 6-Bromo-N-(4-fluorobenzyl)pyridin-3-amine (3c)

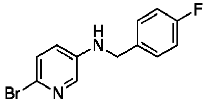

The above compound (**3c**) was prepared from **1b** and **2b** following Method A and purified by column chromatography on silica gel (EtOAc/hexane, 15:85 to 30:70) to give **3c** (36%) as a brown oil; **¹H NMR** (CDCl₃, 600 MHz) 7.77 (1 H, s), 7.31 (2 H, t, *J* = 7.3 Hz), 7.20 (1 H, d, *J* = 8.5 Hz), 7.04 (2 H, t, *J* = 8.5 Hz), 6.78 (1 H, dd, *J* = 8.5, 2.4 Hz), 4.29 (2 H, s); HRMS (ESI-TOF) *m*/*z*: [M + H]⁺ calculated for C₁₂H₁₁⁷⁹BrFN₂ and C₁₂H₁₁⁸¹BrFN₂ 281.0085, 283.0064; found 281.0051, 283.0031.

### Example 3 - General Procedure for the Preparation of N-Benzylhalopyrimidin-amine-Method B

### N-Benzyl-5-bromopyridin-3-amine (5a)

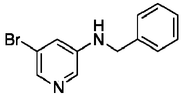

To a solution of 5-bromopyridin-3-amine (**4a**) (330.0 mg, 1.91 mmol) in anhydrous acetonitrile (10 mL) was added benzaldehyde (**2a**) (0.19 mL, 1.91 mmol), triethylsilane (1.62 mL, 10.11 mmol) and trifluoroacetic acid (0.82 mL, 10.68 mmol) under nitrogen. The resulting mixture was refluxed for 16 h. After being quenched with sat. NaHCO₃ (5 mL), water and EtOAc were added. The layers were separated. The combined organic phases were washed with brine, dried over anhydrous Na₂SO₄, filtered, and concentrated. The residue was purified by column chromatography on silica gel (EtOAc/hexane, 40:60) to afford **5a** (377.5 mg, 75%) as a yellow solid; **¹H NMR** (CDCl₃, 600 MHz) 7.96 (1 H, d, *J* = 2.4 Hz), 7.93 (1 H, d, *J* = 2.4 Hz), 7.37-7.29 (5 H, m), 7.00 (1 H, d, *J* = 2.4 Hz), 4.49 (1 H, br), 4.29 (2 H, s); HRMS (ESI-TOF) *m*/*z:* [M + H]⁺ calculated for C₁₂H₁₂⁷⁹BrN₂ and C₁₂H₁₂⁸¹BrN₂ 263.0178, 265.0158; found 263.0156, 265.0138.

### 5-Bromo-N-(4-fluorobenzyl)pyridin-3-amine (5b)

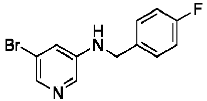

The above compound (**5b**) was prepared from **4a** and **2b** following Method B and purified by column chromatography on silica gel (EtOAc/hexane, 40:60) to give **5b** (92%) as a pale yellow solid; **¹H NMR** (CDCl₃, 600 MHz) 8.00 (1 H, s), 7.98 (1 H, s), 7.31 (2 H, dd, *J* = 8.5, 6.1 Hz), 7.06 (2 H, t, *J* = 8.5 Hz), 7.01 (1 H, s), 4.30 (3 H, s); HRMS (ESI-TOF) *m*/*z*: [M + H]⁺ calculated for C₁₂H₁₁⁷⁹BrFN₂ and C₁₂H₁₁⁸¹BrFN₂ 281.0084, 283.0064; found 281.0074, 283.0056.

### N-Benzyl-5-bromo-2-methylpyridin-3-amine (5c)

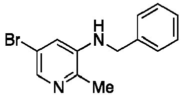

The above (**5c**) compound was prepared from **4b** and **2a** following Method B and purified by column chromatography on silica gel (EtOAc/hexane, 30:70) to give **5c** (45%) as a yellow oil; **¹H NMR** (CDCl₃, 600 MHz) 7.92 (1 H, s), 7.39-7.32 (5 H, m), 6.94 (1 H, s), 4.32 (2 H, d, *J* = 6.1 Hz), 4.02 (1 H, br), 2.36 (3 H, s); HRMS (ESI-TOF) m/z: [M + H]⁺ calculated for C₁₃H₁₄⁷⁹BrN₂ and C₁₃H₁₄⁸¹BrN₂ 277.0335, 279.0314; found 277.0335, 279.0318.

### 5-Bromo-N-(4-fluorobenzyl)-2methylpyridin-3-amine (5d)

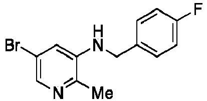

The above compound (**5d**) was prepared from **4b** and **2b** following Method B and purified by column chromatography on silica gel (EtOAc/hexane, 30:70) to give **5d** (34%) as a yellow oil; **¹H NMR** (CDCl₃, 600 MHz) 7.93 (1 H, s), 7.32 (2 H, dd, *J* = 8.5, 4.9 Hz), 7.06 (2 H, t, *J* = 8.5 Hz), 6.92 (1 H, s), 4.30 (2 H, d, *J* = 4.9 Hz), 3.98 (1 H, br), 2.37 (3 H, s); HRMS (ESI-TOF) *m*/*z*: [M + H]⁺ calculated for C₁₃H₁₃⁷⁹BrFN₂ and C₁₃H₁₃⁸¹ BrFN₂ 295.0220, 297.0274; found 295.0231, 297.0211.

The synthetic schemes for intermediates **3a-b**, and **5a-d**, discussed *supra,* are shown in Figure 1.

### Di-tert-butyl 5-bromo-2-oxo-1H-benzo[d]imidazole-1,3(2H)-dicarboxylate (7)

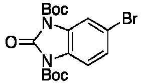

To a solution of 5-bromo-1*H*-benzo[*d*]imidazol-2(3*H*)-one (0.5 g, 2.36 mmol) in THF (10 mL) was added (Boc)₂O (2.1 g, 9.44 mmol) and DMAP (0.3 g, 2.44 mmol). The resulting mixture was stirred at room temperature for 12 h. After being quenched with H₂O (5 mL), the aqueous layer was extracted with EtOAc (2 × 20 mL). The combined organic extracts were washed with brine, dried over anhydrous Na₂SO₄, filtered, and concentrated. The residue was purified by column chromatography on silica gel (EtOAc/hexane, 25:75) to afford 7 (0.95 g, 98%) as a white solid; **¹H NMR** (CDCl₃, 600 MHz) 8.10 (1 H, s), 7.76 (1 H, d), 7.37 (1 H, d), 1.68 (9 H, s), 1.67 (9 H, s).

### 5-(4,4,5,5-Tetramethyl-1,3,2-dioxaborolan-2-yl)-1H-benzo[d]imidazol-2(3H)-one trifluoroacetate salt (8a)

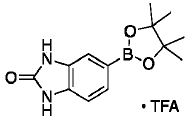

To a solution of di-tert-butyl 5-bromo-2-oxo-1*H*-benzo[*d*]imidazole-1,3(2*H*)-dicarboxylate (**7**) 0.9 g, 2.18 mmol), bis(pinacolato)diboron (1.1 g, 4.36 mmol) and Pd(dppf)Cl₂ (90 mg, 0.11 mmol) in 1,4-dioxane (15 mL) was added KOAc (0.65 g, 6.54 mmol) under nitrogen. The resulting mixture was stirred at 80 °C for 12 h. The reaction was allowed to cool to room temperature and filtered through a celite pad. EtOAc and water were added. The layers were separated. The combined organic phases were washed with brine, dried over anhydrous Na₂SO₄, filtered, and concentrated. The residue was purified by column chromatography on silica gel (EtOAc/hexane, 10:90) to afford intermediate di-tert-butyl 2-oxo-5-(4,4,5,5-tetramethyl-1,3,2-dioxaborolan-2-yl)-1*H*-benzo[*d*]imidazole-1,3(2*H*)-dicarboxylate as a white solid. To a solution of intermediate in CH₂Cl₂ (10 mL) was added TFA (2.5 mL). The reaction mixture was stirred at room temperature for 12 h. The solvent was removed *in vacuo* and the product (**8a**) was used in next step without further purification; **¹H NMR** (DMSO-*d₆*, 600 MHz) 10.73 (1 H, s), 10.60 (1 H, s), 7.24 (1 H, d, *J* = 8.5 Hz), 7.13 (1 H, s), 6.88 (1 H, d, *J* = 7.3 Hz), 1.23 (12 H, s). HRMS (ESI-TOF) *m*/*z:* [M + H]⁺ calculated for C₁₃H₁₈BN₂O₃ 261.1405; found 261.1404.

### Example 4 - General Procedure for the Preparation of Phenylboronic Acid Pinacol Ester-Method C

### 5-(4,4,5,5-Tetramethyl-1,3,2-dioxaborolan-2-yl)-1H-indole (10a).

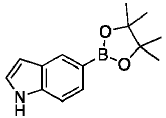

To a mixture of **9a** (150.0 mg, 0.76 mmol), bis(pinacolato)diboron (291.4 mg, 1.15 mmol), KOAc (225.2 mg, 2.30 mmol), and PdCl₂(dppf)•CH₂Cl₂ (65.5 mg, 0.07 mmol) was added anhydrous 1,4-dioxane (5 mL) under nitrogen. The reaction was put into a preheated oil bath (80 °C), and stirred for 16 h. After being quenched by the addition of water, the aqueous layer was extracted with EtOAc (2 × 15 mL). The combined organic extracts were washed with brine, dried over anhydrous Na₂SO₄, filtered, and concentrated. The residue was purified by column chromatography on silica gel (EtOAc/hexane, 15:85) to afford **10a** (149.4 mg, 80%) as a yellow solid; **¹H NMR** (CDCl₃, 600 MHz) 8.28 (1 H, s), 8.21 (1 H, s), 7.66 (1 H, d, *J* = 7.3 Hz), 7.38 (1 H, d, *J* = 8.5 Hz), 7.18 (1 H, s), 6.57 (1 H, s), 1.38 (12 H, s); HRMS (ESI-TOF) *m*/*z*: [M + H]⁺ calculated for C₁₄H₁₉BNO₂ 244.1503; found 244.1504.

### 6-(4,4,5,5-tetramethyl-1,3,2-dioxaborolan-2-yl)-1H-indole (10b)

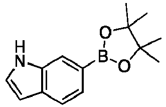

The above compound (**10b**) was prepared from **9b** following Method C and purified by column chromatography on silica gel (EtOAc/hexane, 15:85) to give **10b** (72%) as a white solid; **¹H NMR** (CDCl₃, 600 MHz) 8.30 (1 H, s), 7.93 (1 H, s), 7.68 (1 H, d, *J* = 7.3 Hz), 7.56 (1 H, d, *J* = 7.3 Hz), 6.58 (1 H, s), 1.40 (12 H, s); HRMS (ESI-TOF) *m*/*z*: [M + H]⁺ calculated for C₁₄H₁₉BNO₂ 244.1503; found 244.1571.

### 1-Methyl-5-(4,4,5,5-tetramethyl-1,3,2-dioxaborolan-2-yl)-1H-benzo[d]imidazol-2(3H)-one (10c)

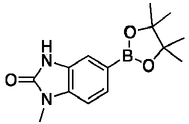

The above compound (**10c**) was prepared from **9c** following Method C and purified by column chromatography on silica gel (EtOAc/hexane, 40:60) to give **10c** (64%) as a light brown solid; **¹H NMR** (CDCl₃, 600 MHz) 10.21 (1 H, s), 7.58-7.57 (2 H, m), 6.98 (1 H, d, *J* = 8.5 Hz), 3.44 (3 H, s), 1.35 (12 H, s); HRMS (ESI-TOF) *m*/*z:* [M + H]⁺ calculated for C₁₄H₂₀BN₂O₃ 275.1561; found 275.1552.

### 1-Methyl-6-(4,4,5,5-tetramethyl-1,3,2-dioxaborolan-2-yl)-1H-benzo[d]imidazol-2(3H)-one (10d)

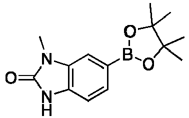

The above compound (**10d**) was prepared from **9d** following Method C and purified by column chromatography on silica gel (EtOAc/hexane, 40:60) to give **10d** (75%) as a white solid; **¹H NMR** (CDCl₃, 600 MHz) 10.79 (1 H, s), 7.60 (1 H, d, *J* = 7.3 Hz), 7.45 (1 H, s), 7.17 (1 H, d, *J* = 7.3 Hz), 3.48 (3 H, s), 1.38 (12 H, s); HRMS (ESI-TOF) *m*/*z*: [M + H]⁺ calculated for C₁₄H₂₀BN₂O₃ 275.1561; found 275.1561.

### 5-(4,4,5,5-Tetramethyl-1,3,2-dioxaborolan-2-yl)indolin-2-one (10e)

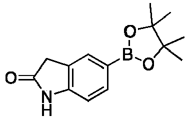

The above compound (**10e**) was prepared from **9e** following Method C and purified by column chromatography on silica gel (EtOAc/hexane, 50:50) to give **10e** (94%) as a brown solid; **¹H NMR** (CDCl₃, 600 MHz) 9.18 (1 H, s), 7.71 (1 H, d, *J* = 8.5 Hz), 7.68 (1 H, s), 6.92 (1 H, d, *J* = 7.3 Hz), 3.54 (2 H, s), 1.35 (12 H, s); HRMS (ESI-TOF) *m*/*z*: [M + H]⁺ calculated for C₁₄H₁₉BNO₃ 260.1453; found 260.1461.

The synthetic schemes for intermediates **7**, **8a**, and **10a-e**, discussed *supra,* are shown in Figure 2.

### Example 5 - General Procedure for the Preparation of Benzimidazolonyl/indolyl Benzyl Heterocyclic Amines-Method D

### 5-(6-(Benzylamino)pyridin-3-yl)-1H-benzo[d]imidazol-2(3H)-one (11a)

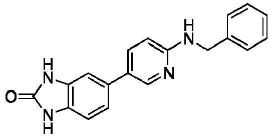

To a mixture of **8a** (26.6 mg, 0.07 mmol), **3a** (20.0 mg, 0.06 mmol), and Pd(PPh₃)₄ (7.4 mg, 0.006 mmol) was added anhydrous acetonitrile (1 mL) and anhydrous DMF (1 mL) under nitrogen. The reaction was stirred at room temperature for 10 min then 1 M Na₂CO₃ (0.19 mL, 0.19 mmol) was added. The mixture was put into a preheated oil bath (90 °C), and stirred for 16 h. After being quenched by the addition of water, the aqueous layer was extracted with EtOAc (2 × 10 mL). The combined organic extracts were washed with brine, dried over anhydrous Na₂SO₄, filtered, and concentrated. The residue was purified by column chromatography on silica gel (MeOH/CH₂Cl₂, 2.5:97.5 to 10:90) to afford **11a** (5.6 mg, 25%) as a yellow solid; **¹H NMR** (CD₃OD, 600 MHz) 8.14 (1 H, s), 7.68 (1 H, d, *J* = 9.8 Hz), 7.36 (2 H, d, *J* = 6.1 Hz), 7.31 (2 H, d, *J* = 6.1 Hz), 7.23-7.17 (3 H, m), 7.07 (1 H, d, *J* = 8.5 Hz), 6.61 (1 H, d, *J* = 8.5 Hz), 4.53 (2 H, s); HRMS (ESI-TOF) *m*/*z:* [M + H]⁺ calculated for C₁₉H₁₇N₄O 317.1397; found 317.1401.

### 5-(6-((4-Fluorobenzyl)amino)pyridin-3-yl)-1H-benzo[d]imidazol-2(3H)-one (11b)

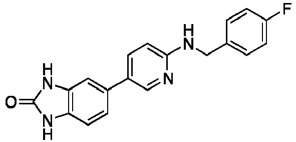

The above compound (**11b**) was prepared from **8a** and **3b** following Method D and purified by column chromatography on silica gel (MeOH/CH₂Cl₂, 2.5:97.5 to 10:90) to give **11b** (48%) as a white solid; **¹H NMR** (CD₃OD, 600 MHz) 8.14 (1 H, s), 7.68 (1 H, d, *J* = 8.5 Hz), 7.37 (1 H, s), 7.19-7.16 (2 H, m), 7.07 (1 H, d, *J* = 7.3 Hz), 7.03 (1 H, t, *J* = 8.5 Hz), 6.60 (1 H, d, *J* = 8.5 Hz), 4.51 (2 H, s); HRMS (ESI-TOF) *m*/*z:* [M + H]⁺ calculated for C₁₉H₁₆FN₄O 335.1303; found 335.1301.

### 5-(5-((4-Fluorobenzyl)amino)pyridin-2-yl)-1H-benzo[d]imidazol-2(3H)-one (11c)

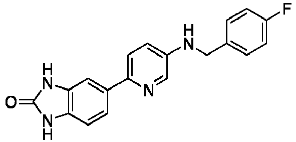

The above compound (**11c**) was prepared from **8a** and **3c** following Method D and purified by column chromatography on silica gel (MeOH/CH₂Cl₂, 5:95 to 10:90) to give **11c** (38%) as a yellow solid; **¹H NMR** (DMSO-*d₆*, 600 MHz) 10.61 (1 H, s), 10.58 (1 H, s), 8.01 (1 H, d, *J* = 2.4 Hz), 7.54 (1 H, d, *J* = 8.5 Hz), 7.46 (1 H, d, *J* = 8.5 Hz), 7.40 (2 H, dd, *J* = 8.5, 4.9 Hz), 7.16-7.12 (3 H, m), 6.93 (1 H, dd, *J* = 8.5, 2.4 Hz), 6.89 (1 H, d, *J* = 8.5 Hz), 6.57 (1 H, t, *J* = 6.1 Hz), 4.31 (2 H, d, *J* = 6.1 Hz); HRMS (ESI-TOF) *m*/*z:* [M + H]⁺ calculated for C₁₉H₁₆FN₄O 335.1303; found 335.1301.

### N-(4-fluorobenzyl)-5-(1H-indol-5-yl)pyridin-2-amine (11d)

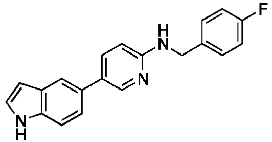

The above compound (**11d**) was prepared from **10a** and **3b** following Method D and purified by column chromatography on silica gel (EtOAc/hexane, 30:70 to 80:20) to give **11d** (39%) as a white solid; **¹H NMR** (CDCl₃, 600 MHz) 8.40 (1 H, s), 8.26 (1 H, s), 7.77 (2 H, s), 7.48 (1 H, d, *J* = 8.5 Hz), 7.39-7.36 (3 H, m), 7.29 (2 H, s), 7.07 (1 H, t, *J* = 8.5 Hz), 6.62 (1 H, s), 6.52 (1 H, d, *J* = 8.5 Hz), 5.28 (1 H, s), 4.57 (2 H, d, *J* = 3.7 Hz); HRMS (ESI-TOF) *m*/*z*: [M + H]⁺ calculated for C₂₀H₁₇FN₃ 318.1401; found 318.1411.

### N-(4-Fluorobenzyl)-5-(1H-indol-6-yl)pyridin-2-amine (11e)

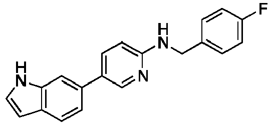

The above compound (**11e**) was prepared from **10b** and **3b** following Method D and purified by column chromatography on silica gel (EtOAc/hexane, 30:70 to 50:50) to give **11e** (46%) as a white solid; **¹H NMR** (CDCl₃, 600 MHz) 8.39 (1 H, s), 8.28 (1 H, s), 7.74 (1 H, d, *J* = 8.5 Hz), 7.68 (1 H, d, *J* = 7.3 Hz), 7.50 (1 H, s), 7.36 (2 H, s), 7.27-7.23 (2 H, m), 7.04 (2 H, t, *J* = 8.5 Hz), 6.57 (1 H, s), 6.48 (1 H, d, *J* = 8.5 Hz), 5.22 (1 H, br), 4.54 (2 H, d, *J* = 4.9 Hz); HRMS (ESI-TOF) *m*/*z:* [M + H]⁺ calculated for C₂₀H₁₇FN₃ 318.1401; found 318.1380.

### Example 6 - General Procedure for the Preparation of Benzimidazolonyl/indolyl benzyl heterocyclic amines-Method E

### 5-(1H-Benzo[d]imidazol-5-yl)-N-(4-fluorobenzyl)pyridin-2-amine (12a)

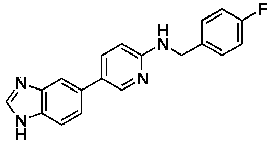

To a mixture of **10f** (30.0 mg, 0.12 mmol), **3b** (40.3 mg, 0.12 mmol), and Pd(PPh₃)₄ (13.9 mg, 0.01 mmol) in a microwave reaction vial was added anhydrous acetonitrile (1 mL) and anhydrous DMF (1 mL) and 1 M Na₂CO₃ (0.25 mL, 0.25 mmol) under nitrogen. The reaction mixture was irradiated under microwave conditions for 10 min at 160 °C. After being quenched by the addition of water, the aqueous layer was extracted with EtOAc (2 × 10 mL). The combined organic extracts were washed with brine, dried over anhydrous Na₂SO₄, filtered, and concentrated. The residue was purified by column chromatography on silica gel (MeOH/CH₂Cl₂, 5:95 to 10:90) to afford **12a** (8.3 mg, 21%) as a white solid; **¹H NMR** (CD₃OD, 600 MHz) 8.22 (1 H, s), 8.16 (1 H, s), 7.76 (1 H, d, *J* = 8.5 Hz), 7.70 (1 H, s), 7.64 (1 H, d, *J* = 8.5 Hz), 7.43 (1 H, d, *J* = 7.3 Hz), 7.38-7.37 (2 H, m), 7.03 (2 H, t, *J* = 8.5 Hz), 6.63 (1 H, d, *J* = 8.5 Hz), 4.52 (2 H, s); HRMS (ESI-TOF) *m*/*z:* [M + H]⁺ calculated for C₁₉H₁₆FN₄ 319.1354; found 319.1305.

### 6-(-5-(Benzylamino)pyridin-3-yl)-1-methyl-1H-benzo[d]imidazol-2(3H)-one (12b)

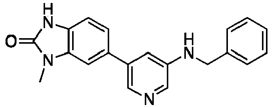

The above compound (**12b**) was prepared from **10d** and **5a** following Method E and purified by column chromatography on silica gel (MeOH/CH₂Cl₂, 5:95) to give **12b** (82%) as a white solid; **¹H NMR** (CD₃OD, 600 MHz) 7.98 (1 H, s), 7.86 (1 H, s), 7.40 (2 H, d, *J* = 7.3 Hz), 7.32 (2 H, t, *J* = 7.3 Hz), 7.24-7.20 (3 H, m), 7.16 (1 H, s), 7.09 (1 H, d, *J* = 8.5 Hz), 4.41 (2 H, s), 3.40 (3 H, s); HRMS (ESI-TOF) *m*/*z:* [M + H]⁺ calculated for C₂₀H₁₉N₄O 331.1553; found 331.1559.

### 5-(5-(Benzylamino)pyridin-3-yl)-1-methyl-1H-benzo[d]imidazol-2(3H)-one (12c)

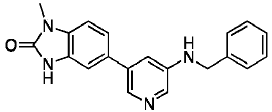

The above compound (**12c**) was prepared from **10c** and **5a** following Method E and purified by column chromatography on silica gel (MeOH/CH₂Cl₂, 5:95) to give **12c** (70%) as a white solid; **¹H NMR** (CD₃OD, 600 MHz) 7.98 (1 H, s), 7.90 (1 H, s), 7.43 (2 H, d, *J=* 8.5 Hz), 7.36 (2 H, t, *J* = 7.3 Hz), 7.30-7.25 (2 H, m), 7.24 (1 H, s), 7.20-7.17 (2 H, m), 4.45 (2 H, s), 3.44 (3 H, s); HRMS (ESI-TOF) *m*/*z:* [M + H]⁺ calculated for C₂₀H₁₉N₄O 331.1553; found 331.1569.

### 5-(5-(Benzylamino)pyridin-3-yl)-1H-benzo[d]imidazol-2(3H)-one (13a)

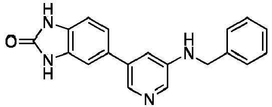

The above compound (**13a**) was prepared from **8b** and **5a** following Method E and purified by column chromatography on silica gel (MeOH/CH₂Cl₂, 5:95 to 10:90) to give **13a** (24%) as a yellow solid; **¹H NMR** (DMSO-*d₆*, 600 MHz) 10.70-10.69 (2 H, m), 7.96 (1 H, s), 7.91 (1 H, s), 7.39-7.38 (2 H, m), 7.33 (2 H, t, *J* = 7.3 Hz), 7.23 (1 H, t, *J* = 7.3 Hz), 7.13 (1 H, d, *J* = 6.1 Hz), 7.05 (2 H, s), 6.97 (1 H, d, *J* = 7.3 Hz), 6.59 (1 H, br), 4.36 (2 H, d, *J* = 4.9 Hz); HRMS (ESI-TOF) *m*/*z:* [M + H]⁺ calculated for C₁₉H₁₇N₄O 317.1397; found 317.1404.

### 5-(5-((4-Fluorobenzyl)amino)pyridin-3-yl)-1H-benzo[d]imidazol-2(3H)-one (13b)

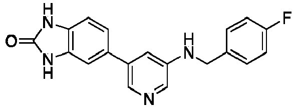

The above compound (**13b**) was prepared from **8b** and **5b** following Method E and purified by column chromatography on silica gel (MeOH/CH₂Cl₂, 5:95 to 10:90) to give **13b** (26%) as a yellow solid; **¹H NMR** (DMSO-*d₆*, 600 MHz) 10.70 (1 H, s), 10.68 (1 H, s), 7.96 (1 H, d, *J* = 2.4 Hz), 7.90 (1 H, d, *J* = 2.4 Hz), 7.42 (2 H, dd, *J* = 7.9, 6.1 Hz), 7.17-7.12 (3 H, m), 7.04 (2 H, d, *J* = 7.3 Hz), 6.97 (1 H, d, *J* = 8.5 Hz), 6.56 (1 H, t, *J* = 6.1 Hz), 4.35 (2 H, s); HRMS (ESI-TOF) *m*/*z*: [M + H]⁺ calculated for C₁₉H₁₆FN₄O 335.1303; found 335.1300.

### 5-(5-(Benzylamino)-6-methylpyridin-3-yl)- IH-benzo[d]imidazol-2(3H)-one (13c)

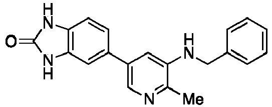

The above compound (**13c**) was prepared from **8b** and **5c** following Method E and purified by column chromatography on silica gel (MeOH/CH₂Cl₂, 5:95 to 10:90) to give **13c** (28%) as a yellow solid; **¹H NMR** (CD₃OD, 600 MHz) 7.83 (1 H, s), 7.39 (2 H, d, *J* = 7.3 Hz), 7.33 (2 H, d, *J* = 6.1 Hz), 7.22 (1 H, t, *J* = 7.3 Hz), 7.08-7.03 (3 H, m), 6.93 (1 H, s), 4.50 (2 H, s), 2.45 (3 H, s); HRMS (ESI-TOF) *m*/*z:* [M + H]⁺ calculated for C₂₀H₁₉N₄O 331.1553; found 331.1547.

### 5-(5-((4-Fluorobenzyl)amino)-6-methylpyridin-3-yl)-1H-benzo[d]imidazol-2(3H)-one (13d)

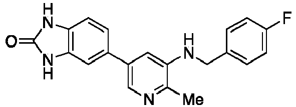

The above compound (**13d**) was prepared from **8b** and **5d** following Method E and purified by column chromatography on silica gel (MeOH/CH₂Cl₂, 5:95 to 10:90) to give **13d** (29%) as a yellow solid; **¹H NMR** (CD₃OD, 600 MHz) 7.84 (1 H, s), 7.41 (2 H, t, *J* = 7.3 Hz), 7.10-7.04 (5 H, m), 6.92 (1 H, s), 4.48 (2 H, s), 2.45 (3 H, s); HRMS (ESI-TOF) *m*/*z*: [M + H]⁺ calculated for C₂₀H₁₈FN₄O 349.1459; found 349.1476.

### 5-(5-(Benzylamino)pyridin-3-yl)indolin-2-one (13e)

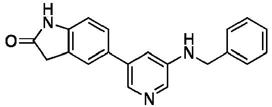

The above compound (**13e**) was prepared from **10e** and **5a** following Method E and purified by column chromatography on silica gel (MeOH/CH₂Cl₂, 5:95) to give **13e** (69%) as a pale yellow solid; **¹H NMR** (CD₃OD, 600 MHz) 7.93 (1 H, s), 7.85 (1 H, s), 7.42 (1 H, s), 7.40-7.36 (3 H, m), 7.32 (2 H, t, *J* = 7.9 Hz), 7.23 (1 H, t, *J* = 7.3 Hz), 7.12 (1 H, s), 6.94 (1 H, d, *J* = 7.3 Hz), 4.40 (2 H, s), 3.30 (2 H, s)*; HRMS (ESI-TOF) *m*/*z*: [M + H]⁺ calculated for C₂₀H₁₈N₃O 316.1444; found 316.1441.
* *A two protons peak is hidden in a methanol-d₄ peak.*

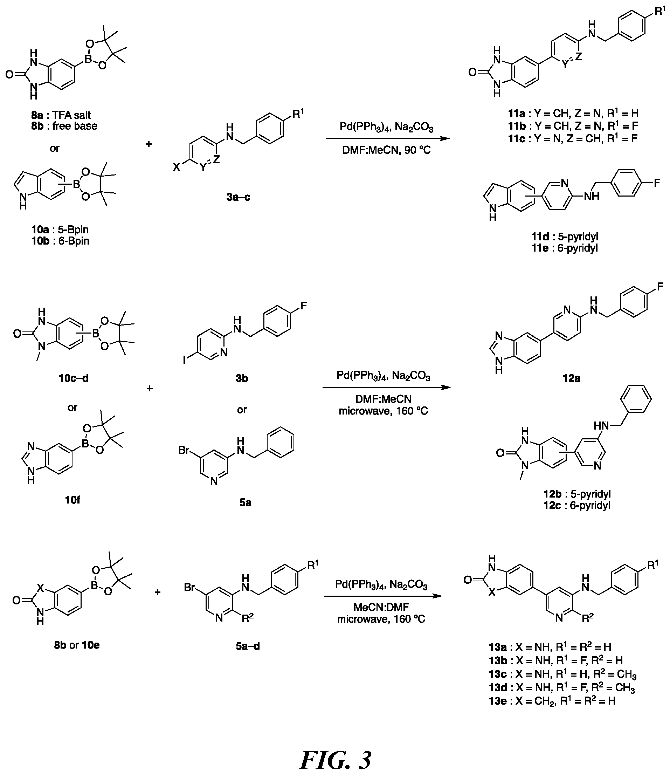

The synthetic schemes for compounds **11a-e**, **12a-c**, and **13a-e**, discussed *supra,* are shown in Figure 3.

### 5-Bromo-N-(pyridin-2-ylmethyl)pyridin-3-amine (15a)

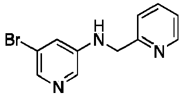

The above compound (**15a**) was prepared from **4a** and **14a** following Method B and purified by column chromatography on silica gel (EtOAc/hexane, 40:60 to 60:40) to give **15a** (70%) as a yellow oil; **¹H NMR** (CDCl₃, 600 MHz) 8.60 (1 H, d, *J* = 3.7 Hz), 8.04 (1 H, d, *J* = 2.4 Hz), 8.01 (1 H, s), 7.69 (1 H, t, *J* = 7.3 Hz), 7.30 (1 H, d, *J* = 7.3 Hz), 7.24 (1 H, t, *J* = 7.3 Hz), 7.08 (1 H, s), 5.21 (1 H, br), 4.43 (2 H, d, *J* = 4.9 Hz); HRMS (ESI-TOF) *m*/*z*: [M + H]⁺ calculated for C₁₁H₁₁⁷⁹BrN₃ and C₁₁H₁₁⁸¹BrN₃ 264.0131, 266.0111; found 264.0134, 266.0117.

### 5-Bromo-N-(pyridin-3-ylmethyl)pyridin-3-amine (15b)

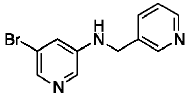

The above compound (**15b**) was prepared from **4a** and **14b** following Method B and purified by column chromatography on silica gel (EtOAc/hexane, 40:60 to 60:40) to give **15b** (78%) as a yellow solid; **¹H NMR** (CDCl₃, 600 MHz) 8.62 (1 H, s), 8.56 (1 H, d, *J=* 3.7 Hz), 8.03 (1 H, s), 7.97 (1 H, s), 7.67 (1 H, d, *J* = 7.3 Hz), 7.30 (1 H, dd, *J* = 9.2, 4.9 Hz), 7.02 (1 H, s), 4.37 (2 H, d, *J* = 4.9 Hz), 4.31 (1 H, br); HRMS (ESI-TOF) *m*/*z*: [M + H]⁺ calculated for C₁₁H₁₁⁷⁹BrN₃ and C₁₁H₁₁⁸¹BrN₃ 264.0131, 266.0111; found 264.0130, 266.0110.

### 5-Bromo-N-(pyridin-4-ylmethyl)pyridin-3-amine (15c)

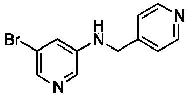

The above compound (**15c**) was prepared from **4a** and **14c** following Method B and purified by column chromatography on silica gel (MeOH/CH₂Cl₂, 5:95 to 10:90) to give **15c** (29%) as a yellow oil; **¹H NMR** (CDCl₃, 600 MHz) 8.59 (2 H, d, *J* = 6.1 Hz), 8.04 (1 H, s), 7.96 (1 H, d, *J* = 2.4 Hz), 7.27-7.26 (2 H, m), 6.96 (1 H, t, *J* = 2.4 Hz), 4.39 (3 H, s); HRMS (ESI-TOF) *m*/*z*: [M + H]⁺ calculated for C₁₁H₁₁⁷⁹BrN₃ and C₁₁H₁₁⁸¹BrN₃ 264.0131, 266.0111; found 264.0124, 266.0105.

### -5-Bomo-N-(2-fluorobenzyl)pyridin-3-amine (17a)

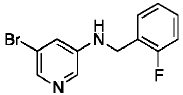

The above compound (**17a**) was prepared from **4a** and **16a** following Method B and purified by column chromatography on silica gel (EtOAc/hexane, 20:80 to 30:70) to give **17a** (98%) as a yellow oil; **¹H NMR** (CDCl₃, 600 MHz) 8.00 (1 H, s), 7.97 (1 H, d, *J* = 2.4 Hz), 7.34-7.27 (2 H, m), 7.13 (1 H, t, *J* = 7.3 Hz), 7.09 (1 H, t, *J* = 8.5 Hz), 7.04 (1 H, s), 7.34 (1 H, t, *J* = 7.3 Hz), 4.39 (2 H, d, *J* = 4.9 Hz), 4.25 (1 H, br); HRMS (ESI-TOF) *m*/*z*: [M + H]⁺ calculated for C₁₂H₁₁⁷⁹BrFN₂ and C₁₂H₁₁⁸¹BrFN₂ 281.0064, 283.0064; found 281.0428, 283.0411.

### 5-Bromo-N-(3-fluorobenzyl)pyridin-3-amine (17b)

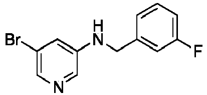

The above compound (**17b**) was prepared from **4a** and **16b** following Method B and purified by column chromatography on silica gel (EtOAc/hexane, 20:80 to 30:70) to give 17b (82%) as a yellow oil; **¹H NMR** (CDCl₃, 600 MHz) 8.00 (1 H, s), 7.94 (1 H, d, *J* = 2.4 Hz), 7.32 (1 H, q, *J* = 6.1 Hz), 7.11 (1 H, d, *J* = 7.3 Hz), 7.04 (1 H, d, *J* = 9.8 Hz), 6.99 (2 H, s), 4.39 (1 H, br), 4.33 (2 H, d, *J=* 6.1 Hz); HRMS (ESI-TOF) *m*/*z*: [M + H]⁺ calculated for C₁₂H₁₁⁷⁹BrFN₂ and C₁₂H₁₁⁸¹BrFN₂ 281.0064, 283.0064; found 281.0373, 283.0354.

### 5-Bromo-N-(2-chlorobenzyl)pyridin-3-amine (17c)

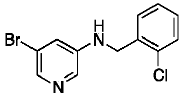

The above compound (**17c**) was prepared from **4a** and **16c** following Method B and purified by column chromatography on silica gel (EtOAc/hexane, 20:80 to 30:70) to give **17c** (96%) as a yellow solid; **¹H NMR** (CDCl₃, 600 MHz) 7.99 (1 H, s), 7.95 (1 H, s), 7.40 (1 H, t, *J* = 3.7 Hz), 7.34 (1 H, t, *J* = 3.7 Hz), 7.25-7.24 (2 H, m), 7.00 (1 H, s), 6.99 (2 H, s), 4.42 (3 H, s); HRMS (ESI-TOF) *m*/*z*: [M + H]⁺ calculated for C₁₂H₁₁⁷⁹BrClN₂ and C₁₂H₁₁⁸¹BrClN₂ 296.9789, 298.9760; found 297.0093, 299.0080.

### 5-Bromo-N-(3-chlorobenzyl)pyridin-3-amine (17d)

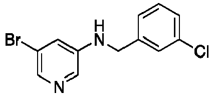

The above compound (**17d**) was prepared from **4a** and **16d** following Method B and purified by column chromatography on silica gel (EtOAc/hexane, 20:80 to 30:70) to give **17d** (94%) as a yellow oil; **¹H NMR** (CDCl₃, 600 MHz) 8.02 (1 H, s), 7.96 (1 H, s), 7.34-7.22 (4 H, m), 7.00 (1 H, s), 4.32 (2 H, d, *J* = 6.1 Hz), 4.24 (1 H, br); HRMS (ESI-TOF) *m*/*z*: [M + H]⁺ calculated for C₁₂H₁₁⁷⁹BrClN₂ and C₁₂H₁₁⁸¹BrClN₂ 296.9789, 298.9760; found 297.0080, 299.0076.

### 5-Bromo-N-(4-chlorobenzyl)pyridin-3-amine (17e)

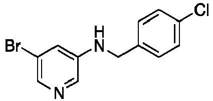

The above compound (**17e**) was prepared from **4a** and **16e** following Method B and purified by column chromatography on silica gel (EtOAc/hexane, 20:80 to 30:70) to give **17e** (88%) as a pale yellow solid; **¹H NMR** (CDCl₃, 600 MHz) 8.02 (1 H, s), 7.95 (1 H, s), 7.34 (2 H, d, *J* = 7.3 Hz), 7.28 (2 H, d, *J* = 7.3 Hz), 6.99 (1 H, s), 4.31 (2 H, d, *J* = 4.9 Hz), 4.21 (1 H, br); HRMS (ESI-TOF) *m*/*z*: [M + H]⁺ calculated for C₁₂H₁₁⁷⁹BrClN₂ and C₁₂H₁₁⁸¹BrClN₂ 296.9789, 298.9760; found 297.0053, 299.0033.

### 3-(((5-Bromopyridin-3-yl)amino)methyl)benzonitrile (17f)

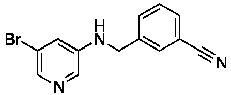

The above compound (**17f**) was prepared from **4a** and **16f** following Method B and purified by column chromatography on silica gel (EtOAc/hexane, 30:70 to 50:50) to give **17f** (88%) as a yellow oil; **¹H NMR** (CDCl₃, 600 MHz) 8.05 (1 H, s), 7.95 (1 H, s), 7.65 (1 H, s), 7.60 (2 H, t, *J* = 8.5 Hz), 7.49 (1 H, t, *J* = 7.3 Hz), 6.98 (1 H, s), 4.41 (2 H, d, *J* = 6.1 Hz), 4.32 (1 H, br); HRMS (ESI-TOF) *m*/*z*: [M + H]⁺ calculated for C₁₃H₁₁⁷⁹BrN₃ and C₁₃H₁₁⁸¹BrN₃ 288.0131, 290.0111; found 288.0142, 290.0120.

### 4-(((5-Bromopyridin-3-yl)amino)methyl)benzonitrile (17g)

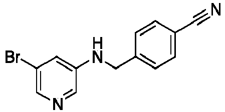

The above compound (**17g**) was prepared from **4a** and **16g** following Method B and purified by column chromatography on silica gel (EtOAc/hexane, 30:70 to 50:50) to give **17g** (89%) as a yellow solid; **¹H NMR** (CDCl₃, 600 MHz) 8.02 (1 H, s), 7.94 (1 H, s), 7.65 (2 H, d, *J* = 7.3 Hz), 7.45 (2 H, d, *J* = 7.3 Hz), 6.96 (1 H, s), 4.43 (3 H, s); HRMS (ESI-TOF) *m*/*z*: [M + H]⁺ calculated for C₁₃H₁₁⁷⁹BrN₃ and C₁₃H₁₁⁸¹BrN₃ 288.0131, 290.0111; found 288.0359, 290.0338.

### N-([1,1'-Biphenyl)-4-ylmethyl)-5-bromopyridin-3-amine (17h)

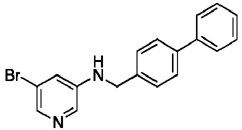

The above compound (**17h**) was prepared from **4a** and **16h** following Method B and purified by column chromatography on silica gel (EtOAc/hexane, 30:70 to 50:50) to give **17h** (84%) as a yellow solid; **¹H NMR** (CDCl₃, 600 MHz) 8.02 (1 H, s), 7.99 (1 H, s), 7.65 (1 H, s), 7.60-7.58 (4 H, m), 7.45 (2 H, t, *J* = 7.3 Hz), 7.41 (2 H, d, *J* = 8.5 Hz), 7.36 (1 H, t, *J* = 7.3 Hz), 7.05 (1 H, s), 4.37 (2 H, d, *J* = 6.1 Hz), 4.26 (1 H, br); HRMS (ESI-TOF) *m*/*z*: [M + H]⁺ calculated for C₁₈H₁₆⁷⁹BrN₂ and C₁₈H₁₆⁸¹BrN₂ 339.0492, 341.0471; found 339.0494, 341.0476.

### 5-Bromo-N-(4-methoxybenzyl)pyridin-3-amine (17i)

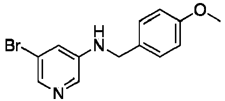

The above compound (**17i**) was prepared from **4a** and **16i** following Method B and purified by column chromatography on silica gel (EtOAc/hexane, 30:70 to 50:50) to give **17i** (71%) as a pale yellow solid; **¹H NMR** (CDCl₃, 600 MHz) 8.00 (1 H, s), 7.97 (1 H, d, *J* = 2.4 Hz), 7.28 (2 H, s, *J* = 7.3 Hz), 7.04 (1 H, t, *J* = 2.4 Hz), 6.92 (2 H, d, *J* = 8.5 Hz), 4.26 (2 H, d, *J* = 6.1 Hz), 4.20 (1 H, br), 3.83 (3 H, s); HRMS (ESI-TOF) *m*/*z*: [M + H]⁺ calculated for C₁₃H₁₄⁷⁹BrN₂₀ and C₁₃H₁₄⁸¹BrN₂₀ 293.0285, 295.0264; found 293.0435, 295.0418.

### 3-(((5-Bromopyridin-3-yl)amino)methyl)phenol (17j)

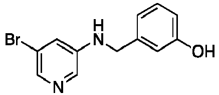

The above compound (17j) was prepared from **4a** and **16j** following Method B and purified by column chromatography on silica gel (EtOAc/hexane, 30:70 to 40:60) to give **17j** (79%) as a pale yellow solid; **¹H NMR** (CDCl₃, 600 MHz) 7.99 (1 H, s), 7.94 (1 H, s), 7.23 (1 H, t, *J* = 7.3 Hz), 7.01 (1 H, s), 6.89 (1 H, d, *J* = 7.3 Hz), 6.81 (1 H, s), 6.77 (1 H, d, *J* = 7.3 Hz), 4.29 (2 H, d, *J =* 4.9 Hz), 4.25 (1 H, br); HRMS (ESI-TOF) *m*/*z:* [M + H]⁺ calculated for C₁₂H₁₂⁷⁹BrN₂O and C₁₂H₁₂⁸¹BrN₂O 279.0128, 281.0108; found 279.0329, 281.0301.

### 5-Bromo-N-(3-methoxybenzyl)pyridin-3-amine (17k)

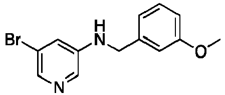

The above compound (17k) was prepared from **4a** and **16k** following Method B and purified by column chromatography on silica gel (EtOAc/hexane, 30:70 to 40:60) to give **17k** (95%) as a yellow oil; **¹H NMR** (CDCl₃, 600 MHz) 8.00 (1 H, s), 7.96 (1 H, d, *J* = 2.4 Hz), 7.28 (1 H, t, *J* = 8.5 Hz), 7.02 (1 H, s), 6.92 (1 H, d, *J* = 7.3 Hz), 6.88 (1 H, s), 6.84 (1 H, d, *J* = 8.5 Hz), 4.30 (2 H, d, *J* = 6.1 Hz), 4.21 (1 H, br), 3.81 (3 H, s); HRMS (ESI-TOF) *m*/*z:* [M + H]⁺ calculated for C₁₃H₁₄⁷⁹BrN₂O and C₁₃H₁₄⁸¹BrN₂O 293.0285, 295.0264; found 293.0482, 295.0464.

### 5-Bromo-N-(3-(trifluoromethoxy)benzyl)pyridin-3-amine (171)

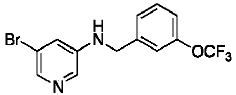

The above compound (171) was prepared from **4a** and **161** following Method B and purified by column chromatography on silica gel (EtOAc/hexane, 30:70 to 40:60) to give **171** (99%) as a yellow oil; **¹H NMR** (CDCl₃, 600 MHz) 8.02 (1 H, s), 7.96 (1 H, d, *J* = 2.4 Hz), 7.39 (1 H, t, *J* = 8.5 Hz), 7.28 (1 H, d, *J* = 7.3 Hz), 7.20 (1 H, s), 7.16 (1 H, d, *J* = 8.5 Hz), 7.00 (1 H, s), 4.36 (2 H, d, *J* = 4.9 Hz), 4.32 (1 H, br); HRMS (ESI-TOF) *m*/*z:* [M + H]⁺ calculated for C₁₃H₁₁⁷⁹BrF₃N₂O and C₁₃H₁₁⁸¹Br F₃N₂O 347.0002, 348.9981; found 346.9989, 348.9968.

### 5-Bromo-N-(naphthalen-1-ylmethyl)pyridin-3-amine (19a)

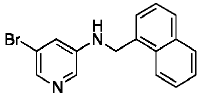

The above compound **(19a)** was prepared from **4a** and **18a** following Method B and purified by column chromatography on silica gel (EtOAc/hexane, 30:70 to 50:50) to give **19a** (89%) as a white solid; **¹H NMR** (CDCl₃, 600 MHz) 8.03 (1 H, s), 7.99 (1 H, d, *J* = 2.4 Hz), 7.91 (1 H, d, *J* = 8.5 Hz), 7.85 (1 H, d, *J* = 7.3 Hz), 7.57-7.53 (2 H, m), 7.49 (1 H, d, *J* = 6.1 Hz), 7.45 (1 H, t, *J* = 8.5 Hz), 7.09 (1 H, s), 4.72 (2 H, d, *J* = 6.1 Hz), 4.18 (1 H, br); HRMS (ESI-TOF) *m*/*z:* [M + H]⁺ calculated for C₁₆H₁₄⁷⁹BrN₂ and C₁₆H₁₄⁸¹BrN₂ 313.0335, 315.0315; found 313.0329, 315.0309.

### 5-Bromo-N-(naphthalen-2-ylmethyl)pyridin-3-amine (19b)

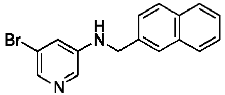

The above compound **(19b)** was prepared from **4a** and **18b** following Method B and purified by column chromatography on silica gel (EtOAc/hexane, 30:70) to give **19b** (78%) as a yellow solid; **¹H NMR** (CDCl₃, 600 MHz) 8.01 (1 H, s), 7.98 (1 H, s), 7.86-7.80 (3 H, m), 7.77 (1 H, s), 7.50-7.49 (2 H, m), 7.43 (1 H, d, *J* = 8.5 Hz), 7.04 (1 H, s), 4.45 (2 H, d, *J* = 6.1 Hz), 4.37 (1 H, br); HRMS (ESI-TOF) *m*/*z:* [M + H]⁺ calculated for C₁₆H₁₄⁷⁹BrN₂ and C₁₆H₁₄⁸¹BrN₂ 313.0335, 315.0315; found 313.0313, 315.0296.

### Example 7 - General Procedure for the Preparation of N-Naphthyliodopyrimidin-amine-Method F

### 5-lodo-N-(naphthalen-1-ylmethyl)pyridin-2-amine (21a)

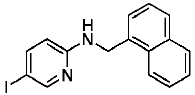

A solution of 5-iodopyridin-2-amine **(20a)** (0.16g, 0.64 mmol), 1-naphthaldehyde **(18a)** (0.1g, 0.64 mmol.) and acetic acid (4 mg, 0.064 mmol) in MeOH (2 mL) was stirred at 65 °C for 4 h. After cooled to room temperature, NaBH₃CN (0.1g, 1.96 mmol.) was then added. The reaction mixture was stirred at room temperature for 12 h. The reaction was quenched by addition of 2 N NaOH (1 mL) and extracted with CH₂Cl₂. The organic layer was separated, dried over anhydrous Na₂SO₄, filtered, and concentrated. The residue was purified by column chromatography on silica gel (EtOAc/hexane, 40:60) to give **21a** as solid (80 mg, 34%); **¹H NMR** (CDCl₃, 600 MHz) 8.25 (1 H, br), 8.04-8.02 (1 H, d), 7.93-7.92 (1 H, d), 7.85-7.84 (1 H, d), 7.68-7.66 (1 H, m), 7.59-7.50 (4 H, m), 7.46-7.43 (1 H, m), 6.34-6.32 (1 H, m), 4.97 (2 H, s); HRMS (ESI-TOF) *m*/*z:* [M + H]⁺ calculated for C₁₆H₁₄IN₂ 361.0196; found 361.0195.

### 6-Iodo-N-(naphthalen-1-ylmethyl)pyridin-3-amine (21b)

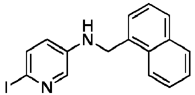

The above compound **(21b)** was prepared from **20b** and **18a** following Method F and purified by column chromatography on silica gel (EtOAc/hexane, 40:60) to give **21b** (44%) as a solid; **¹H NMR** (CDCl₃, 600 MHz) 8.06 (1 H, br), 8.01-8.00 (1 H, d), 7.93-7.92 (1 H, d), 7.86-7.85 (1 H, d), 7.59-7.54 (3 H, m), 7.51-7.44 (3 H, m), 6.72-6.70 (1 H, dd), 4.76 (2 H; s); HRMS (ESI-TOF) *m*/*z:* [M + H]⁺ calculated for C₁₆H₁₄IN₂ 361.0196; found 361.0188.

### 5-Bromo-N-phenylpyridin-3-amine (23)

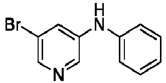

Compound **4a** (100.0 mg, 0.58 mmol), phenylboronic acid **(22)** (77.5 mg, 0.64 mmol) and copper (II) acetate (10.5 mg, 0.06 mmol) were dissolved in anhydrous 1,2-dichloroethane (4 mL). The reaction was stirred at room temperature for 1 h in opened-vial for 16 h then the second portion of phenylboronic acid **(22)** (77.5 mg, 0.64 mmol) was added. The reaction mixture was stirred at room temperature for another 24 h at room temperature. The reaction mixture was filtered through a celite pad. Water was added and the aqueous layer was extracted with EtOAc (2 × 10 mL). The combined organic extracts were washed with brine, dried over anhydrous Na₂SO₄, filtered, and concentrated. The residue was purified by column chromatography on silica gel (EtOAc/hexane, 20:80 to 30:70) to afford **23** (64.4 mg, 45%) as a yellow solid; **¹H NMR** (CDCl₃, 600 MHz) 8.27 (1 H, s), 8.19 (1 H, s), 7.55 (1 H, s), 7.37 (2 H, t, *J* = 7.3 Hz), 7.14 (2 H, d, *J* = 7.3 Hz), 7.10 (1 H, t, *J* = 7.3 Hz), 5.84 (1 H, br); HRMS (ESI-TOF) *m*/*z:* [M + H]⁺ calculated for C₁₁H₁₀⁷⁹BrN₂ and C₁₁H₁₀⁸¹BrN₂ 249.0022, 251.0002; found 249.0013, 250.9993.

### 5-Bromo-N-(1-phenylethyl)pyridin-3-amine (25)

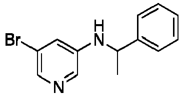

To a solution **4a** (100.0 mg, 0.58 mmol) in anhydrous DMF (3 mL) was added 60% NaH (30.0 mg, 0.75 mmol) at 0 °C under nitrogen and stirred for 30 min. **24** (0.09 mL, 0.69 mmol) was added. The reaction was stirred at 0 °C and slowly increased to room temperature then heated at 50 °C for 16 h. After being quenched by the addition of water, the aqueous layer was extracted with EtOAc (2 × 20 mL). The combined organic extracts were washed with brine, dried over anhydrous Na₂SO₄, filtered, and concentrated. The residue was purified by column chromatography on silica gel (EtOAc/hexane, 20:80 to 30:70) to afford **25** (87.5 mg, 55%) as a yellow oil; **¹H NMR** (CDCl₃, 600 MHz) 7.87 (1 H, s), 7.81 (1 H, s), 7.29-7.25 (4 H, m), 7.20-7.18 (1 H, m), 6.80 (1 H, s), 4.38 (1 H, quin, *J* = 6.1 Hz), 4.32 (1 H, br), 1.46 (3 H, d, *J* = 7.3 Hz); HRMS (ESI-TOF) *m*/*z:* [M + H]⁺ calculated for C₁₃H₁₄⁷⁹BrN₂ and C₁₃H₁₄⁸¹BrN₂ 277.0335, 279.0315; found 277.0719, 279.0703.

### Example 8 - General Procedure for the Preparation of N-Phenylbenzamide; N-(3-Bromophenyl)benzamide (27)-Method G

### N-(3-Bromophenyl)benzamide (27)

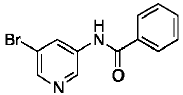

To a mixture of **4a** (150.0 mg, 0.867 mmol), HATU (310.0 mg, 0.815 mmol), and benzoic acid **(26)** (70 mg, 0.570 mmol) in anhydrous DMF (6 mL) was added *N,N-*diisopropylethylamine (0.18 mL, 1.01 mmol) under nitrogen. The reaction was stirred at room temperature for 10 min then 1 M Na₂CO₃ (0.19 mL, 0.19 mmol) was added. The reaction mixture was stirred at 80 °C for 16 h. After being quenched by the addition of water, the aqueous layer was extracted with EtOAc (2 × 20 mL). The combined organic extracts were washed with brine, dried over anhydrous Na₂SO₄, filtered, and concentrated. The residue was purified by column chromatography on silica gel (EtOAc/hexane, 30:70 to 40:60) to afford **27** (104.8 mg, 66%) as a yellow solid; **¹H NMR** (CDCl₃, 600 MHz) 8.63 (1 H, s), 8.56 (1 H, s), 8.52 (1 H, s), 8.35 (1 H, d, *J* = 2.4 Hz), 7.84 (2 H, d, *J* = 7.3 Hz), 7.54 (1 H, t, *J* = 7.3 Hz), 7.44 (2 H, t, *J* = 7.3 Hz); HRMS (ESI-TOF) *m*/*z:* [M + H]⁺ calculated for C₁₂H₁₀⁷⁹BrN₂O and C₁₂H₁₀⁸¹BrN₂O 276.9972, 278.9951; found 276.9979, 278.9957.

### 5-Bromo-N-phenethylpyridin-3-amine (29a)

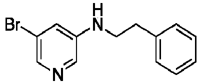

The above compound **(29a)** was prepared from **4a** and **28** following Method B and purified by column chromatography on silica gel (EtOAc/hexane, 30:70) to give **29a** (24%) as a yellow solid; **¹H NMR** (CDCl₃, 600 MHz) 7.97 (1 H, s), 7.88 (1 H, d, *J* = 2.4 Hz), 7.33 (2 H, t, *J* = 7.3 Hz), 7.26 (1 H, t, *J* = 7.3 Hz), 7.21 (2 H, d, *J* = 7.3 Hz), 6.99 (1 H, s), 3.88 (1 H, br), 3.39 (2 H, t, *J* = 7.3 Hz), 2.92 (2 H, t, *J* = 6.1 Hz); HRMS (ESI-TOF) *m*/*z:* [M + H]⁺ calculated for C₁₃H₁₄⁷⁹BrN₂ and C₁₃H₁₄⁸¹BrN₂ 277.0335, 279.0315; found 277.0478, 279.0461.

### 5-Bromo-N-(3-phenylpropyl)pyridin-3-amine (29b)

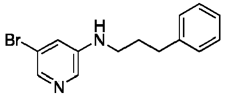

The above compound **(29b)** was prepared from **4a** and **28b** following Method B and purified by column chromatography on silica gel (EtOAc/hexane, 30:70) to give **29b** (44%) as a yellow solid; **¹H NMR** (CDCl₃, 500 MHz) 7.97 (1 H, s), 7.87 (1 H, s), 7.31 (2 H, t, *J* = 7.6 Hz), 7.24-7.19 (3 H, m), 6.93 (1 H, s), 3.72 (1 H, br), 3.13 (2 H, q, *J* = 6.1 Hz), 2.74 (2 H, t, *J* = 7.1 Hz), 1.97 (2 H, quin, *J* = 7.4 Hz); HRMS (ESI-TOF) *m*/*z:* [M + H]⁺ calculated for C₁₄H₁₆⁷⁹BrN₂ and C₁₄H₁₆⁸¹BrN₂ 291.0492, 293.0471; found 291.0481, 293.0472.

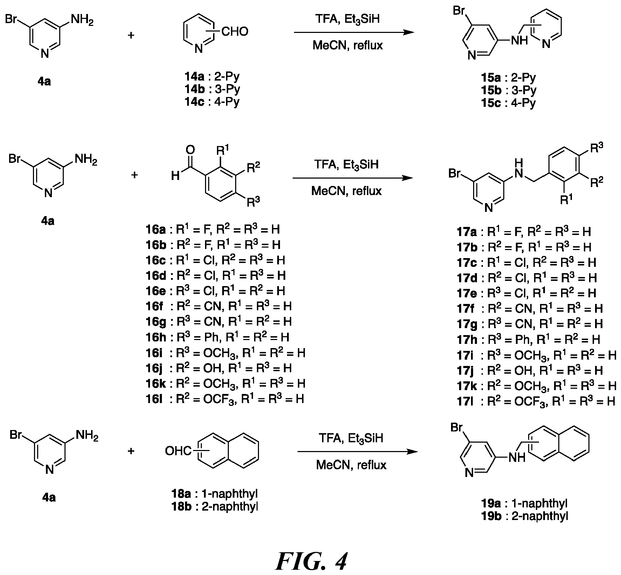

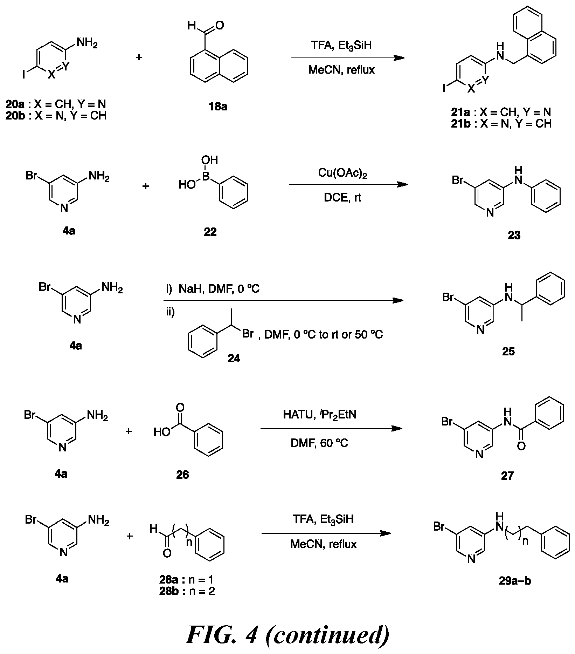

The synthetic schemes for intermediates **15a-c, 17a-1,** and **19a-b, 21a-b, 23, 25,** 27, and 29a-b, discussed *supra,* are shown in Figure 4.

### 5-(5-((2-Fluorobenzyl)amino)pyridin-3-yl)-1H-benzo[d]imidazol-2(3H)-one (30a)

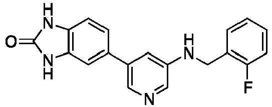

The above compound **(30a)** was prepared from **8b** and **17a** following Method E and purified by column chromatography on silica gel (MeOH/CH₂Cl₂, 5:95 to 10:90) to give **30a** (19%) as a yellow solid; **¹H NMR** (DMSO-d₆, 600 MHz) 10.70 (1 H, s), 10.69 (1 H, s), 8.52 (2 H, s), 7.98 (1 H, s), 7.93 (1 H, s), 7.42 (1 H, t, *J* = 8.5 Hz), 7.21-7.13 (3 H, m), 7.06 (2 H, s), 6.98 (1 H, d, *J* = 7.3 Hz), 6.52 (1 H, t, *J* = 6.1 Hz), 4.41 (2 H, d, *J* = 6.1 Hz); HRMS (ESI-TOF) *m*/*z:* [M + H]⁺ calculated for C₁₉H₁₆FN₄O 335.1303; found 335.1309.

### 5-(5-((3-Fluorobenzyl)amino)pyridin-3-yl)-1H-benzo[d]imidazol-2(3H)-one (30b)

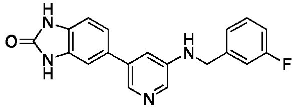

The above compound **(30b)** was prepared from **8b** and **17b** following Method E and purified by column chromatography on silica gel (MeOH/CH₂Cl₂, 5:95 to 10:90) to give **30b** (29%) as a yellow solid; **¹H NMR** (DMSO-d₆, 600 MHz) 10.70 (1 H, s), 10.69 (1 H, s), 7.97 (1 H, s), 7.90 (1 H, d, *J* = 2.4 Hz), 7.39-7.36 (1 H, m), 7.24-7.22 (1 H, m), 7.20-7.18 (1 H, m), 7.13 (1 H, d, *J* = 7.3 Hz), 7.07-7.04 (3 H, m), 6.97 (1 H, d, *J* = 7.3 Hz), 6.62-6.60 (1 H, m), 4.40 (2 H, d, *J* = 6.1 Hz); HRMS (ESI-TOF) *m*/*z:* [M + H]⁺ calculated for C₁₉H₁₆FN₄O 335.1303; found 335.1613.

### 5-(5-((2-Chlorobenzyl)amino)pyridin-3-yl)-1H-benzo[d]imidazol-2(3H)-one (30c)

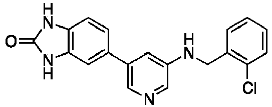

The above compound **(30c)** was prepared from **8b** and **17c** following Method E and purified by column chromatography on silica gel (MeOH/CH₂Cl₂, 5:95 to 10:90) to give **30c** (17%) as a yellow solid; **¹H NMR** (DMSO-*d₆*, 600 MHz) 10.69-10.68 (2 H, m), 7.99 (1 H, s), 7.90 (1 H, s), 7.47 (1 H, d, *J* = 8.5 Hz), 7.44 (1 H, d, *J* = 7.3 Hz), 7.31-7.27 (2 H, m), 7.13 (1 H, d, *J* = 6.1 Hz), 7.06 (1 H, s), 7.02 (1 H, s), 6.97 (1 H, d, *J* = 8.5 Hz), 6.60 (1 H, t, *J* = 4.5 Hz), 4.43 (2 H, d, *J =* 6.1 Hz); HRMS (ESI-TOF) *m*/*z:* [M + H]⁺ calculated for C₁₉H₁₆ClN₄O 351.1007; found 351.0997.

### 5-(5-((3-Chlorobenzyl)amino)pyridin-3-yl)-1H-benzo[d]imidazol-2(3H)-one (30d)

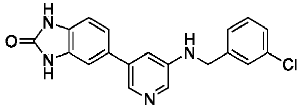

The above compound **(30d)** was prepared from **8b** and **17d** following Method E and purified by column chromatography on silica gel (MeOH/CH₂Cl₂, 5:95 to 10:90) to give **30c** (26%) as a yellow solid; **¹H NMR** (DMSO-*d₆*, 600 MHz) 10.70-10.69 (2 H, m), 7.98 (1 H, s), 7.90 (1 H, s), 7.44 (1 H, s), 7.36-7.34 (2 H, m), 7.30-7.29 (1 H, m), 7.13 (1 H, d, *J* = 7.3 Hz), 7.05 (2 H, d, *J* = 7.3 Hz), 6.97 (1 H, d, *J* = 7.9 Hz), 6.61-6.60 (1 H, m), 4.39 (2 H, d, *J* = 4.9 Hz); HRMS (ESI-TOF) *m*/*z:* [M + H]⁺ calculated for C₁₉H₁₆ClN₄O 351.1007; found 351.1009.

### 5-(5-((4-Chlorobenzyl)amino)pyridin-3-yl)-1H-benzo[d]imidazol-2(3H)-one (30e)

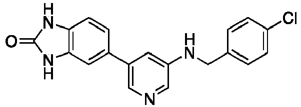

The above compound **(30e)** was prepared from **8b** and **17e** following Method E and purified by column chromatography on silica gel (MeOH/CH₂Cl₂, 5:95 to 10:90) to give **30d** (29%) as a yellow solid; **¹H NMR** (DMSO-*d₆*, 600 MHz) 10.70-10.69 (2 H, m), 7.96 (1 H, s), 7.89 (1 H, s), 7.41-7.39 (4 H, m), 7.13 (1 H, d, *J* = 7.3 Hz), 7.03 (2 H, d, *J* = 13.4 Hz), 6.97 (1 H, d, *J* = 7.3 Hz), 6.61(1 H, s), 4.39 (2 H, d, *J* = 6.1 Hz); HRMS (ESI-TOF) *m*/*z:* [M + H]⁺ calculated for C₁₉H₁₆ClN₄O 351.1007; found 351.1031.

### 3-(((5-(2-Oxo-2,3-dihydro-1H-benzo[d]imidazol-5-yl)pyridin-3-yl)amino)methyl)benzonitrile (30f)

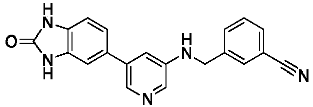

The above compound **(30f)** was prepared from **8b** and **17f** following Method E and purified by column chromatography on silica gel (MeOH/CH₂Cl₂, 5:95 to 10:90) to give **30f** (29%) as a yellow solid; **¹H NMR** (CD₃OD, 600 MHz) 7.99 (1 H, s), 7.86 (1 H, d, *J* = 2.4 Hz), 7.76 (1 H, s), 7.72 (1 H, d, *J* = 7.3 Hz), 7.62 (1 H, d, *J* = 7.3 Hz), 7.52 (1 H, t, *J* = 7.3 Hz), 7.21-7.19 (2 H, m), 7.14 (1 H, s), 7.10 (1 H, d, *J* = 8.5 Hz), 4.49 (2 H, s); HRMS (ESI-TOF) *m*/*z:* [M + H]⁺ calculated for C₂₀H₁₆N₅O 342.1349; found 342.1348.

### 4-(((5-(2-Oxo-2,3-dihydro-1H-benzo[d]imidazol-5-yl)pyridin-3-yl)amino)methyl)benzonitrile (30g)

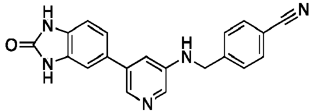

The above compound **(30g)** was prepared from **8b** and **17i** following Method E and purified by column chromatography on silica gel (MeOH/CH₂Cl₂, 5:95 to 10:90) to give **30g** (33%) as a yellow solid; **¹H NMR** (DMSO-*d₆*, 600 MHz) 10.70 (1 H, s), 10.69 (1 H, s), 7.98 (1 H, d, *J* = 2.4 Hz), 7.89 (1 H, d, *J* = 1.8 Hz), 7.80 (2 H, d, *J* = 7.3 Hz), 7.57 (2 H, d, *J* = 7.3 Hz), 7.12 (1 H, d, *J* = 9.8 Hz), 7.03 (1 H, s), 7.02 (1 H, t, *J*= 2.4 Hz), 6.97 (1 H, d, *J* = 8.5 Hz), 6.71 (1 H, t, *J* = 6.1 Hz), 4.48 (2 H, d, *J* = 6.1 Hz); HRMS (ESI-TOF) *m*/*z:* [M + H]⁺ calculated for C₂₀H₁₆N₅O 342.1349; found 342.1348.

### 5-(5-(([1,1'-Biphenyl]-4-ylmethyl)amino)pyridin-3-yl)-1H-benzo[d]imidazol-2(3H)-one (30h)

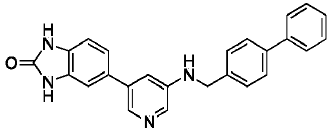

The above compound **(30h)** was prepared from **8b** and **17j** following Method E and purified by column chromatography on silica gel (MeOH/CH₂Cl₂, 5:95 to 10:90) to give 30h (18%) as a yellow solid; **¹H NMR** (DMSO-d₆, 600 MHz) 10.70 (1 H, s), 10.68 (1 H, s), 7.96 (1 H, d, *J* = 3.0 Hz), 7.93 (1 H, d, *J* = 2.4 Hz), 7.63 (4 H, d, *J* = 6.1 Hz), 7.47 (2 H, d, *J* = 8.5 Hz), 7.43 (2 H, t, *J* = 7.3 Hz), 7.33 (1 H, t, *J* = 8.5 Hz), 7.14 (1 H, dd, *J* = 7.3, 2.4 Hz), 7.07 (1 H, d, *J* = 6.1 Hz), 6.97 (1 H, d, *J* = 7.3 Hz), 6.62 (1 H, t, *J* = 6.1 Hz), 4.41 (2 H, d, *J* = 6.1 Hz); HRMS (ESI-TOF) *m*/*z:* [M + H]⁺ calculated for C₂₅H₂₁N₄O 393.1710; found 393.1697.

### 5-(5-((4-Methoxybenzyl)amino)pyridin-3-yl)-1H-benzo[d]imidazol-2(3H)-one (30i)

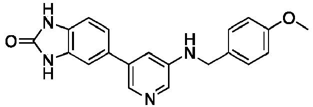

The above compound **(30i)** was prepared from **8b** and **17k** following Method E and purified by column chromatography on silica gel (MeOH/CH₂Cl₂, 5:95 to 10:90) to give 30i (22%) as a pale yellow solid; **¹H NMR** (DMSO-d₆, 600 MHz) 10.73-10.71 (2 H, m), 7.95 (1 H, s), 7.90 (1 H, s), 7.30 (2 H, d, *J* = 8.5 Hz), 7.13 (1 H, d, *J* = 7.3 Hz), 7.05 (1 H, s), 7.03 (1 H, s), 6.97 (1 H, d, *J* = 7.3 Hz), 6.89 (2 H, d, *J* = 7.3, 2.4 Hz), 6.50 (1 H, t, *J* = 6.1 Hz), 4.27 (2 H, d, *J* = 4.9 Hz), 3.71 (3 H, s); HRMS (ESI-TOF) *m*/*z:* [M + H]⁺ calculated for C₂₀H₁₉N₄O₂ 347.1503; found 347.1667.

### 5-(5-((1-Phenylethyl)amino)pyridin-3-yl)-1H-benzo[d]imidazol-2(3H)-one (30j)

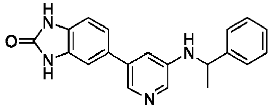

The above compound **(30j)** was prepared from **8b** and **25** following Method E and purified by column chromatography on silica gel (MeOH/CH₂Cl₂, 5:95 to 10:90) to give **30j** (30%) as a pale yellow solid; **¹H NMR** (CD₃OD, 600 MHz) 7.87 (1 H, s), 7.78 (1 H, d, *J* = 2.4 Hz), 7.39 (2 H, d, *J* = 6.7 Hz), 7.31 (2 H, t, *J* = 7.3 Hz), 7.20 (2 H, t, *J* = 7.3 Hz), 7.11-7.10 (2 H, m), 7.06 (1 H, d, *J* = 8.5 Hz), 7.02 (1 H, s), 4.54 (1 H, q, *J* = 6.1 Hz), 1.53 (3 H, d, *J* = 6.1 Hz); HRMS (ESI-TOF) *m*/*z:* [M + H]⁺ calculated for C₂₀H₁₉N₄O 331.1553; found 331.1968.

### 5-(5-(Phenethylamino)pyridin-3-yl)-1H-benzo[d]imidazol-2(3H)-one (30k)

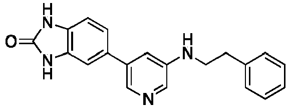

The above compound **(30k)** was prepared from **8b** and **29** following Method E and purified by column chromatography on silica gel (MeOH/CH₂Cl₂, 5:95 to 10:90) to give **30k** (36%) as a yellow solid; **¹H NMR** (DMSO-d₆, 600 MHz) 10.71 (2 H, s), 7.97 (1 H, s), 7.91 (1 H, s), 7.29 (3 H, s), 7.19 (2 H, d, *J* = 7.3 Hz), 7.11 (1 H, s), 7.04 (1 H, s), 6.98 (1 H, d, *J* = 8.5 Hz), 6.02 (1 H, s), 3.34 (2 H, m), 2.86 (2 H, t, *J* = 6.1 Hz); HRMS (ESI-TOF) *m*/*z:* [M + H]⁺ calculated for C₂₀H₁₉N₄O 331.1553; found 331.1548.

### N-(5-(2-Oxo-2,3-dihydro-1H-benzo[d]imidazol-5-yl)pyridin-3-yl)benzamide (301)

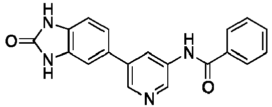

The above compound **(301)** was prepared from **8b** and **27** following Method E and purified by column chromatography on silica gel (MeOH/CH₂Cl₂, 10:90) to give **301** (36%) as a yellow solid; **¹H NMR** (DMSO-*d₆*, 600 MHz) 10.82 (1 H, s), 10.79 (1 H, s), 10.52 (1 H,s), 8.89 (1 H, s), 8.57 (1 H, s), 8.43 (1 H, s), 8.00 (2 H, d, *J* = 6.1 Hz), 7.64-7.56 (3 H, m), 7.28 (1 H, d, *J* = 7.3 Hz), 7.19 (1H, s), 7.05 (1 H, d, *J* = 7.3 Hz); HRMS (ESI-TOF) *m*/*z:* [M + H]⁺ calculated for C₁₉H₁₅N₄O₂ 331.1190; found 331.1356.

### 5-(5-(Phenylamino)pyridin-3-yl)-1H-benzo[d]imidazol-2(3H)-one (30m)

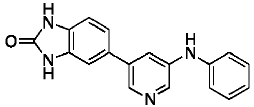

The above compound **(30m)** was prepared from **8b** and **23** following Method E and purified by column chromatography on silica gel (MeOH/CH₂Cl₂, 10:90) to give **30m** (27%) as a brown solid; **¹H NMR** (DMSO-*d₆*, 600 MHz) 10.73 (2 H, s), 8.43 (1 H, s), 8.28-8.24 (2 H, m), 7.55 (1 H, s), 7.28 (2 H, t, *J* = 7.3 Hz), 7.21 (1 H, d), 7.15-7.13 (3 H, m), 7.01 (1 H, d, *J* = 8.5 Hz), 6.90 (1 H, t, *J* = 7.3 Hz); HRMS (ESI-TOF) *m*/*z:* [M + H]⁺ calculated for C₁₈H₁₅N₄O 303.1240; found 303.1246.

### 1-Methyl-6-(5-((1-phenylethyl)amino)pyridin-3-yl)-1H-benzo[d]imidazol-2(3H)-one (30n)

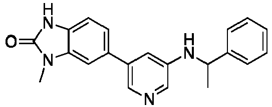

The above compound **(30n)** was prepared from **10d** and **25** following Method E and purified by column chromatography on silica gel (MeOH/CH₂Cl₂, 5:95) to give **30n** (68%) as a pale yellow solid; **¹H NMR** (DMSO-*d₆*, 600 MHz) 10.90 (1 H, s), 7.98 (1 H,s), 7.84 (1 H, s), 7.41 (2 H, d, *J* = 7.3 Hz), 7.30 (2 H, t, *J* = 7.3 Hz), 7.19-7.18 (2 H, m), 7.11 (1 H, d, *J* = 8.5 Hz), 7.00-6.99 (2 H, m), 6.52 (1 H, d, *J* = 6.1 Hz), 4.61 (1 H, quin, *J* = 7.3 Hz), 3.29 (3 H, s), 1.45 (3 H, d, *J* = 6.1 Mz); HRMS (ESI-TOF) *m*/*z:* [M + H]⁺ calculated for C₂₁H₂₁N₄O 345.1710; found 345.1735.

### 1-Methyl-6-(5-((1-phenylethyl)amino)pyridin-3-yl)-1H-benzo[d]imidazol-2(3H)-one (30o)

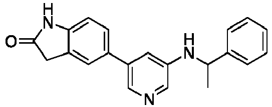

The above compound **(30o)** was prepared from **10e** and **25** following Method E and purified by column chromatography on silica gel (MeOH/CH₂Cl₂, 5:95) to give **30o** (58%) as a light brown solid; **¹H NMR** (DMSO-*d₆*, 600 MHz) 10.45 (1 H, s), 7.90 (1 H,s), 7.84 (1 H, s), 7.39 (2 H, d, *J* = 7.3 Hz), 7.34-7.28 (4 H, m), 7.18 (1 H, t, *J* = 7.3 Hz), 6.93 (1 H, s), 6.84 (1 H, d, *J* = 8.5 Hz), 6.50 (1 H, d, *J* = 6.1 Hz), 4.60 (1 H, quin, *J* = 6.1 Hz), 3.50 (2 H, s), 1.44 (3 H, d, *J* = 7.3 Mz); HRMS (ESI-TOF) *m*/*z:* [M + H]⁺ calculated for C₂₁H₂₀N₃O 330.1601; found 330.1628.

### 5-(5-((3-Phenylpropyl)amino)pyridin-3-yl)-1H-benzo[d]imidazol-2(3H)-one (30p)

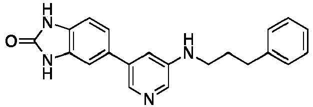

The above compound **(30p)** was prepared from **8b** and **29b** following Method E and purified by column chromatography on silica gel (MeOH/CH₂Cl₂, 5:95 to 10:90) to give **30p** (28%) as a light brown solid; **¹H NMR** (DMSO-d₆, 600 MHz) 10.67 (2 H, s), 7.96 (1 H, s), 7.88 (1 H, d, *J* = 2.0 Hz), 7.29-7.26 (2 H, m), 7.24-7.22 (2 H, m), 7.18-7.16 (2 H, m), 7.10 (1 H, s), 7.00-6.98 (2 H, m), 5.94 (1 H, t, *J* = 5.6 Hz), 3.10 (2 H, q, *J* = 6.1 Hz), 2.70 (2 H, t, *J* = 7.6 Hz), 1.86 (2 H, quin; *J* = 7.6 Hz); HRMS (ESI-TOF) *m*/*z:* [M + H]⁺ calculated for C₂₁H₂₁N₄O 345.1710; found 345.1734.

### 6-(5-((2-Fluorobenzyl)amino)pyridin-3-yl)-1-methyl-1H-benzo[d]imidazol-2(3H)-one (30q)

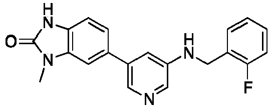

The above compound **(30q)** was prepared from **10e** and **17a** following Method E and purified by column chromatography on silica gel (MeOH/CH₂Cl₂, 5:95) to give **30q** (84%) as a white solid; **¹H NMR** (CD₃OD, 600 MHz) 8.02 (1 H, t, *J* = 1.5 Hz), 7.88 (1 H, d, *J* = 2.5 Hz), 7.44 (1 H, t, *J* = 7.6 Hz), 7.29-7.25 (3 H, m), 7.21 (1 H, t, *J* = 2.5 Hz), 7.15-7.08 (3 H, m), 4.48 (2 H, s), 3.42 (3 H, s); HRMS (ESI-TOF) *m*/*z:* [M + H]⁺ calculated for C₂₀H₁₈FN₄O 349.1460; found 349.1464.

### 6-(5-((2-Chlorobenzyl)amino)pyridin-3-yl)-1-methyl-1H-benzo[d]imidazol-2(3H)-one (30r)

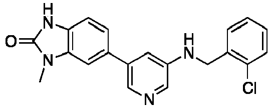

The above compound **(30r)** was prepared from **10e** and **17c** following Method E and purified by column chromatography on silica gel (MeOH/CH₂Cl₂, 5:95) to give **30r** (58%) as a yellow solid; **¹H** NMR (DMSO-d₆, 600 MHz) 10.89 (1 H, s), 8.06 (1 H, d, *J* = 16.8 Hz), 7.92 (1 H, s), 7.46 (1 H, t, *J* = 6.6 Hz), 7.40 (1 H, d, *J* = 7.1 Hz), 7.34-7.28 (3 H, m), 7.20-7.18 (1 H, m), 7.10 (1 H, d, *J* = 6.6 Hz), 7.02 (1 H, dd, *J* = 8.1, 2.5 Hz), 6.58-6.53 (1 H, m), 4.46-4.37 (2 H, m), 3.31 (3 H, s); HRMS (ESI-TOF) *m*/*z:* [M + H]⁺ calculated for C₂₀H₁₈ClN₄O 365.1164; found 365.1186.

### 5-(5-((Pyridin-2-ylmethyl)amino)pyridin-3-yl)-1H-benzo[d]imidazol-2(3H)-one (31a)

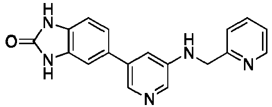

The above compound **(31a)** was prepared from **8b** and **15a** following Method E and purified by column chromatography on silica gel (MeOH/CH₂Cl₂, 10:90) to give **31a** (12%) as a yellow solid; **¹H** NMR (CD₃OD, 600 MHz) 8.52 (1 H, d, *J* = 4.9 Hz), 7.98 (1 H, s), 7.87 (1 H, d, *J* = 2.4 Hz), 7.80 (1 H, t, *J* = 7.3 Hz), 7.50 (1 H, d, *J* = 7.3 Hz), 7.31 (1 H, dd, *J* = 7.3, 4.9 Hz), 7.20 (1 H, d, *J* = 9.8 Hz), 7.17 (1 H, s), 7.13 (1 H, s), 7.09 (1 H, d, *J =* 7.3 Hz), 4.53 (2 H, s); HRMS (ESI-TOF) *m*/*z:* [M + H]⁺ calculated for C₁₈H₁₆N₅O 318.1349; found 318.1358.

### 5-(5-((Pyridin-3-ylmethyl)amino)pyridin-3-yl)-1H-benzo[d]imidazol-2(3H)-one (31b)

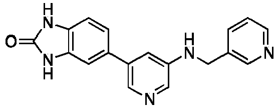

The above compound **(31b)** was prepared from **8b** and **15b** following Method E and purified by column chromatography on silica gel (MeOH/CH₂Cl₂, 10:90: 15:85) to give **31b**(5%) as a yellow solid; **¹H NMR** (CD₃OD, 600 MHz) 8.60 (1 H, s), 8.43 (1 H, d, *J* = 3.7 Hz), 7.99 (1 H, s), 7.90-7.89 (2 H, m), 7.42 (1 H, dd, *J* = 7.3, 4.9 Hz), 7.22 (1 H, d, *J* = 8.5 Hz), 7.18-7.17 (2 H, m), 7.10 (1 H, d, *J* = 8.5 Hz), 4.49 (2 H, s); HRMS (ESI-TOF) *m*/*z:* [M + H]⁺ calculated for C₁₈H₁₆N₅O 318.1349; found 318.1341.

### 5-(5-((Pyridin-4-ylmethyl)amino)pyridin-3-yl)-1H-benzo[d]imidazol-2(3H)-one (31c)

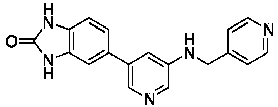

The above compound **(31c)** was prepared from **8b** and **15c** following Method E and purified by column chromatography on silica gel (MeOH/CH₂Cl₂, 10:90) to give **31c** (8%) as a yellow solid; **¹H NMR** (CD₃OD, 600 MHz) 8.48 (2 H, d, *J* = 4.9 Hz), 7.99 (1 H, s), 7.85 (1 H, d, *J* = 2.4 Hz), 7.47 (2 H, d, *J* = 6.1 Hz), 7.20-7.17 (2 H, m), 7.11-7.08 (2 H, m), 4.52 (2 H, s); HRMS (ESI-TOF) *m*/*z:* [M + H]⁺ calculated for C₁₈H₁₆N₅O 318.1349; found 318.1351.

### 5-(5-((Naphthalen-1-ylmethyl)amino)pyridin-3-yl)-1H-benzo[d]imidazol-2(3H)-one (31d)

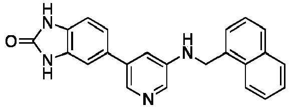

The above compound **(31d)** was prepared from **8b** and **19a** following Method E and purified by column chromatography on silica gel (MeOH/CH₂Cl₂, 5:95) to give **31d** (24%) as a yellow solid; **¹H NMR** (DMSO-*d₆*, 600 MHz) 10.68 (2 H, s), 8.17 (1 H, d, *J* = 8.5 Hz), 7.98-7.95 (3 H, m), 7.85 (1 H, d, *J* = 7.3 Hz), 7.59-7.53 (3 H, m), 7.47 (1 H, t, *J* = 8.5 Hz), 7.15-7.13 (2 H, m), 7.07 (1 H, s), 6.96 (1 H, d, *J* = 7.3 Hz), 6.56 (1 H, t, *J* = 4.9 Hz), 4.81 (2 H, d, *J* = 4.9 Hz); HRMS (ESI-TOF) *m*/*z:* [M + H]⁺ calculated for C₂₃H₁₉N₄O 367.1553; found 367.1770.

### 5-(5-((Naphthalen-2-ylmethyl)amino)pyridin-3-yl)-1H-benzo[d]imidazol-2(3H)-one (31e)

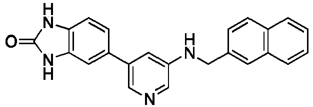

The above compound **(31e)** was prepared from **8b** and **19b** following Method E and purified by column chromatography on silica gel (MeOH/CH₂Cl₂, 5:95) to give **31e** (22%) as a yellow solid; **¹H NMR** (DMSO-*d₆*, 600 MHz) 10.69-10.68 (2 H, m), 7.96 (2 H, d, *J* = 4.9 Hz), 7.89-7.86 (4 H, m), 7.55 (1 H, d, *J* = 7.3 Hz), 7.48-7.46 (2 H, m), 7.13-7.10 (2 H, m), 7.06 (1 H, s), 6.96 (1 H, d, *J*= 8.5 Hz), 6.68 (1 H, t, *J* = 4.9 Hz), 4.54 (2 H, d, *J* = 4.9 Hz); HRMS (ESI-TOF) *m*/*z:* [M + H]⁺ calculated for C₂₃H₁₉N₄O 367.1553; found 367.1535.

### 5-(5-Aminopyridin-3-yl)-1H-benzo[d]imidazol-2(3H)-one (31f)

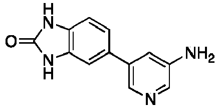

The above compound (31f) was prepared from **8b** and **4a** following Method E and purified by column chromatography on silica gel (MeOH/CH₂Cl₂, 10:90 to 15:85) to give **31f** (7%) as a brown solid; **¹H NMR** (CD₃OD, 600 MHz) 8.09 (1 H, s), 7.91 (1 H, s), 7.59 (1 H, s), 7.32 (1 H, d, *J* = 6.1 Hz), 7.30 (1 H, s), 7.15 (1 H, d, *J* = 8.5 Hz); HRMS (ESI-TOF) *m*/*z:* [M + H]⁺ calculated for C₁₂H₁₁N₄O 227.0928; found 227.0913.

### Example 9 - General Procedure for the Preparation of 5-(((Naphthalen-1-ylmethyl)amino)pyridinyl)-1H-benzo[d]imidazol-2(3H)-one-Method H

### 5-(6-((Naphthalen-1-ylmethyl)amino)pyridin-3-yl)-1H-benzo[d]imidazol-2(3H)-one (32a)

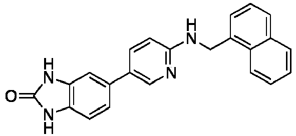

To a solution **21a** (40 mg, 0.11 mmol), **8b** (30 mg, 0.11 mmol) and Pd(dppf)Cl₂ (10 mg, 0.011 mmol) in DMF (2 mL) and H₂O (0.5 mL) was added K₃PO₄ (75 mg, 0.33 mmol). The mixture was stirred at 90°C under nitrogen for 12 h. After cooled to room temperature, the mixture was concentrated and purified by column chromatography on silica gel (MeOH/CH₂Cl₂, 20:80) to give **32a; ¹H NMR** (DMSO-d₆, 600 MHz) 10.80 (1 H, s), 10.76 (1 H, s), 8.20 (1 H, br), 8.14-8.13 (2 H, d), 8.02-8.00 (1 H, d), 7.94-7.93 (1 H, d), 7.64-7.59 (2 H, m), 7.57-7.56 (1 H, s), 7.53-7.51 (1 H, t), 7.22-7.21 (1 H, dd), 7.17 (1 H, s), 7.11-7.09 (1 H, d), 7.03-7.01 (1 H, d), 5.06 (2 H, d,); HRMS (ESI-TOF) *m*/*z:* [M + H]⁺ calculated for C₂₃H₁₉N₄O 367.1553; found 367.1550.

### 5-(5-((Naphthalen-1-ylmethyl)amino)pyridin-2-yl)-1H-benzo[d]imidazol-2(3H)-one (32b)

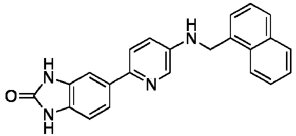

The above compound **(32b)** was prepared from **8b** and **19a** following Method H and purified by column chromatography on silica gel (MeOH/CH₂Cl₂, 10:90) to give **32b** as a light yellow solid; **¹H NMR** (DMSO-*d₆*, 600 MHz) 10.98 (1 H, s), 10.94 (1 H, s), 8.16-8.14 (1 H, d), 8.02-8.01 (1 H, d), 8.00-7.98 (1 H, d), 7.95-7.94 (1 H, d), 7.91-7.90 (1 H, d), 7.64-7.56 (4 H, m), 7.51-7.49 (1 H, t), 7.45-7.43 (1 H, dd), 7.41 (1 H, s), 7.09-7.07 (1 H, d), 4.88 (2 H, d,); HRMS (ESI-TOF) *m*/*z:* [M + H]⁺ calculated for C₂₃H₁₉N₄O 367.1553; found 367.1558.

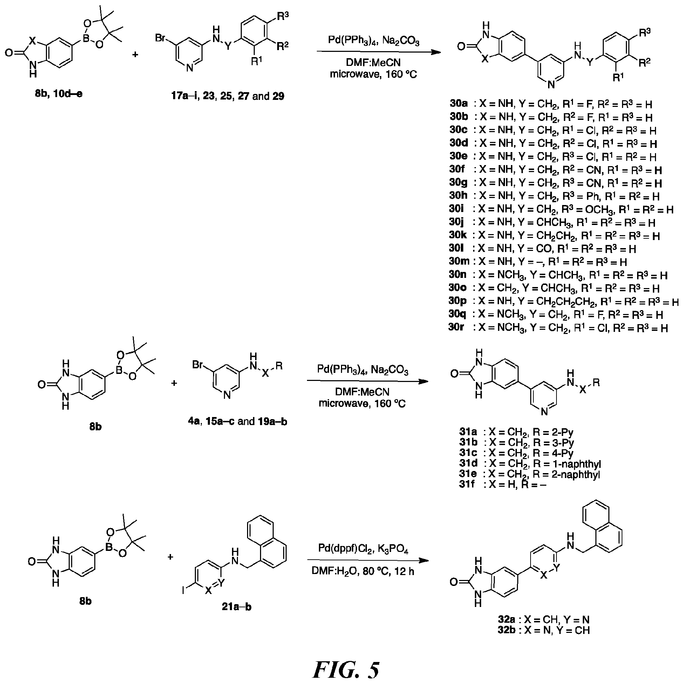

The synthetic schemes for compounds **30a-r, 31a-f,** and **32a-b,** discussed *supra,* are shown in Figure 5.

### Example 10 - General Procedure for the Preparation of (((5-(2-Oxo-2,3-dihydro-1H-benzo[d]imidazol-5-yl)pyridin-3-yl)amino)methyl)benzamide-Method I

### 3-(((5-(2-Oxo-2,3-dihydro-1H-benzo[d]imidazol-5-yl)pyridin-3-yl)amino)methyl)benzamide (33a)

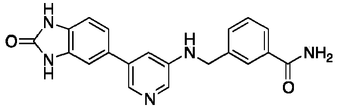

To a mixture of **30f** (8.0 mg, 0.023 mmol) and K₂CO₃ (1.0 mg, 0.007 mmol) in anhydrous DMF (0.2 mL) was added 50% hydrogen peroxide (0.01 mL) at 0°C. The reaction was stirred at 0 °C to room temperature for 16 h. The reaction was filtered and solvent was removed *in vacuo.* The crude product was washed with ether and hexane to afford **33a** (8.3 mg, quantitative yield) as a brown solid; **¹H NMR** (DMSO-*d₆*, 600 MHz) 7.96 (2 H, s), 7.92 (1 H, s), 7.88 (1 H, s), 7.73 (1 H, d, *J* = 7.3 Hz), 7.52 (1 H, d, *J* = 7.3 Hz), 7.40 (1 H, t, *J* = 7.3 Hz), 7.35 (1 H, s), 7.06-7.01 (3 H, m), 6.90 (1 H, d, *J* = 7.3 Hz), 6.59 (1 H, t, *J* = 4.9 Hz), 4.40 (2 H, d, *J* = 4.9 Hz); HRMS (ESI-TOF) *m*/*z:* [M + H]⁺ calculated for C₂₀H₁₈N₅O₂ 360.1455; found 360.1459.

### 4-(((5-(2-Oxo-2,3-dihydro-1H-benzo[d]imidazol-5-yl)pyridin-3-yl)amino)methyl)benzamide (33b)

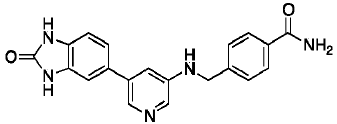

The above compound **(33b)** was prepared from **30g** following Method I and give 33b (99%) as a yellow solid; **¹H NMR** (DMSO-d₆, 600 MHz) 7.96 (1 H, s), 7.91-7.88 (2 H, m), 7.82 (2 H, d, *J* = 7.3 Hz), 7.44 (2 H, d, *J* = 8.5 Hz), 7.30 (1 H, s), 7.08 (1 H, d, *J* = 8.5 Hz), 7.02 (2 H, s), 6.94 (1 H, d, *J* = 8.5 Hz), 6.62 (1 H, t, *J* = 6.1 Hz), 4.42 (2 H, d, *J* = 6.1 Hz); HRMS (ESI-TOF) *m*/*z:* [M + H]⁺ calculated for C₂₀H₁₈N₅O₂ 360.1455; found 360.1675.

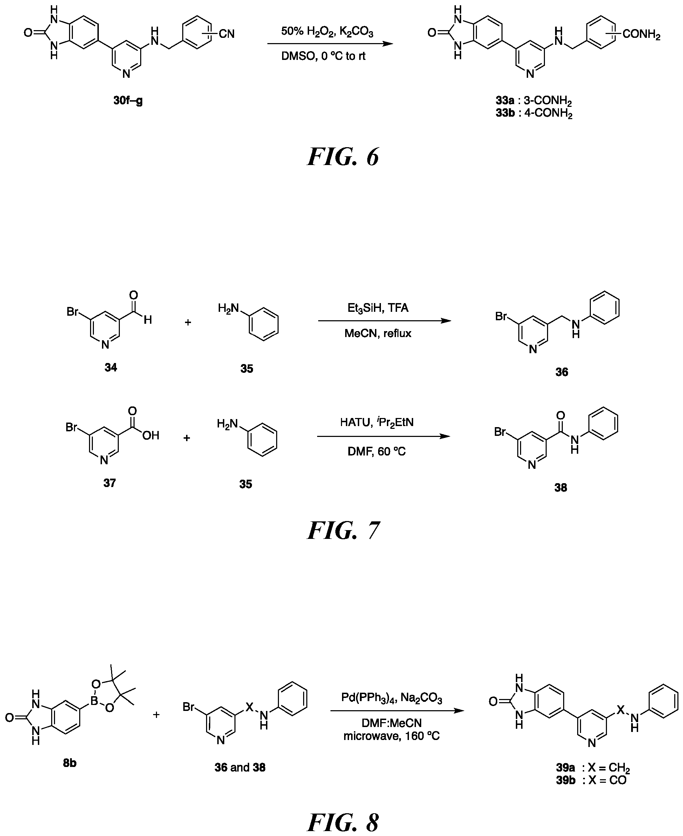

The synthetic schemes for compounds **33a-b,** discussed *supra,* are shown in Figure 6.

### N-((5-Bromopyridin-3-yl)methyl)aniline (36)

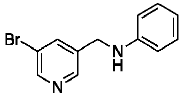

The above compound **(36)** was prepared from **34** and **35** following Method B and purified by column chromatography on silica gel (EtOAc/hexane, 20:80) to give **36** (86%) as a yellow oil; **¹H NMR** (CDCl₃, 600 MHz) 8.56 (1 H, d, *J* = 2.4 Hz), 8.54 (1 H, s), 7.86 (1 H, s), 7.19 (2 H, t, *J* = 7.3 Hz), 6.76 (1 H, t, *J* = 7.3 Hz), 6.01 (2 H, d, *J* = 7.3 Hz), 4.37 (2 H, d, *J* = 4.9 Hz), 4.12 (1 H, Br); HRMS (ESI-TOF) *m*/*z:* [M + H]⁺ calculated for C₁₂H₁₂⁷⁹Br₂ and C₁₂H₁₂⁸¹Br₂ 263.0179, 265.0158; found 263.0362, 265.0345.

### 5-Bromo-N-phenylnicotinamide (38)

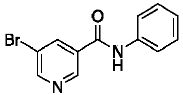

The above compound **(38)** was prepared from **37** and **35** following Method G and purified by column chromatography on silica gel (EtOAc/hexane, 20:80 to 30:70) to give **38** (95%) as a pale yellow solid; **¹H NMR** (CD₃OD, 600 MHz) 9.03 (1 H, s), 8.83 (1 H, s), 8.52 (1 H, s), 7.69 (2 H, d, *J* = 7.3 Hz), 7.36 (2 H, t, *J* = 7.3 Hz), 7.16 (1 H, d, *J* = 7.3 Hz); HRMS (ESI-TOF) *m*/*z:* [M + H]⁺ calculated for C₁₂H₁₀⁷⁹BrN₂O and C₁₂H₁₀⁸¹BrN₂O 276.9972, 278.9951; found 277.0071, 279.0042.

The synthetic schemes for intermediates **36** and **38,** discussed *supra,* are shown in Figure 7.

### Example 11 - General Procedure for the Preparation of Benzimidazolonyl Benzyl Heterocyclic Amines-Method J

### 5-(5-((Phenylamino)methyl)pyridin-3-yl)-1H-benzo[d]imidazol-2(3H)-one (39a)

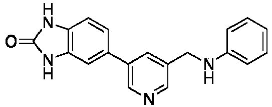

To a mixture of **8b** (35 mg, 0.13 mmol), **36** (35 mg, 0.13 mmol), and Pd(PPh₃)₄ (16 mg, 0.01 mmol) in a microwave reaction vial was added anhydrous DMF (1 mL), anhydrous acetonitrile (1 mL) and 1 M Na₂CO₃ (0.27 mL, 0.27 mmol) under nitrogen. The reaction mixture was irradiated under microwave conditions for 15 min at 160 °C. After being quenched by the addition of water, the aqueous layer was extracted with EtOAc (2 × 10 mL). The combined organic extracts were washed with brine, dried over anhydrous Na₂SO₄, filtered, and concentrated. The residue was purified by column chromatography on silica gel (MeOH/CH₂Cl₂, 5:95 to 10:90) to afford **39a** (14.5 mg, 34%) as a pale yellow solid; **¹H NMR** (DMSO-d₆, 600 MHz) 10.78-10.75 (2 H, m), 8.67 (1 H, s), 8.50 (1 H, s), 7.95 (1 H, s), 7.24-7.04 (5 H, m), 6.61-6.51 (3 H, m), 6.30 (1 H, s), 4.34 (2 H, s); HRMS (ESI-TOF) *m*/*z:* [M + H]⁺ calculated for C₁₉H₁₇N₄O 317.1397; found 317.1389.

### 5-(2-Oxo-2,3-dihydro-1H-benzo[d]imidazol-5-yl)-N-phenylnicotinamide (39b)

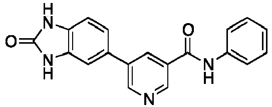

The above compound **(39b)** was prepared from **8b** and **38** following Method J and purified by column chromatography on silica gel (MeOH/CH₂Cl₂, 5:95 to 10:90) to give **39b** (37%) as a yellow solid; **¹H NMR** (DMSO-d₆, 600 MHz) 10.86 (1 H, s), 10.81 (1 H, s), 10.50 (1 H, s), 9.00 (2 H, d, *J* = 2.4 Hz), 8.47 (1 H, s), 7.78 (2 H, d, *J* = 8.5 Hz), 7.41-7.34 (4 H, m), 7.13 (1 H, t, *J* = 7.3 Hz), 7.07 (1 H, d, *J* = 7.3 Hz); HRMS (ESI-TOF) *m*/*z:* [M + H]⁺ calculated for C₁₉H₁₅N₄O₂ 331.1190; found 331.1424.

The synthetic schemes for compounds **39a-b,** discussed *supra,* are shown in Figure 8.

### 3-Bromo-2-methyl-6-nitroaniline (41)

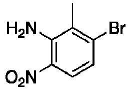

To a solution of **40** (500 mg, 3.29 mmol) in acetic acid (25 mL) was added NBS (585 mg, 3.29 mmol). The reaction mixture was refluxed for 2 h. After being quenched by the addition of water, the aqueous layer was extracted with EtOAc (2 × 20 mL). The combined organic extracts were washed with brine, dried over anhydrous Na₂SO₄, filtered, and concentrated. The residue was purified by column chromatography on silica gel (EtOAc/hexane, 10:90 to 15:85) to afford **41** (661.1 mg, 87%) as an orange solid; **¹H NMR** (CDCl₃, 600 MHz) 7.41(1 H, d, *J* = 8.5 Hz), 6.56 (1 H, d, *J* = 8.5 Hz), 4.77 (2 H, br), 2.44 (3 H, s); HRMS (ESI-TOF) *m*/*z:* [M + H]⁺ calculated for C₇H₈⁷⁹BrN₂O₂ and C₇H₈⁸¹BrN₂O₂ 230.9764, 232.9744; found 230.9727, 232.9710.

### 4-Bromo-3-methylbenzene-1,2-diamine (42a)

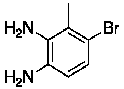

To a mixture of **41** (600 mg, 2.60 mmol), iron (725 mg, 13.0 mmol) and ammonium chloride (736 mg, 13.8 mmol) were added ethanol (20 mL) and water (4 mL). The reaction mixture was refluxed for 3 h. After cooled down, the reaction was filtered through a Celite pad and solvent was removed *in vacuo.* EtOAc and water were added. The aqueous layer was extracted with EtOAc (2 × 20 mL). The combined organic extracts were washed with brine, dried over anhydrous Na₂SO₄, filtered, and concentrated. The residue was purified by column chromatography on silica gel (EtOAc/hexane, 50:50) to afford **42a** (449.2 mg, 86%) as a brown oil; **¹H NMR** (CDCl₃, 600 MHz) 6.90 (1 H, d, *J =* 8.5 Hz), 6.50 (1 H, d, *J* = 8.5 Hz), 3.54 (2 H, br), 3.29 (2 H, br), 2.30 (3 H, s); HRMS (ESI-TOF) *m*/*z:* [M + H]⁺ calculated for C₇H₁₀⁷⁹BrN₂ and C₇H₁₀⁸¹BrN₂ 201.0022, 203.0002; found 201.0112, 203.0095.

### Example 12 - General Procedure for the Preparation of Benzimidazolone-Method K

### 5-Bromo-4-methyl-1H-benzo[d]imidazol-2(3H)-one (43a)

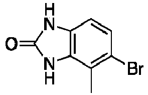

To a solution of **42a** (200 mg, 1.00 mmol) in anhydrous dichloromethane (10 mL) was added triethylamine (0.36 mL, 2.59 mmol). The reaction was purged with nitrogen and stirred in an ice-bath. Triphosgene (148 mg, 0.497 mmol) was added under nitrogen at 0 °C. The reaction mixture was stirred at 0 °C for 40 min. After being quenched by the addition of saturated sodium bicarbonate, the aqueous layer was extracted with EtOAc (2 × 15 mL). The combined organic extracts were washed with brine, dried over anhydrous Na₂SO₄, filtered, and concentrated. The crude product was washed with hexane and ether to afford **43a** (168.2 mg, 74%) as a light brown solid; **¹H NMR** (DMSO-d₆, 600 MHz) 10.93 (1 H, s), 10.74 (1 H, s), 7.12 (1 H, d, *J* = 8.5 Hz), 6.70 (1 H, d, *J* = 8.5 Hz), 2.28 (3 H, s); HRMS (ESI-TOF) *m*/*z:* [M + H]⁺ calculated for C₈H₈⁷⁹BrN₂O and C₈H₈⁸¹BrN₂O 226.9815, 228.9715; found 226.9801, 228.9781.

### 5-Bromo-6-methyl-1H-benzo[d]imidazol-2(3H)-one (43b)

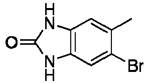

The above compound **(43b)** was prepared from **42b** following Method K to give **43b** (76%) as a light brown solid; **¹H NMR** (DMSO-d₆, 600 MHz) 10.86 (1 H, s), 10.70 (1 H, s), 10.63 (1 H, s), 7.05 (1 H, s), 6.89 (1 H, s), 2.28 (3 H, s); HRMS (ESI-TOF) *m*/*z:* [M + H]⁺ calculated for C₈H₈⁷⁹BrN₂O and C₈H₈⁸¹BrN₂O 226.9815, 228.9715; found 226.9911, 228.9890.

### 4-Methyl-5-(4,4,5,5-tetramethyl-1,3,2-dioxaborolan-2-yl)-1H-benzo[d]imidazol-2(3H)-one (44a)

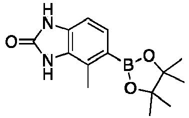

The above compound **(44a)** was prepared from **43a** following Method C. A mixture of 1,4-dioxane and DMF (4:1) was used as a solvent and the mixture was stirred at 80 °C for 67 h . The reaction was purified by column chromatography on silica gel (MeOH/CH₂Cl₂, 2.5:97.5 to 5:95) to give **44a** (36%) as a light brown solid; **¹H NMR** (CDCl₃, 600 MHz) 10.42 (1 H, s), 10.34 (1 H, s), 7.60 (1 H, d, *J* = 7.3 Hz ), 6.99 (1 H, d, *J* = 8.5 Hz), 2.64 (3 H, s), 1.36 (12 H, s); HRMS (ESI-TOF) *m*/*z:* [M + H]⁺ calculated for C₁₄H₂₀BN₂O₃ 275.1561; found 275.1570.

### 5-Methyl-6-(4,4,5,5-tetramethyl-1,3,2-dioxaborolan-2-yl)-1H-benzo[d]imidazol-2(3H)-one (44b)

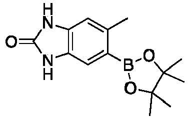

The above compound **(44b)** was prepared from **43b** following Method C. A mixture of 1,4-dioxane and DMF (4:1) was used as a solvent and the mixture was stirred at 80 °C for 67 h . The reaction was purified by column chromatography on silica gel (MeOH/CH₂Cl₂, 2.5:97.5 to 5:95) to give **44b** (30%) as a brown solid; **¹H NMR** (CDCl₃, 600 MHz) 9.80 (1 H, s), 9.17 (1 H, s), 7.51 (1 H, s), 6.90 (1 H, s), 2.55 (3 H, s), 1.33 (12 H, s); HRMS (ESI-TOF) *m*/*z:* [M + H]⁺ calculated for C₁₄H₂₀BN₂O₃ 275.1561; found 275.1572.

### 5-(5-(Benzylamino)pyridin-3-yl)-4-methyl-1H-benzo[d]imidazol-2(3H)-one (45a)

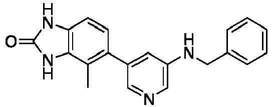

The above compound **(45a)** was prepared from **44a** and **5a** following Method J and purified by column chromatography on silica gel (MeOH/CH₂Cl₂, 5:95 to 10:90) to give **45a** (51%) as a pale yellow solid; **¹H NMR** (DMSO-d₆, 600 MHz) 10.71 (1 H, s), 10.62 (1 H, s), 7.94 (1 H, s), 7.66 (1 H, s), 7.36-7.22 (5 H, m), 6.78-6.74 (3 H, m), 6.58 (1 H, s), 4.31 (2 H, s), 2.02 (3 H, s); HRMS (ESI-TOF) *m*/*z:* [M + H]⁺ calculated for C₂₀H₁₉N₄O 331.1553; found 331.1573.

### 5-(5-(Benzylamino)pyridin-3-yl)-6-methyl-1H-benzo[d]imidazol-2(3H)-one (45b)

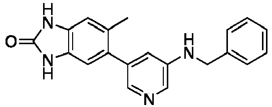

The above compound **(45b)** was prepared from **44b** and **5a** following Method J and purified by column chromatography on silica gel (MeOH/CH₂Cl₂, 5:95 to 10:90) to give **45b** (69%) as a light brown solid; **¹H NMR** (DMSO-d₆, 600 MHz) 10.58-10.52 (2 H, m), 7.94 (1 H, s), 7.65 (1 H, s), 7.34-7.24 (5 H, m), 6.77-6.59 (4 H, m), 4.31 (2 H, s), 2.04 (3 H, s); HRMS (ESI-TOF) *m*/*z:* [M + H]⁺ calculated for C₂₀H₁₉N₄O 331.1553; found 331.1534.

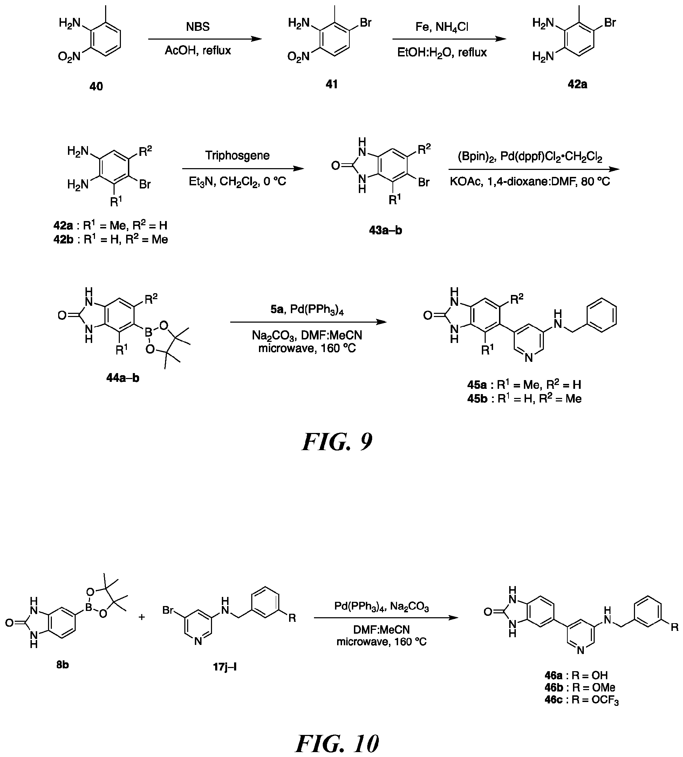

The synthetic schemes for compounds **45a-b,** discussed *supra,* are shown in Figure 9.

### 5-(5-((3-Hydroxybenzyl)amino)pyridin-3-yl)-1H-benzo[d]imidazol-2(3H)-one (46a)

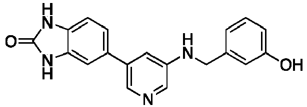

The above compound **(46a)** was prepared from **8b** and **17j** following Method J and purified by column chromatography on silica gel (MeOH/CH₂Cl₂, 10:90) to give **46a** (30%) as a yellow solid; **¹H NMR** (DMSO-d₆, 600 MHz) 10.70-10.69 (2 H, m), 9.32 (1 H, s), 7.95 (1 H, s), 7.89 (1 H, d, *J* = 2.4 Hz), 7.14-7.09 (2 H, m), 7.05-7.01 (2 H, m), 6.97 (1 H, d, *J* = 7.3 Hz), 6.79-6.76 (2 H, m), 6.60 (1 H, d, *J* = 7.3 Hz), 6.54 (1 H, s), 4.27 (2 H, d, *J* = 6.1 Hz); HRMS (ESI-TOF) *m*/*z:* [M + H]⁺ calculated for C₁₉H₁₇N₄O₂ 333.1346; found 333.1345.

### 5-(5-((3-Methoxybenzyl)amino)pyridin-3-yl)-1H-benzo[d]imidazol-2(3H)-one (46b)

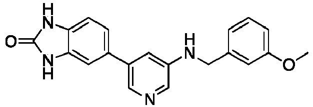

The above compound **(46b)** was prepared from **8b** and **17k** following Method J and purified by column chromatography on silica gel (MeOH/CH₂Cl₂, 5:95 to 10:90) to give **46b** (38%) as a yellow solid; **¹H NMR** (DMSO-d₆, 600 MHz) 10.71-10.69 (2 H, m), 7.96 (1 H, s), 7.90 (1 H, s), 7.24 (1 H, t, *J* = 8.5 Hz), 7.13 (1 H, d, *J* = 8.5 Hz), 7.05 (1 H, s), 7.12 (1 H, s), 6.97-6.95 (3 H, m), 6.79 (1 H, d, *J* = 6.1 Hz), 6.56 (1 H, t, *J* = 6.1 Hz), 4.33 (2 H, d, *J* = 6.1 Hz), 3.71 (3 H, s); HRMS (ESI-TOF) *m*/*z:* [M + H]⁺ calculated for C₂₀H₁₉N₄O₂ 347.1503; found 347.1492.

### 5-(5-((3-(Trifluoromethoxy)benzyl)amino)pyridin-3-yl)-1H-benzo[d]imidazol-2(3H)-one (46c)

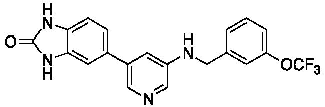

The above compound **(46c)** was prepared from **8b** and **171** following Method J and purified by column chromatography on silica gel (MeOH/CH₂Cl₂, 5:95 to 10:90) to give **46c** (40%) as a yellow solid; **¹H NMR** (DMSO-*d₆*, 600 MHz) 10.71-10.69 (2 H, m), 7.98 (1 H, s), 7.91 (1 H, s), 7.47 (1 H, t, *J* = 7.3 Hz), 7.42 (1 H, d, *J* = 8.1 Hz), 7.36 (1 H, s), 7.22 (1 H, d, *J* = 8.5 Hz), 7.11 (1 H, d, *J* = 7.3 Hz), 7.06 (2 H, s), 6.96 (1 H, d, *J* = 7.3 Hz), 6.64 (1 H, s), 4.44 (2 H, d, *J* = 4.3 Hz); HRMS (ESI-TOF) *m*/*z:* [M + H]⁺ calculated for C₂₀H₁₆F₃N₄O₂ 401.1220; found 401.1196.

The synthetic schemes for compounds **46a-c,** discussed *supra,* are shown in Figure 10.

### 5-Bromo-N-(furan-2-ylmethyl)pyridin-3-amine (48a)

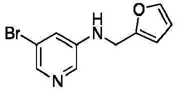

The above compound **(48a)** was prepared from **4a** and **47a** following Method B and purified by column chromatography on silica gel (EtOAc/hexane, 30:70) to give **48a** (56%) as a brown solid; **¹H NMR** (CDCl₃, 500 MHz) 8.03 (1 H, d, *J* = 1.5 Hz), 7.99 (1 H, d, *J* = 2.5 Hz), 7.38 (1 H, s), 7.09 (1 H, t, *J* = 2.5 Hz), 6.34 (1 H, t, *J* = 2.5 Hz), 6.26 (1 H, d, *J* = 3.0 Hz), 4.32 (2 H, d, *J* = 5.6 Hz), 4.17 (1 H, br); HRMS (ESI-TOF) *m*/*z:* [M + H]⁺ calculated for C₁₀H₁₀⁷⁹BrN₂O and C₁₀H₁₀⁸¹BrN₂O 252.9971, 254.9951; found 252.9993, 254.9974.

### 5-Bromo-N-(thiophen-2-ylmethyl)pyridin-3-amine (48b)

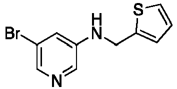

The above compound **(48b)** was prepared from **4a** and **47b** following Method B and purified by column chromatography on silica gel (EtOAc/hexane, 30:70) to give **48b** (68%) as a yellow solid; **¹H NMR** (CDCl₃, 500 MHz) 8.04 (1 H, d, *J* = 1.5 Hz), 7.99 (1 H, d, *J* = 2.5 Hz), 7.25 (1 H, s), 7.08 (1 H, t, *J* = 2.0 Hz), 7.03 (1 H, d, *J* = 3.6 Hz), 6.99 (1 H, dd, *J* = 5.1, 3.6 Hz), 4.52 (2 H, d, *J* = 5.6 Hz), 4.20 (1 H, br); HRMS (ESI-TOF) *m*/*z:* [M + H]⁺ calculated for C₁₀H₁₀⁷⁹BrN₂S and C₁₀H₁₀⁸¹BrN₂S 268.9743, 270.9722; found 268.9744, 270.9722.

### N-((1H-Imidazol-2-yl)methyl)-5-bromopyridin-3-amine (48c)

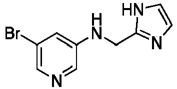

The above compound **(48c)** was prepared from **4a** and **47c** following Method B and purified by column chromatography on silica gel (MeOH/CHCl₃, 5:95 to 10:90) to give **48c** (37%) as a brown solid; **¹H NMR** (CD₃OD₃, 500 MHz) 7.89 (1 H, d, *J* = 2.5 Hz), 7.83 (1 H, d, *J* = 2.0 Hz), 7.15 (1 H, t, *J* = 2.0 Hz), 6.98 (2 H, s), 4.38 (2 H, s); HRMS (ESI-TOF) *m*/*z:* [M + H]⁺ calculated for C₉H₁₀⁷⁹BrN₄ and C₉H₁₀⁸¹BrN₄ 253.0083, 255.0063; found 253.0078, 255.0056.

### N-((1H-imidazol-4-yl)methyl)-5-bromopyridin-3-amine (48d)

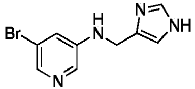

The above compound **(48d)** was prepared from **4a** and **47d** following Method B and purified by column chromatography on silica gel (MeOH/CHCl₃, 10:90 to 15:95) to give **48d** (87%) as a pale yellow solid; **¹H NMR** (CD₃OD₃, 500 MHz) 7.89 (1 H, d, *J* = 2.5 Hz), 7.78 (1 H, d, *J* = 1.5 Hz), 7.63 (1 H, *d, J* = 1.0 Hz), 7.19 (1 H, t, *J* = 2.5 Hz), 7.00 (1 H, s), 4.27 (2 H, s); HRMS (ESI-TOF) *m*/*z:* [M + H]⁺ calculated for C₉H₁₀⁷⁹BrN₄ and C₉H₁₀⁸¹BrN₄ 253.0083, 255.0063; found 253.0094, 255.0072

### N-((1H-pyrazol-4-yl)methyl)-5-bromopyridin-3-amine (48e)

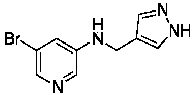

The above compound **(48e)** was prepared from **4a** and **47e** following Method B and purified by column chromatography on silica gel (MeOH/CHCl₃, 5:95 to 10:90) to give **48e** (63%) as a pale yellow solid; **¹H NMR** (CDCl₃, 500 MHz) 8.02-8.00 (2 H, m), 7.78 (1 H, d, *J* = 1.5 Hz), 7.56 (1 H, d, *J* = 2.0 Hz), 7.10 (1 H, t, *J*= 2.0 Hz), 6.29 (1 H, d, *J* = 2.0 Hz), 4.39 (3 H, m); HRMS (ESI-TOF) *m*/*z:* [M + H]⁺ calculated for C₉H₁₀⁷⁹BrN₄ and C₉H₁₀⁸¹BrN₄ 253.0083, 255.0063; found 253.0084, 255.0063.

### 5-Bromo-N-(oxazol-2-ylmethyl)pyridin-3-amine (48f)

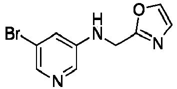

The above compound **(48f)** was prepared from **4a** and **47f** following Method B and purified by column chromatography on silica gel (EtOAC/hexane, 30:70 to 90:10) to give **48f** (84%) as a pale yellow solid; **¹H NMR** (CDCl₃, 500 MHz) 8.06 (1 H, s), 8.03 (1 H, d, *J* = 2.0 Hz), 7.64 (1 H, s), 7.13 (1 H, s), 7.10 (1 H, s), 4.58 (1 H, br), 4.46 (2 H, d, *J* = 6.1 Hz); HRMS (ESI-TOF) *m*/*z:* [M + H]⁺ calculated for C₉H₉⁷⁹BrN₃O and C₉H₉⁸¹BrN₃O 253.9924, 255.9903; found 253.9928, 255.9907.

### 5-(5-((Furan-2-ylmethyl)amino)pyridin-3-yl)-1H-benzo[d]imidazol-2(3H)-one (49a)

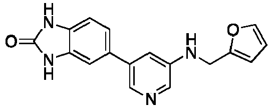

The above compound **(49a)** was prepared from **8b** and **48a** following Method E and purified by column chromatography on silica gel (MeOH/CH₂Cl₂, 5:95 to 10:90) to give **49a** (30%) as a light brown solid; **¹H NMR** (DMSO-d₆, 500 MHz) 10.68-10.67 (2 H, m), 7.99 (1 H, d, *J* = 1.5 Hz), 7.96 (1 H, d, *J* = 2.5 Hz), 7.58 (1 H, s), 7.17 (1 H, dd, *J* = 8.1, 1.5 Hz), 7.14 (1 H, t, *J* = 2.0 Hz), 7.10 (1 H, s), 6.99 (1 H, d, *J* = 8.1 Hz), 6.40-6.38 (2 H, m), 6.33 (1 H, d, *J* = 3.0 Hz), 4.35 (2 H, d, *J* = 6.1 Hz); HRMS (ESI-TOF) *m*/*z:* [M + H]⁺ calculated for C₁₇H₁₅N₄O₂ 307.1190; found 307.1181.

### 5-(5-((Thiophen-2-ylmethyl)amino)pyridin-3-yl)-1H-benzo[d]imidazol-2(3H)-one (49b)

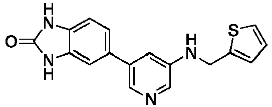

The above compound **(49b)** was prepared from **8b** and **48b** following Method E and purified by column chromatography on silica gel (MeOH/CH₂Cl₂, 5:95 to 10:90) to give **49b** (27%) as a light brown solid; **¹H NMR** (DMSO-*d₆*, 500 MHz) 10.68-10.66 (2 H, m), 7.99 (1 H, s), 7.94 (1 H, d, *J* = 2.5 Hz), 7.37 (1 H, d, *J* = 4.6 Hz), 7.16 (1 H, dd, *J* = 8.1, 1.0 Hz), 7.12 (1 H, t, *J* = 2.0 Hz), 7.08 (2 H, s), 6.99-6.96 (2 H, m), 6.55 (1 H, t, *J* = 5.6 Hz), 4.56 (2 H, d, *J* = 6.1 Hz); HRMS (ESI-TOF) *m*/*z:* [M + H]⁺ calculated for C₁₇H₁₅N₄OS 323.0961; found 323.1013.

### 5-(5-(((1H-Imidazol-2-yl)methyl)amino)pyridin-3-yl)-1H-benzo[d]imidazol-2(3H)-one (49c)

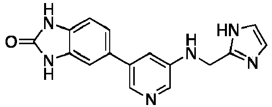

The above compound **(49c)** was prepared from **8b** and **48c** following Method E and purified by column chromatography on silica gel (MeOH/CH₂Cl₂, 5:95 to 20:80) to give **49c** (40%) as a light brown solid; **¹H NMR** (DMSO-d₆, 500 MHz) 11.89 (1 H, br), 10.69 (1 H, s), 10.66 (1 H, s), 7.99 (1 H, d, *J* = 1.5 Hz), 7.96 (1 H, d, *J* = 2.5 Hz), 7.16-7.14 (2 H, m), 7.08 (1 H, s), 7.02 (1 H, s), 6.98 (1 H, d, *J* = 8.1 Hz), 6.83 (1 H, s), 7.08 (2 H, s), 6.36 (1 H, t, *J* = 6.1 Hz), 4.32 (2 H, d, *J* = 5.6 Hz); HRMS (ESI-TOF) *m*/*z:* [M + H]⁺ calculated for C₁₆H₁₅N₆O 307.1203; found 307.1301.

### 5-(5-(((1H-Imidazol-4-yl)methyl)amino)pyridin-3-yl)-1H-benzo[d]imidazol-2(3H)-one (49d)

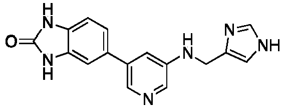

The above compound **(49d)** was prepared from **8b** and **48d** following Method E and purified by column chromatography on silica gel (MeOH/CH₂Cl₂, 15:85 to 20:80) to give **49d** (15%) as a light brown solid; **¹H NMR** (DMSO-*d₆*, 500 MHz) 10.67-10.66 (2 H, m), 8.21 (1 H, s), 7.97-7.95 (2 H, m), 7.56 (1 H, s), 7.17 (1 H, dd, *J* = 8.1, 1.5 Hz), 7.13 (1 H, s), 7.10 (1 H, s), 6.98 (1 H, d, *J* = 8.1 Hz), 6.15 (1 H, t, *J* = 5.1 Hz), 4.21 (2 H, s); HRMS (ESI-TOF) *m*/*z:* [M + H]⁺ calculated for C₁₆H₁₅N₆O 307.1302; found 307.1302.

### 5-(5-(((1H-Pyrazol-3-yl)methyl)amino)pyridin-3-yl)-1H-benzo[d]imidazol-2(3H)-one (49e)

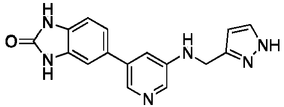

The above compound **(49e)** was prepared from **8b** and **48e** following Method E and purified by column chromatography on silica gel (MeOH/CH₂Cl₂, 5:95 to 10:90) to give **49e** (17%) as a light brown solid; **¹H NMR** (DMSO-d₆, 500 MHz) 10.67-10.66 (2 H, m), 7.97-7.94 (2 H, m), 7.62 (1 H, s), 7.17-7.09 (3 H, m), 6.98 (1 H, d, *J* = 8.1 Hz), 6.29 (1 H, br), 6.19 (1 H, s), 4.30 (2 H, br); HRMS (ESI-TOF) *m*/*z:* [M + H]⁺ calculated for C₁₆H₁₅N₆O 307.1302; found 307.1288.

### 5-(5-((Oxazol-2-ylmethyl)amino)pyridin-3-yl)-1H-benzo[d]imidazol-2(3H)-one (49f)

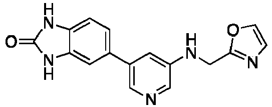

The above compound **(49f)** was prepared from **8b** and **48f** following Method E and purified by column chromatography on silica gel (MeOH/CH₂Cl₂, 5:95 to 10:90) to give **49f** (12%) as a light brown solid; **¹H NMR** (DMSO-*d₆*, 500 MHz) 10.70 (1 H, s), 10.67 (1 H, s), 8.04 (1 H, s), 8.02 (1 H, s), 7.96 (1 H, s), 7.17-7.14 (3 H, m), 7.09 (1 H, s), 6.99 (1 H, d, *J* = 8.1 Hz), 6.62 (1 H, t, *J* = 6.6 Hz), 4.52 (2 H, d, *J* = 6.1 Hz); HRMS (ESI-TOF) *m*/*z:* [M + H]⁺ calculated for C₁₆H₁₄N₅O₂ 308.1142; found 308.1156.

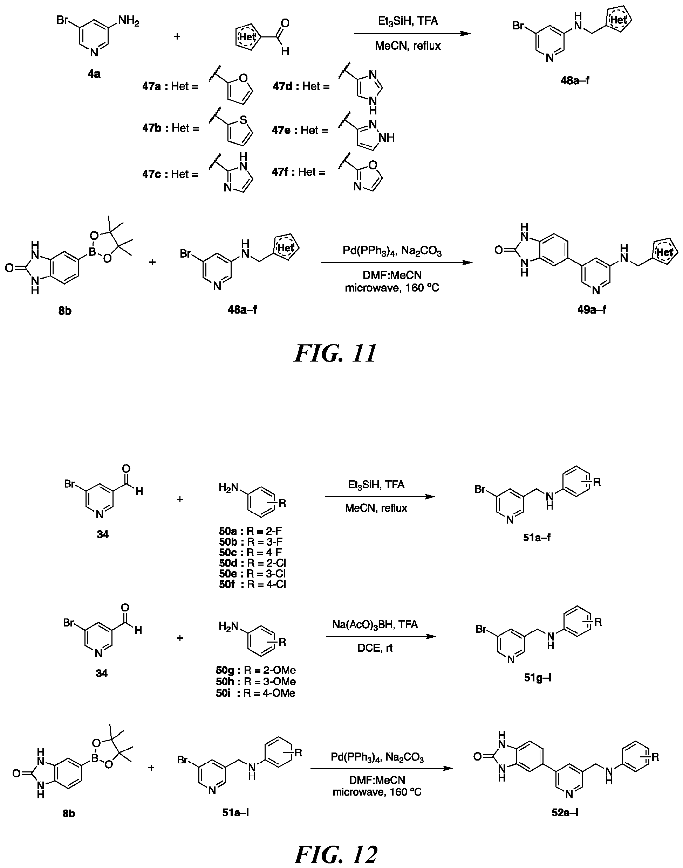

The synthetic schemes for compounds **49a-f,** discussed *supra,* are shown in Figure 11.

### N-((5-Bromopyridin-3-yl)methyl)-2-fluoroaniline (51a)

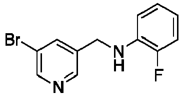

The above compound **(51a)** was prepared from **34** and **50a** following Method B and purified by column chromatography on silica gel (EtOAC/hexane, 20:80) to give **51a** (91%) as a yellow oil; **¹H NMR** (CDCl₃, 500 MHz) 8.60 (1 H, d, *J* = 2.0 Hz), 8.55 (1 H, s), 7.86 (1 H, s), 7.01 (1 H, ddd, *J* = 11.7, 8.1, 1.2 Hz), 6.96 (1 H, t, *J* = 8.1 Hz), 6.71-6.66 (1 H, m), 6.59 (1 H, t, *J* = 8.6 Hz), 4.41-4.40 (3 H, m); HRMS (ESI-TOF) *m*/*z:* [M + H]⁺ calculated for C₁₂H₁₁⁷⁹BrFN₂ and C₁₂H₁₁⁸¹BrFN₂ 281.0085, 283.0064; found 281.0176, 283.0155.

### N-((5-Bromopyridin-3-yl)methyl)-3-fluoroaniline (51b)

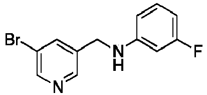

The above compound **(51b)** was prepared from **34** and **50b** following Method B and purified by column chromatography on silica gel (EtOAC/hexane, 15:85) to give **51b** (88%) as a yellow oil; **¹H NMR** (CDCl₃, 500 MHz) 8.60 (1 H, d, *J* = 2.0 Hz), 8.53 (1 H, s), 7.84 (1 H, s), 7.11 (1 H, q, *J* = 6.6 Hz), 6.45 (1 H, td, *J* = 8.4, 2.5 Hz), 6.38 (1 H, dd, *J* = 8.1, 2.0 Hz), 6.29 (1 H, dt, *J* = 11.2, 2.5 Hz), 4.36 (2 H, d, *J* = 5.6 Hz), 4.22 (1 H, br); HRMS (ESI-TOF) *m*/*z:* [M + H]⁺ calculated for C₁₂H₁₁⁷⁹BrFN₂ and C₁₂H₁₁⁸¹BrFN₂ 281.0085, 283.0064; found 281.0271, 283.0252.

### N-((5-Bromopyridin-3-yl)methyl)-4-fluoroaniline (51c)

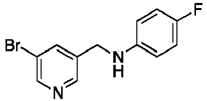

The above compound **(51c)** was prepared from **34** and **50c** following Method B and purified by column chromatography on silica gel (EtOAC/hexane, 15:85) to give **51c** (89%) as a yellow oil; **¹H NMR** (CDCl₃, 500 MHz) 8.60 (1 H, d, *J* = 2.0 Hz), 8.53 (1 H, d, *J* = 1.5 Hz), 7.85 (1 H, d, *J* = 1.5 Hz), 6.91-6.88 (2 H, m), 6.55-6.53 (2 H, m), 4.33 (2 H, d, *J* = 4.1 Hz), 3.99 (1 H, br); HRMS (ESI-TOF) *m*/*z:* [M + H]⁺ calculated for C₁₂H₁₁⁷⁹BrFN₂ and C₁₂H₁₁⁸¹BrFN₂ 281.0085, 283.0064; found 281.0169, 283.0149.

### N-((5-Bromopyridin-3-yl)methyl)-2-chloroaniline (51d)

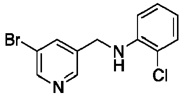

The above compound **(51d)** was prepared from **34** and **50d** following Method B and purified by column chromatography on silica gel (EtOAC/hexane, 20:80) to give **51d** (97%) as a yellow oil; **¹H NMR** (CDCl₃, 500 MHz) 8.60 (1 H, d, *J* = 1.5 Hz), 8.54 (1 H, s), 7.84 (1 H, s), 7.29 (1 H, dd, *J* = 7.9, 1.0 Hz), 7.10 (1 H, t, *J* = 7.6 Hz), 6.69 (1 H, t, *J* = 8.1 Hz), 6.54 (1 H, d, *J* = 8.1 Hz), 4.80 (1 H, br), 4.44 (2 H, d, *J* = 5.6 Hz); HRMS (ESI-TOF) *m*/*z:* [M + H]⁺ calculated for C₁₂H₁₁⁷⁹BrClN₂ and C₁₂H₁₁⁸¹BrClN₂ 296.9789, 298.9769; found 296.9784, 298.9765.

### N-((5-Bromopyridin-3-yl)methyl)-3-chloroaniline (51e)

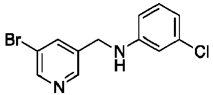

The above compound **(51e)** was prepared from **34** and **50e** following Method B and purified by column chromatography on silica gel (EtOAC/hexane, 30:70) to give **51e** (21%) as a yellow oil; **¹H NMR** (CDCl₃, 500 MHz) 8.61 (1 H, s), 8.53 (1 H, s), 7.83 (1 H, s), 7.09 (1 H, t, *J* = 7.9 Hz), 6.73 (1 H, d, *J* = 8.1 Hz), 6.59 (1 H, s), 6.47 (1 H, d, *J* = 7.1 Hz), 4.35 (2 H, d, *J* = 5.6 Hz), 4.18 (1 H, br); HRMS (ESI-TOF) *m*/*z:* [M + H]⁺ calculated for C₁₂H₁₁⁷⁹BrClN₂ and C₁₂H₁₁⁸¹BrClN₂ 296.9789, 298.9769; found 296.9793, 298.9775.

### N-((5-Bromopyridin-3-yl)methyl)-4-chloroaniline (51f)

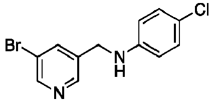

The above compound **(51f)** was prepared from **34** and **50f** following Method B and purified by column chromatography on silica gel (EtOAC/hexane, 20:80 to 30:70) to give **51f** (82%) as a yellow solid; **¹H NMR** (CDCl₃, 500 MHz) 8.60 (1 H, d, *J* = 2.0 Hz), 8.53 (1 H, s), 7.83 (1 H, s), 7.13 (2 H, d, *J* = 8.6 Hz), 6.53 (2 H, d, *J* = 8.6 Hz), 4.34 (2 H, d, *J* = 5.6 Hz), 4.12 (1 H, br); HRMS (ESI-TOF) *m*/*z:* [M + H]⁺ calculated for C₁₂H₁₁⁷⁹BrClN₂ and C₁₂H₁₁⁸¹BrClN₂ 296.9789, 298.9769; found 293.0273, 295.0256.

### Example 13 - General Procedure for the Preparation of N-Bromopyridinylmethylaniline-Method L

### N-((5-Bromopyridin-3-yl)methyl)-2-methoxyaniline (51g)

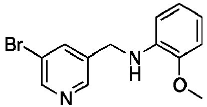

To a solution of 2-methoxyaniline **(50g)** (0.05 mL, 0.45 mmol) and sodium triacetoxyborohydride (256 mg, 2.7 mmol) in anhydrous 1,2-dichloroethane (8 mL) was added 5-bromonicotinaldehyde **(34)** (200.0 mg, 0.91 mmol) and trifluoroacetic acid (0.10 mL, 1.3 mmol) under nitrogen. The resulting mixture was stirred at room temperature for 1 h. Sodium triacetoxyborohydride (256 mg, 2.7 mmol) was added then the reaction was stirred at room temperature for 17 h. After being quenched with saturated NaHCO₃ (3 mL), the aqueous layer was extracted with EtOAc (2 × 20 mL). The combined organic extracts were washed with brine, dried over anhydrous Na₂SO₄, filtered, and concentrated. The residue was purified by column chromatography on silica gel (EtOAc/hexane, 30: 70) to afford **51g** (121.5 mg, 93%) as a yellow oil; **¹H NMR** (CDCl₃, 600 MHz) 8.58 (1 H, s), 8.54 (1 H, s), 7.86 (1 H, s), 6.83-6.80 (2 H, m), 6.72 (1 H, dt, *J* = 7.4, 1.5 Hz), 6.50 (1 H, dd, *J* = 7.6, 1.0 Hz), 4.70 (1 H, br), 4.38 (2 H, s), 3.88 (3 H, s); HRMS (ESI-TOF) *m*/*z:* [M + H]⁺ calculated for C₁₃H₁₄⁷⁹BrN₂O and C₁₃H₁₄⁸¹BrClN₂O 293.0285, 295.0264; found 293.0420, 295.0404.

### N-((5-Bromopyridin-3-yl)methyl)-3-methoxyaniline (51h)

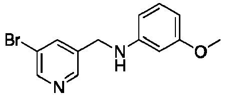

The above compound **(51h)** was prepared from **34** and **50h** following Method L and purified by column chromatography on silica gel (EtOAC/hexane, 30:70 to 45:55) to give **51h** (82%) as a brown oil; **¹H NMR** (CDCl₃, 500 MHz) 8.59 (1 H, d, *J* = 1.5 Hz), 8.54 (1 H, s), 7.85 (1 H, s), 7.09 (1 H, t, *J* = 8.1 Hz), 6.33 (1 H, dd, *J* = 8.1, 1.5 Hz), 6.23 (1 H, dd, *J* = 7.9, 1.5 Hz), 6.16 (1 H, t, *J* = 2.0 Hz), 4.36 (2 H, s), 4.11 (1 H, br), 3.76 (3 H, s); HRMS (ESI-TOF) *m*/*z:* [M + H]⁺ calculated for C₁₃H₁₄⁷⁹BrN₂O and C₁₃H₁₄⁸¹BrClN₂O 293.0285, 295.0264; found 293.0639, 295.0623.

### N-((5-Bromopyridin-3-yl)methyl)-4-methoxyaniline (51i)

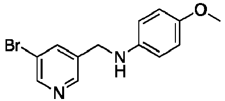

The above compound **(51i)** was prepared from **34** and **50i** following Method L and purified by column chromatography on silica gel (EtOAC/hexane, 40:60 to 50:50) to give 51i (72%) as a yellow oil; **¹H NMR** (CDCl₃, 500 MHz) 8.59 (1 H, d, *J* = 1.5 Hz), 8.53 (1 H, s), 7.86 (1 H, s), 6.78 (1 H, d, *J* = 4.6 Hz), 6.58 (1 H, d, *J* = 9.2 Hz), 4.32 (2 H, s), 3.74 (4 H, m); HRMS (ESI-TOF) *m*/*z:* [M + H]⁺ calculated for C₁₃H₁₄⁷⁹BrN₂O and C₁₃H₁₄⁸¹BrClN₂O 293.0285, 295.0264; found 293.0310, 295.0289.

### 5-(5-(((2-Fluorophenyl)amino)methyl)pyridin-3-yl)-1H-benzo[d]imidazol-2(3H)-one (52a)

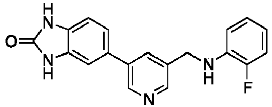

The above compound **(52a)** was prepared from **8b** and **51a** following Method E and purified by column chromatography on silica gel (MeOH/CH₂Cl₂, 5:95 to 10:90) to give **52a** (35%) as a yellow solid; **¹H NMR** (DMSO-d₆, 500 MHz) 10.73 (1 H, s), 10.71 (1 H, s), 8.66 (1 H, d, *J* = 2.0 Hz), 8.51 (1 H, s), 7.97 (1 H, s), 7.24 (1 H, dd, *J* = 8.1, 1.5 Hz), 7.16 (1 H, s), 7.02-6.98 (2 H, m), 6.88 (1 H, t, *J* = 8.1 Hz), 6.69 (1 H, t, *J* = 9.2 Hz), 6.54-6.50 (1 H, m), 6.26 (1 H, t, *J* = 6.6 Hz), 4.42 (2 H, d, *J* = 6.1 Hz); HRMS (ESI-TOF) *m*/*z:* [M + H]⁺ calculated for C₁₉H₁₆FN₄O 335.1303; found 335.1294.

### 5-(5-(((3-Fluorophenyl)amino)methyl)pyridin-3-yl)-1H-benzo[d]imidazol-2(3H)-one (52b)

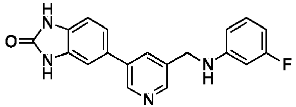

The above compound **(52b)** was prepared from **8b** and **51b** following Method E and purified by column chromatography on silica gel (MeOH/CH₂Cl₂, 5:95 to 10:90) to give **52b** (37%) as a pale yellow solid; **¹H NMR** (DMSO-*d₆*, 500 MHz) 10.74 (1 H, s), 10.72 (1 H, s), 8.68 (1 H, d, *J* = 1.5 Hz), 8.50 (1 H, s), 7.95 (1 H, s), 7.25 (1 H, dd, *J* = 8.1, 1.5 Hz), 7.17 (1 H, s), 7.07-7.01 (2 H, m), 6.61 (1 H, t, *J* = 6.1 Hz), 6.45 (1 H, dd, *J* = 8.4, 1.5 Hz), 6.38 (1 H, dt, *J* = 12.2, 2.0 Hz), 6.28 (1 H, td, *J =* 8.1, 2.0 Hz), 4.35 (2 H, d, *J* = 6.1 Hz); HRMS (ESI-TOF) *m*/*z:* [M + H]⁺ calculated for C₁₉H₁₆FN₄O 335.1303; found 335.1390.

### 5-(5-(((4-Fluorophenyl)amino)methyl)pyridin-3-yl)-1H-benzo[d]imidazol-2(3H)-one (52c)

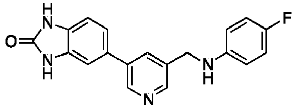

The above compound **(52c)** was prepared from **8b** and **51c** following Method E and purified by column chromatography on silica gel (MeOH/CH₂Cl₂, 5:95 to 10:90) to give **52c** (35%) as a yellow solid; **¹H NMR** (DMSO-*d₆*, 500 MHz) 10.74 (1 H, s), 10.71 (1 H, s), 8.67 (1 H, s), 8.49 (1 H, s), 7.94 (1 H, s), 7.24 (1 H, d, *J* = 8.6 Hz), 7.17 (1 H, s), 7.02 (1 H, d, *J* = 8.1 Hz), 6.89 (2 H, t, *J* = 8.6 Hz), 6.60 (2 H, dd, *J* = 8.9, 4.6 Hz), 6.21 (1 H, t, *J* = 5.6 Hz), 4.32 (2 H, d, *J* = 6.1 Hz); HRMS (ESI-TOF) *m*/*z:* [M + H]⁺ calculated for C₁₉H₁₆FN₄O 335.1303; found 335.1327.

### 5-(5-(((2-Chlorophenyl)amino)methyl)pyridin-3-yl)-1H-benzo[d]imidazol-2(3H)-one (52d)

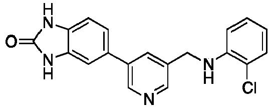

The above compound **(52d)** was prepared from **8b** and **51d** following Method E and purified by column chromatography on silica gel (MeOH/CH₂Cl₂, 5:95 to 10:90) to give **52d** (38%) as a yellow solid; **¹H NMR** (DMSO-d₆, 500 MHz) 10.74 (1 H, s), 10.72 (1 H, s), 8.66 (1 H, d, *J* = 1.5 Hz), 8.49 (1 H, s), 7.95 (1 H, s), 7.23 (2 H, t, *J* = 7.6 Hz), 7.15 (1 H, s), 7.05-7.01 (2 H, m), 6.66 (1 H, d, *J* = 8.1 Hz), 6.55 (1 H, t, *J* = 7.1 Hz), 6.22 (1 H, t, *J* = 6.1 Hz), 4.90 (2 H, d, *J* = 6.6 Hz); HRMS (ESI-TOF) *m*/*z:* [M + H]⁺ calculated for C₁₉H₁₆ClN₄O 351.1008; found 351.1012.

### 5-(5-(((3-Chlorophenyl)amino)methyl)pyridin-3-yl)-1H-benzo[d]imidazol-2(3H)-one (52e)

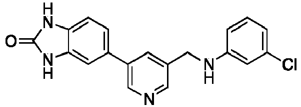

The above compound **(52e)** was prepared from **8b** and **51e** following Method E and purified by column chromatography on silica gel (MeOH/CH₂Cl₂, 5:95 to 10:90) to give **52e** (26%) as a light brown solid; **¹H NMR** (DMSO-*d₆*, 500 MHz) 10.74 (1 H, s), 10.72 (1 H, s), 8.66 (1 H, d, *J* = 1.5 Hz), 8.49 (1 H, s), 7.95 (1 H, s), 7.23 (2 H, t, *J* = 7.6 Hz), 7.15 (1 H, s), 7.05-7.01 (2 H, m), 6.66 (1 H, d, *J* = 8.1 Hz), 6.55 (1 H, t, *J* = 7.1 Hz), 6.22 (1 H, t, *J* = 6.1 Hz), 4.90 (2 H, d, *J* = 6.6 Hz); HRMS (ESI-TOF) *m*/*z:* [M + H]⁺ calculated for C₁₉H₁₆ClN₄O 351.1008; found 351.1009.

### 5-(5-(((4-Chlorophenyl)amino)methyl)pyridin-3-yl)-1H-benzo[d]imidazol-2(3H)-one (52f)

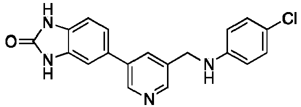

The above compound **(52f)** was prepared from **8b** and **51f** following Method D and purified by column chromatography on silica gel (MeOH/CH₂Cl₂, 5:95) to give **52f** (66%) as a white solid; **¹H NMR** (DMSO-d₆, 600 MHz) 10.76 (1 H, s), 10.74 (1 H, s), 8.68 (1 H, s), 8.48 (1 H, s), 7.94 (1 H, s), 7.24 (1 H, d, *J =* 7.3 Hz), 7.17 (1 H, s), 7.06 (2 H, d, *J* = 8.5 Hz), 7.02 (1 H, d, *J =* 7.3 Hz), 6.62 (1 H, d, *J=* 8.5 Hz), 6.50 (1 H, t, *J =* 6.1 Hz), 4.34 (2 H, d, *J* = 4.9 Hz); HRMS (ESI-TOF) *m*/*z:* [M + H]⁺ calculated for C₁₉H₁₆ClN₄O 351.1008; found 351.1016.

### 5-(5-(((2-Methoxyphenyl)amino)methyl)pyridin-3-yl)-1H-benzo[d]imidazol-2(3H)-one (52g)

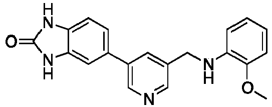

The above compound (52g) was prepared from 8b and 51g following Method E and purified by column chromatography on silica gel (MeOH/CH₂Cl₂, 5:95 to 10:90) to give 52g (34%) as a yellow solid; **¹H NMR** (DMSO-d₆, 500 MHz) 10.73 (1 H, s), 10.70 (1 H, s), 8.65 (1 H, s), 8.48 (1 H, s), 7.93 (1 H, s), 7.22 (1 H, d, *J* = 6.6 Hz), 7.15 (1 H, s), 7.01 (1 H, d, *J* = 8.1 Hz), 6.80 (1 H, d, *J* = 7.6 Hz), 6.68 (1 H, t, *J* = 7.6 Hz), 6.52-6.48 (2 H, m), 5.70 (1 H, t, *J* = 6.1 Hz), 4.40 (2 H, d, *J* = 6.1 Hz), 3.79 (3 H, s); HRMS (ESI-TOF) *m*/*z:* [M + H]⁺ calculated for C₂₀H₁₉N₄O₂ 347.1503; found 347.1520.

### 5-(5-(((3-Methoxyphenyl)amino)methyl)pyridin-3-yl)-1H-benzo[d]imidazol-2(3H)-one (52h)

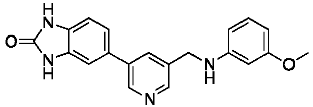

The above compound **(52h)** was prepared from **8b** and **51h** following Method E and purified by column chromatography on silica gel (MeOH/CH₂Cl₂, 5:95 to 10:90) to give **52h** (39%) as a yellow solid; **¹H NMR** (DMSO-d₆, 500 MHz) 10.74 (1 H, s), 10.71 (1 H, s), 8.66 (1 H, d, *J* = 2.0 Hz), 8.49 (1 H, s), 7.94 (1 H, s), 7.24 (1 H, dd, *J* = 8.1, 1.5 Hz), 7.17 (1 H, s), 7.01 (1 H, d, *J* = 7.6 Hz), 6.94 (1 H, t, *J* = 7.6 Hz), 6.30 (1 H, t, *J* = 6.1 Hz), 6.22 (1 H, dd, *J* = 8.1, 1.5 Hz), 6.16 (1 H, s), 6.11 (1 H, dd, *J* = 7.9, 1.5 Hz), 4.33 (2 H, d, *J* = 6.1 Hz), 3.62 (3 H, s); HRMS (ESI-TOF) *m*/*z:* [M + H]⁺ calculated for C₂₀H₁₉N₄O₂ 347.1503; found 347.1521.

### 5-(5-(((4-Methoxyphenyl)amino)methyl)pyridin-3-yl)-1H-benzo[d]imidazol-2(3H)-one (52i)

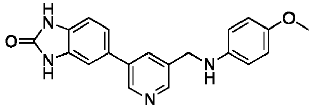

The above compound **(52i)** was prepared from **8b** and **51i** following Method E and purified by column chromatography on silica gel (MeOH/CH₂Cl₂, 5:95 to 10:90) to give **52i** (40%) as a yellow solid; **¹H NMR** (DMSO-d₆, 500 MHz) 10.74 (1 H, s), 10.71 (1 H, s), 8.66 (1 H, d, *J* = 2.0 Hz), 8.48 (1 H, d, *J* = 1.5 Hz), 7.94 (1 H, s), 7.24 (1 H, d, *J* = 8.1 Hz), 7.17 (1 H, s), 7.01 (1 H, d, *J* = 8.1 Hz), 6.68 (2 H, d, *J* = 8.6 Hz), 6.57 (2 H, d, *J* = 9.2 Hz), 5.88 (1 H, t, *J* = 6.1 Hz), 4.29 (2 H, d, *J* = 6.1 Hz), 3.60 (3 H, s); HRMS (ESI-TOF) *m*/*z:* [M + H]⁺ calculated for C₂₀H₁₉N₄O₂ 347.1503; found 347.1504.

The synthetic schemes for compounds **52a-i,** discussed *supra,* are shown in Figure 12.

### Example 14 - General Procedure for the Preparation of tert-Butyl (2-(3-nitrophenoxy)alkyl)carbamate-Method M

### tert-Butyl (2-(3-nitrophenoxy)ethyl)carbamate (54a)

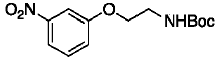

To a solution of 3-nitorphenol **(53)** (300 mg, 2.16 mmol) in anhydrous THF (5 mL) was added triphenylphosphine (735 mg, 2.80 mmol) and tert-Butyl (2-hydroxyethyl)carbamate (452 mg, 2.80 mmol) under nitrogen at 0 °C. Diisopropyl (E)-diazene-1,2-dicarboxylate (0.55 mL, 2.80 mmol) was slowly added. The resulting mixture was stirred at 0 °C and slowly increased to room temperature for 21 h. After being quenched with water (5 mL), the aqueous layer was extracted with EtOAc (2 × 20 mL). The combined organic extracts were washed with brine, dried over anhydrous Na₂SO₄, filtered, and concentrated. The residue was purified by column chromatography on silica gel (EtOAc/hexane, 10: 90 to 15:85) to afford **54a** (567.2 mg, 93%) as a yellow oil; **¹H NMR** (CDCl₃, 600 MHz) 7.81 (1 H, d, *J* = 8.5 Hz), 7.70 (1 H, s), 7.42 (1 H, t, *J* = 8.4 Hz), 7.21 (1 H, d, *J* = 8.5 Hz), 5.01 (1 H, br), 4.09 (2 H, s), 3.57 (2 H, s), 1.44 (9 H, s); HRMS (ESI-TOF) *m*/*z:* [M + H - C₄H_{8]}⁺ calculated for C₉H₁₁N₂O₅ 227.0663; found 227.0658.

### tert-Butyl (2-(2-(3-nitrophenoxy)ethoxy)ethyl)carbamate (54b)

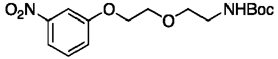

The above compound **(54b)** was prepared from **53** and *tert*-butyl (2-(2-hydroxyethoxy)ethyl)carbamate following Method M and purified by column chromatography on silica gel (EtOAc/hexane, 20: 80 to 40:60) to give **54b** (94%) as a yellow oil; **¹H NMR** (CDCl₃, 600 MHz) 7.76 (1 H, d, *J* = 7.3 Hz), 7.70 (1 H, s), 7.37 (1 H, t, *J* = 7.3 Hz), 7.21 (1 H, s), 4.89 (1 H, quit, *J* = 6.1 Hz), 4.15 (2 H, t, *J* = 4.9 Hz), 3.80 (2 H, t, *J* = 4.9 Hz), 3.56 (2 H, t, *J* = 4.9 H), 3.29 (2 H, q, *J* = 3.7 Hz), 1.37 (9 H, s); HRMS (ESI-TOF) *m*/*z:* [M + H - C₄H₈]⁺ calculated for C₁₁H₁₅N₂O₆ 271.0925; found 271.0970.

### Example 15 - General Procedure for the Preparation of tert-Butyl (2-(3-aminophenoxy)alkyl)carbamate-Method N

### tert-Butyl (2-(3-aminophenoxy)ethyl)carbamate (55a)

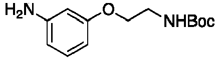

To a solution of **54a** (540 mg, 1.91 mmol) in anhydrous MeOH (15 mL) was added 10% palladium on carbon (54 mg) was added. The resulting mixture was purged with hydrogen then stirred at room temperature for 16 h. The reaction was filtered through a Celite pad and solvent was removed under reduced pressure. The crude product was extracted with EtOAc (2 × 30 mL) and water (10 mL). The combined organic extracts were washed with brine, dried over anhydrous Na₂SO₄, filtered, and concentrated. The residue was purified by column chromatography on silica gel (EtOAc/hexane, 20:80 to 30:70) to afford **55a** (365.9 mg, 76%) as a brown oil; **¹H NMR** (CDCl₃, 600 MHz) 7.05 (1 H, t, *J* = 7.3 Hz), 6.31-6.29 (2 H, m), 6.23 (1 H, s), 4.98 (1 H, br), 3.97 (2 H, t, *J* = 2.1 Hz), 3.66 (2 H, br), 3.51 (2 H, q, *J* = 4.9 Hz), 1.45 (9 H, s); HRMS (ESI-TOF) *m*/*z:* [M + H]⁺ calculated for C₁₃H₂₁N₂O₃ 253.1547; [M + H]⁺ found 253.1733.

### tert-Butyl (2-(2-(3-aminophenoxy)ethoxy)ethyl)carbamate (55b)

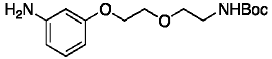

The above compound **(55b)** was prepared from **54b** following Method N and purified by column chromatography on silica gel (EtOAc/hexane, 20: 80 to 30:70) to give **54b** (81%) as a brown oil; **¹H NMR** (CDCl₃, 600 MHz) 7.04 (1 H, t, *J* = 7.3 Hz), 6.33 (1 H, dd, *J* = 8.5, 2.4 Hz), 6.30 (1 H, dd, *J* = 8.5, 2.4 Hz), 6.27 (1 H, t), 4.99 (1 H, br), 4.07 (2 H, t, *J* = 4.9 Hz), 3.79 (2 H, t, *J* = 4.9 Hz), 3.67 (2 H, br), 3.60 (2 H, t, *J* = 4.9 H), 3.34 (2 H, q, *J* = 3.7 Hz), 1.44 (9 H, s); HRMS (ESI-TOF) *m*/*z:* [M + H]⁺ calculated for C₁₅H₂₅N₂O₄ 297.1809; found 297.1868.

### Example 16 - General Procedure for the Preparation of 5-Bromo-N-alkyl-2-nitroaniline-Method O

### 5-Bromo-N-ethyl-2-nitroaniline (57a)

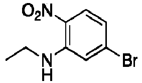

To a solution of 4-bromo-2-fluoro-1-nitrobenzene **(56)** (500 mg, 2.27 mmol) and potassium carbonate (628 mg, 4.55 mmol) in anhydrous DMF (5 mL) was added 2 M ethanamine in THF (2.3 mL, 4.55 mmol). The resulting mixture was stirred at 80 °C for 22 h. After being quenched with water (10 mL), the aqueous layer was extracted with EtOAc (2 × 30 mL). The combined organic extracts were washed with brine, dried over anhydrous Na₂SO₄, filtered, and concentrated. The residue was purified by column chromatography on silica gel (EtOAc/hexane, 5: 95 to 10:90) to afford **57a** (520.0 mg, 93%) as a yellow solid; **¹H NMR** (CDCl₃, 500 MHz) 8.03 (1 H, d, *J* = 9.2 Hz), 7.97 (1 H, br), 7.01 (1 H, d, *J* = 1.5 Hz), 6.75 (1 H, dd, *J* = 9.2, 1.5 Hz), 3.36-3.30 (2 H, m), 1.38 (3 H, t, *J* = 7.6 Hz); HRMS (ESI-TOF) *m*/*z:* [M + H]⁺ calculated for C₈H₁₀⁷⁹Br N₂O₂ and C₈H₁₀⁸¹Br N₂O₂ 244.9921, 246.9900; found 244.9920, 246.9898.

### 5-Bromo-N-isopropyl-2-nitroaniline (57b)

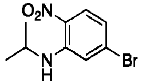

The above compound **(57b)** was prepared from **56** and isopropylamine following Method O and purified by column chromatography on silica gel (EtOAc/hexane, 5:95 to 10:90) to give **57b** (99%) as an orange solid; **¹H NMR** (CDCl₃, 500 MHz) 8.03-8.00 (2 H, m), 7.02 (1 H, d, *J* = 1.5 Hz), 6.72 (1 H, dd, *J* = 8.9, 2.0 Hz), 3.84-3.74 (1 H, m), 1.34 (6 H, d, *J* = 6.6 Hz); HRMS (ESI-TOF) *m*/*z:* [M + H]⁺ calculated for C₉H₁₂⁷⁹Br N₂O₂ and C₉H₁₂⁸¹Br N₂O₂259.0077, 261.0057; found 259.0077, 261.0059.

### 5-Bromo-N-cyclopropyl-2-nitroaniline (57c)

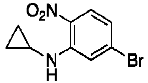

The above compound **(57c)** was prepared from **56** and cyclopropylamine following Method O and purified by column chromatography on silica gel (EtOAc/hexane, 5:95 to 10:90) to give **57c** (99%) as a yellow solid; **¹H NMR** (CDCl₃, 500 MHz) 8.07 (1 H, br), 8.01 (1 H, d, *J* = 9.2 Hz), 7.48 (1 H, d, *J* = 2.0 Hz), 6.81 (1 H, dd, *J* = 9.2, 2.0 Hz), 2.58-2.55 (1 H, m), 0.97-0.93 (2 H, m), 0.69-0.66 (2 H, m); HRMS (ESI-TOF) *m*/*z:* [M + H]⁺ calculated for C₉H₁₀⁷⁹BrN₂O₂ and C₉H₁₀⁸¹BrN₂O₂ 256.9921, 258.9900; found 256.9917, 258.9902.

### 5-Bromo-N-cyclopentyl-2-nitroaniline (57d)

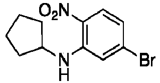

The above compound **(57d)was** prepared from **56** and cyclopentylamine following Method O and purified by column chromatography on silica gel (EtOAc/hexane, 5:95 to 10:90) to give **57d** (97%) as a yellow solid; **¹H NMR** (CDCl₃, 600 MHz) 8.11 (1 H, br), 8.02 (1 H, d, *J =* 9.8 Hz), 7.05 (1 H, s), 6.73 (1 H, dd, *J* = 8.5, 2.4 Hz), 3.94-3.89 (1 H, m), 2.14-2.09 (2 H, m), 1.83-1.78 (2 H, m), 1.73-1.66 (2 H, m), 1.65-1.61 (2 H, m).

### 5-Bromo-2-nitro-N-phenylaniline (57e)

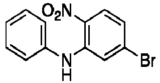

The above compound **(57e)** was prepared from **56** and aniline following Method O. The reaction was stirred at 80°C for 16 h and purified by column chromatography on silica gel (EtOAc/hexane, 5:95 to 10:90) to give **57e** (70%) as an orange solid; **¹H NMR** (CDCl₃, 500 MHz) 9.51 (1 H, br), 8.07 (1 H, d, *J* = 9.2 Hz), 7.46 (2 H, t, *J* = 7.6 Hz), 7.33-7.27 (4 H, m), 6.88 (1 H, dd, *J* = 8.9, 2.0 Hz); HRMS (ESI-TOF) *m*/*z:* [M + H]⁺ calculated for C₁₂H₁₀⁷⁹BrN₂O₂ and C₁₂H₁₀⁸¹BrN₂O₂ 292.9921, 294.9900; found 292.9924, 294.9898

### Example 17 - General Procedure for the Preparation of 5-Bromo-N-phenyl-2-nitroaniline-Method P

### N-(5-Bromo-2-nitrophenyl)pyridin-2-amine (57f)

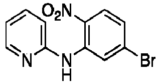

To a solution of pyridine-2-amine (257.0 mg, 2.73 mmol) in anhydrous THF (5 mL) was added potassium tert-butoxide (408 mg, 3.64 mmol) at 0°C. The resulting mixture was purged with nitrogen and stirred at 0 °C for 1 h. 4-Bromo-2-fluoro-1-nitrobenzene **(56)** (400 mg, 1.82 mmol) was added and the reaction was stirred at 0°C for 1 h. After being quenched with water (10 mL), the aqueous layer was extracted with EtOAc (2 × 30 mL). The combined organic extracts were washed with brine, dried over anhydrous Na₂SO₄, filtered, and concentrated. The residue was purified by column chromatography on silica gel (EtOAc/hexane, 5:95 to 10:90) to afford **57f** (418.3 mg, 78%) as an orange solid; **¹H NMR** (CDCl₃, 500 MHz) 10.28 (1 H, br), 9.19 (1 H, d, *J* = 2.0 Hz), 8.40 (1 H, d, *J* = 5.1 Hz), 8.10(1 H, d, *J* = 9.2 Hz), 7.67 (1 H, td, *J* = 8.9, 2.0 Hz), 7.07 (1 H, dd, *J* = 8.9, 2.0 Hz), 7.01 (1 H, dd, *J* = 7.1, 5.1 hz), 6.95 (1 H, d, *J* = 8.1 Hz); HRMS (ESI-TOF) *m*/*z:* [M + H]⁺ calculated for C₁₁H₉⁷⁹BrN₃O₂ and C₁₁H₉⁸¹BrN₃O₂ 293.9873, 295.9853; found 293.9863, 295.9883.

### N-(5-Bromo-2-nitrophenyl)pyridin-3-amine (57g)

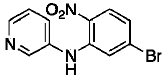

The above compound (**57g**) was prepared from **56** and pyridine-3-amine following Method P and purified by column chromatography on silica gel (EtOAc/hexane, 40:60) to give **57g** (63%) as an orange solid; **¹H NMR** (CDCl₃, 500 MHz) 9.44 (1 H, br), 8.62 (1 H, d, *J* = 2.5 Hz), 8.55 (1 H, dd, *J* = 4.8, 1.5 Hz), 8.10 (1 H, d, *J* = 9.2 Hz), 7.63 (1 H, dd, *J* = 8.1, 2.0 Hz), 7.41 (1 H, dd, *J* = 8.4, 4.6 Hz), 6.97 (1 H, dd, *J* = 9.2, 2.0 Hz); HRMS (ESI-TOF) *m*/*z*: [M + H]⁺ calculated for C₁₁H₉⁷⁹BrN₃O₂ and C₁₁H₉⁸¹BrN₃O₂ 293.9873, 295.9853; found 293.9889, 295.9868.

### N-(5-Bromo-2-nitrophenyl)pyridin-4-amine (57h)

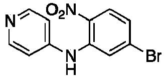

The above compound (**57h**) was prepared from **56** and pyridine-4-amine following Method P and purified by column chromatography on silica gel (MeOH/CH₂Cl₂, 5:95) to give **57h** (60%) as a brown solid; **¹H NMR** (CDCl₃, 500 MHz) 9.37 (1 H, br), 8.57 (2 H, d, *J* = 5.1 Hz), 8.09 (2 H, d, *J* = 9.2 Hz), 7.70 (1 H, d, *J* = 1.5 Hz), 7.14 (2 H, dd, *J* = 4.6, 1.5 Hz), 7.10 (1 H, dd, *J* = 8.9, 1.5 Hz); HRMS (ESI-TOF) *m*/*z*: [M + H]⁺ calculated for C₁₁H₉⁷⁹BrN₃O₂ and C₁₁H₉⁸¹BrN₃O₂ 293.9873, 295.9853; found 293.9853, 295.9832.

### 5-Bromo-N-(2-(2-methoxyethoxy)phenyl)-2-nitroaniline (57i)

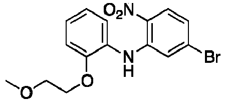

The above compound (**57i**) was prepared from **56** and 2-(2-methoxyethoxy)aniline following Method P. The reaction was stirred from 0°C to room temperature for 2 h and purified by column chromatography on silica gel (EtOAc/hexane, 10:90 to 20:80) to give **57h** (40%) as an orange liquid; **¹H NMR** (CDCl₃, 500 MHz) 9.56 (1 H, br), 8.06 (1 H, d, *J* = 9.2 Hz), 7.40 (2 H, d, *J* = 2.0 Hz), 7.37 (1 H, dd, *J* = 7.4, 1.5 Hz), 7.20 (1 H, td, *J* = 7.1, 2.0 Hz), 7.04-7.01 (2 H, m), 6.87 (1 H, dd, *J* = 8.6, 2.0 Hz), 4.18 (2 H, t, *J* = 4.6 Hz), 3.72 (2 H, t, *J* = 4.6 Hz), 3.39 (3 H, s); HRMS (ESI-TOF) *m*/*z:* [M + H]⁺ calculated for C₁₅H₁₆⁷⁹BrN₂O₄ and C₁₅H₁₆⁸¹BrN₂O₄ 367.0288, 369.0268; found 367.0291, 369.0273.

### 5-Bromo-N-(3-(2-methoxyethoxy)phenyl)-2-nitroaniline (57j)

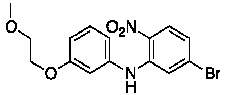

The above compound (**57j**) was prepared from **56** and 3-(2-methoxyethoxy)aniline following Method P. The reaction was stirred from 0°C to room temperature for 4 h and purified by column chromatography on silica gel (EtOAc/hexane, 10:90 to 20:80) to give **57j** (42%) as an orange solid; **¹H NMR** (CDCl₃, 600 MHz) 9.64 (1 H, s), 8.05 (1 H, d, *J* = 9.8 Hz), 7.35-7.32 (2 H, m), 6.88-6.83 (4 H, m), 4.14 (2 H, t, *J* = 4.5 Hz), 3.77 (2 H, t, *J* = 4.5 Hz), 3.46 (3 H, s); HRMS (ESI-TOF) *m*/*z:* [M + H]⁺ calculated for C₁₅H₁₆⁷⁹BrN₂O₄ and C₁₅H₁₆⁸¹BrN₂O₄ 367.0288, 369.0268; found 367.0285, 369.0268.

### 5-Bromo-N-(4-(2-methoxyethoxy)phenyl)-2-nitroaniline (57k)

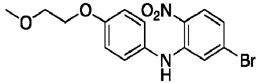

The above compound (**57k**) was prepared from **56** and 4-(2-methoxyethoxy)aniline following Method P. The reaction was stirred from 0°C to room temperature for 4 h and purified by column chromatography on silica gel (EtOAc/hexane, 10:90 to 20:80) to give **57k** (30%) as an orange liquid; **¹H NMR** (CDCl₃, 600 MHz) 9.40 (1 H, br), 8.05 (1 H, d, *J* = 9.1 Hz), 7.18 (2 H, d, *J* = 8.6 Hz), 7.11 (1 H, d, *J* = 1.5 Hz), 7.01 (2 H, d, *J* = 9.2 Hz), 6.82 (1 H, dd, *J* = 9.2, 2.0 Hz), 4.16 (2 H, t, *J* = 4.6 Hz), 3.78 (2 H, t, *J* = 4.6 Hz), 3.48 (3 H, s); HRMS (ESI-TOF) *m*/*z*: [M + H]⁺ calculated for C₁₅H₁₆⁷⁹BrN₂O₄ and C₁₅H₁₆⁸¹BrN₂O₄ 367.0288, 369.0268; found 367.0295, 369.0277.

### tert-Butyl (2-(3-((5-bromo-2-nitrophenyl)amino)phenoxy)ethyl)carbamate (57l)

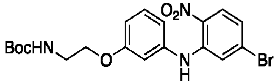

The above compound (**57l**) was prepared from **56** and **55a** following Method O. The reaction was stirred at 80°C for 16 h and purified by column chromatography on silica gel (EtOAc/hexane, 20:80 to 30:70) to give **57e** (32%) as an orange oil; **¹H NMR** (CDCl₃, 600 MHz) 9.46 (1 H, s), 8.06 (1 H, d, *J* = 9.8 Hz), 7.36-7.33 (2 H, m), 6.88 (2 H, d, *J* = 7.3 Hz), 6.82-6.79 (2 H, m), 4.99 (1 H, br), 4.04 (2 H, t, *J* = 4.9 Hz), 3.56 (2 H, q, *J* = 4.9 Hz), 1.45 (9 H, s); HRMS (ESI-TOF) *m*/*z:* [M + H - C₄H₈]⁺ calculated for C₁₅H₁₅⁷⁹BrN₃O₅ and C₁₅H₁₅⁸¹BrN₃O₅ 396.0190, 398.0170; found 396.0197, 398.0169

### tert-Butyl (2-(2-(3-((5-bromo-2-nitrophenyl)amino)phenoxy)ethoxy)ethyl)carbamate (57m)

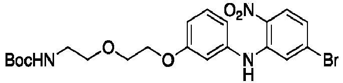

The above compound (**57m**) was prepared from **56** and **55b** following Method O. The reaction was stirred at 80°C for 16 h and purified by column chromatography on silica gel (EtOAc/hexane, 20:80 to 60:40) to give **57e** (35%) as an orange oil; **¹H NMR** (CDCl₃, 600 MHz) 9.46 (1 H, s), 8.06 (1 H, d, *J=* 8.5 Hz), 7.36-7.33 (2 H, m), 6.89-6.82 (4 H, m), 4.95 (1 H, br), 4.14 (2 H, t, *J* = 4.9 Hz), 3.84 (2 H, t, *J* = 4.9 Hz), 3.62 (2 H, t, *J* = 4.9 Hz), 3.35 (2 H, q, *J* = 4.9 Hz), 1.43 (9 H, s); HRMS (ESI-TOF) *m*/*z*: [M + H - C₅H₈O₂]⁺ calculated for C₁₆H₁₉⁷⁹BrN₃O₄ and C₁₆H₁₉⁸¹BrN₃O₄ 396.0554, 398.0533; found 396.0533, 398.0522

### Example 18 - General Procedure for the Preparation of 5-bromo-N¹-alkylbenzene-1,2-diamine-Method Q

### 5-Bromo-N¹-ethylbenzene-1,2-diamine (58a)

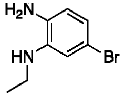

To a solution of **57a** (438 mg, 1.79 mmol) in ethanol (10 mL) and water (2 mL) was added ammonium chloride (507 mg, 9.47 mmol) and iron (499 mg, 8.94 mmol). The resulting mixture was refluxed for 24 h. The reaction was filtered through a Celite pad then solvent was removed under reduced pressure. The crude product was extracted with EtOAc (2 × 40 mL) and water (15 mL). The combined organic extracts were washed with brine, dried over anhydrous Na₂SO₄, filtered, and concentrated. The residue was purified by column chromatography on silica gel (EtOAc/hexane, 15:85 to 20:80) to afford **58a** (343.1 mg, 89%) as a brown liquid; **¹H NMR** (CDCl₃, 500 MHz) 6.76-6.73 (2 H, m), 6.57 (1 H, d, *J* = 8.1 Hz), 3.27 (3 H, br), 3.12 (2 H, q, *J* = 7.1 Hz), 1.30 (1 H, t, *J* = 7.1 Hz); HRMS (ESI-TOF) *m*/*z*: [M + H]⁺ calculated for C₈H₁₂⁷⁹BrN₂ and C₈H₁₂⁸¹BrN₂ 215.0179, 217.0158; found 215.0232, 217.0207.

### 5-Bromo-N¹-isopropylbenzene-1,2-diamine (58b)

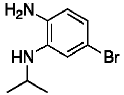

The above compound (**58b**) was prepared from **57b** following Method Q and purified by column chromatography on silica gel (EtOAc/hexane, 30:70) to give **58b** (90%) as a light brown liquid; **¹H NMR** (CDCl₃, 500 MHz) 6.74-6.72 (2 H, m), 6.56 (1 H, d, *J* = 8.6 Hz), 3.59-3.52 (1 H, m), 3.27 (3 H, br), 1.23 (6 H, d, *J* = 6.1 Hz); HRMS (ESI-TOF) *m*/*z*: [M + H]⁺ calculated for C₉H₁₄⁷⁹BrN₂ and C₉H₁₄⁸¹BrN₂ 229.0355, 231.0315; found 229.0472, 231.0448

### Example 19 - General Procedure for the Preparation of 5-bromo-N¹-alkylbenzene-1,2-diamine-Method R

### 5-Bromo-N¹-cyclopentylbenzene-1,2-diamine (58d)

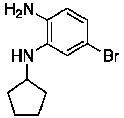

To a solution of **57c** (174 mg, 0.61 mmol) in ethanol (10 mL) and water (6 mL) was added sodium dithionite (850 mg, 4.88 mmol). The resulting mixture was room temperature for 4 h. The reaction was filtered through a Celite pad then solvent was removed under reduced pressure. The crude product was extracted with EtOAc (2 × 40 mL) and water (15 mL). The combined organic extracts were washed with brine, dried over anhydrous Na₂SO₄, filtered, and concentrated. The residue was purified by column chromatography on silica gel (EtOAc/hexane, 15:85) to afford **58d** (106.5 mg, 68%) as a brown liquid; **¹H NMR** (CDCl₃, 600 MHz) 6.75-6.73 (2 H, m), 6.56 (1 H, d, *J* = 8.5 Hz), 3.73 (1 H, quin, *J* = 6.1 Hz), 3.27 (3 H, br), 2.06-2.03 (2 H, m), 1.75-1.71 (2 H, m), 1.67-1.62 (2 H, m), 1.52-1.47 (2 H, m); HRMS (ESI-TOF) *m*/*z*: [M + H]⁺ calculated for C₁₁H₁₆⁷⁹BrN₂ and C₁₁H₁₆⁸¹BrN₂ 255.0492, 257.0471; found 255.0546, 257.0525.

### 5-Bromo-N¹-phenylbenzene-1,2-diamine (58e)

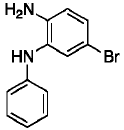

The above compound (**58e**) was prepared from **57e** following Method Q and purified by column chromatography on silica gel (EtOAc/hexane, 20:80 to 30:70) to give **58e** (88%) as a light purple soild; **¹H NMR** (CDCl₃, 500 MHz) 7.26-7.22 (3 H, m), 7.08 (1 H, dd, *J* = 8.4, 2.5 Hz), 6.88 (1 H, t, *J* = 7.1 Hz), 6.78 (2 H, d, *J* = 7.6 Hz), 6.68 (1 H, d, *J* = 8.1 Hz), 5.16 (1 H, br), 3.73 (2 H, br); HRMS (ESI-TOF) *m*/*z*: [M + H]⁺ calculated for C₁₂H₁₂⁷⁹BrN₂ and C₁₂H₁₂⁸¹BrN₂ 263.0179, 265.0158; found 263.0329, 265.0306.

### 5-Bromo-N¹-(pyridin-2-yl)benzene-1,2-diamine (58f)

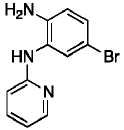

The above compound (**58f**) was prepared from **57f** following Method R and purified by column chromatography on silica gel (EtOAc/hexane, 20:80 to 40:60) to give **58f** (45%) as a brown liquid; **¹H NMR** (CDCl₃, 500 MHz) 8.17 (1 H, d, *J* = 5.1 Hz), 7.47 (1 H, td, *J* = 7.6, 2.0 Hz), 7.36 (1 H, d, *J* = 2.5 Hz), 7.16 (1 H, dd, *J* = 8.6, 2.0 Hz), 6.74-6.92 (2 H, m), 6.47 (1 H, d, *J* = 8.1 Hz), 6.06 (1 H, br), 3.85 (2 H, br); HRMS (ESI-TOF) *m*/*z*: [M + H]⁺ calculated for C₁₁H₁₁⁷⁹BrN₃ and C₁₁H₁₁⁸¹BrN₃ 264.0131, 266.0111; found 264.0148, 266.0128.

### 5-Bromo-N¹-(pyridin-3-yl)benzene-1,2-diamine (58g)

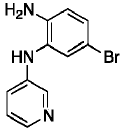

The above compound (**58g**) was prepared from **57g** following Method R and purified by column chromatography on silica gel (MeOH/CH₂Cl₂, 5:95) to give **58g** (42%) as a yellow solid; **¹H NMR** (CDCl₃, 500 MHz) 8.20 (1 H, d, *J* = 2.5 Hz), 8.13 (1 H, dd, *J* = 4.6, 1.5 Hz), 7.23 (1 H, d, *J* = 2.0 Hz), 7.15-7.12 (2 H, m), 7.03-7.01 (1 H, m), 6.70 (1 H, d, *J* = 8.6 Hz), 5.20 (1 H, br), 3.75 (2 H, br); HRMS (ESI-TOF) *m*/*z*: [M + H]⁺ calculated for C₁₁H₁₁⁷⁹BrN₃ and C₁₁H₁₁⁸¹BrN₃ 264.0131, 266.0111; found 264.0131, 266.0110.

### Example 20 - General Procedure for the Preparation of 6-bromo-1-alkyl-1H-benzo[d]imidazol-2(3H)-one-Method S

### 6-Bromo-1-ethyl-1H-benzo[d]imidazol-2(3H)-one (59a)

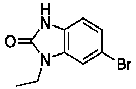

To a solution of **58a** (103.0 mg, 0.48 mmol) in anhydrous dichloromethane (4 mL) was added triphosgene (71.0 mg, 0.24 mmol) and triethylamine (0.17 mL, 1.24 mmol) at 0°C. The resulting mixture was purged with nitrogen then stirred at 0°C for 1 h. After being quenched with water (5 mL), the aqueous layer was extracted with EtOAc (2 × 20 mL). The combined organic extracts were washed with brine, dried over anhydrous Na₂SO₄, filtered, and concentrated. The residue was purified by column chromatography on silica gel (EtOAc/hexane, 20:80 to 40:60) to afford **59a** (95.8 mg, 83%) as an off-solid solid; **¹H NMR** (DMSO-*d₆,* 500 MHz) 10.95 (1 H, s), 7.11 (1 H, dd, *J* = 8.1, 2.0 Hz), 6.91 (1 H, d, *J* = 8.1 Hz), 3.80 (2 H, q, *J* = 7.1 Hz), 1.16 (3 H, t, *J* = 7.1 Hz); HRMS (ESI-TOF) *m*/*z*: [M + H]⁺ calculated for C₉H₁₀⁷⁹BrN₂O and C₉H₁₀⁸¹BrN₂O 240.9972, 242.9951; found 240.9973 242.9953.

### 6-Bromo-1-isopropyl-1H-benzo[d]imidazol-2(3H)-one (59b)

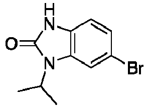

The above compound (**59b**) was prepared from **58b** following Method S and purified by column chromatography on silica gel (EtOAc/hexane, 20:80 to 40:60) to give **59b** (54%) as a pale pink solid; **¹H NMR** (CDCl₃, 500 MHz) 8.07 (1 H, s), 7.17 (1 H, dd, *J* = 8.1, 1.0 Hz), 6.91 (1 H, d, *J* = 8.1 Hz), 4.70-4.65 (1 H, m), 1.53 (6 H, d, *J* = 2.5 Hz); HRMS (ESI-TOF) *m*/*z*: [M + H]⁺ calculated for C₁₀H₁₂⁷⁹BrN₂ and C₁₀H₁₂⁸¹BrN₂O 255.0128, 257.0180; found 255.0128, 257.0106.

### 6-Bromo-1-cyclopropyl-1H-benzo[d]imidazol-2(3H)-one (59c)

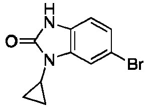

The above compound (**59c**) was prepared from **57c** following Method Q and S. The crude product from first step was used in next step without further purification. The crude product was purified by column chromatography on silica gel (EtOAc/hexane, 30:70 to 60:40) to give **59c** (44% over 2 steps) as a light brown solid; **¹H NMR** (DMSO-*d₆,* 500 MHz) 10.86 (1 H, s), 7.26 (1 H, d, *J* = 1.5 Hz), 7.13 (1 H, dd, *J* = 8.1, 1.5 Hz), 6.88 (1 H, d, *J* = 8.1 Hz), 2.84-2.79 (1 H, m), 1.01-0.98 (2 H, m), 0.86-0.83 (2 H, m); HRMS (ESI-TOF) *m*/*z*: [M + H]⁺ calculated for C₁₀H₁₀⁷⁹BrN₂O and C₁₀H₁₀⁸¹BrN₂O 252.9972, 254.9951; found 252.9983, 254.9963.

### 6-Bromo-1-cyclopentyl-1H-benzo[d]imidazol-2(3H)-one (59d)

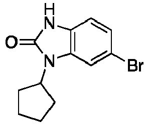

The above compound (**59d**) was prepared from **58d** following Method S and purified by column chromatography on silica gel (EtOAc/hexane, 20:80 to 40:60) to give **59d** (53%) as a white solid; **¹H NMR** (CDCl₃, 600 MHz) 10.59 (1 H, s), 7.18-7.17 (2 H, m), 7.00 (1 H, d, *J* = 8.5 Hz), 4.84 (1 H, quin, *J* = 8.5 Hz), 2.11-1.98 (6 H, m), 1.79-1.75 (2 H, m); HRMS (ESI-TOF) *m*/*z*: [M + H]⁺ calculated for C₁₂H₁₄⁷⁹BrN₂O and C₁₂H₁₄⁸¹BrN₂O 281.0285, 283.0264; found 281.0293, 283.0274.

### 6-Bromo-1-phenyl-1H-benzo[d]imidazol-2(3H)-one (59e)

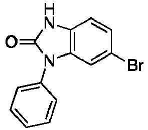

The above compound (**59e**) was prepared from **58e** following Method S. The reaction was stirred at 0°C to room temperature for 16 h and purified by column chromatography on silica gel (EtOAc/hexane, 20:80 to 30:70) to give **59e** (78%) as an off-white solid; **¹H NMR** (CDCl₃, 500 MHz) 9.17 (1 H, s), 7.59-7.56 (2 H, m), 7.53-7.51 (2 H, m), 7.46 (1 H, t, *J* = 7.6 Hz), 7.23 (1 H, dd, *J* = 8.4, 2.0 Hz), 7.17 (1 H, d, *J* = 1.5 Hz), 7.00 (1 H, d, *J* = 8.6 Hz); HRMS (ESI-TOF) *m*/*z*: [M + H]⁺ calculated for C₁₃H₁₀⁷⁹BrN₂O and C₁₃H₁₀⁸¹BrN₂O 288.9972, 290.9951; found 288.9962, 290.9944.

### 6-Bromo-1-(pyridin-2-yl)-1H-benzo[d]imidazol-2(3H)-one (59f)

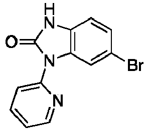

The above compound (**59f**) was prepared from **58f** following Method S. The reaction was stirred at 0°C to room temperature for 1.5 h and purified by column chromatography on silica gel (EtOAc/hexane, 35:65 to 70:30) to give **59f** (44%) as a white solid; **¹H NMR** (CDCl₃, 500 MHz) 8.60-8.59 (1 H, m), 8.27 (1 H, d, *J* = 2.0 Hz), 8.12 (2 H, d, *J* = 8.6 Hz), 7.88 (1 H, td, *J* = 7.4, 2.0 Hz), 7.29-7.26 (1 H, m), 6.96 (1 H, d, *J* = 8.6 Hz); HRMS (ESI-TOF) *m*/*z*: [M + H]⁺ calculated for C₁₂H₉⁷⁹BrN₃O and C₁₂H₉⁸¹BrN₃O 289.9924, 291.9904; found 289.9796, 291.9775.

### 6-Bromo-1-(pyridin--3-yl)-1H-benzo[d]imidazol-2(3H)-one (59g)

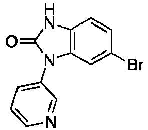

The above compound (**59g**) was prepared from **58g** following Method S and purified by column chromatography on silica gel (MeOH/CH₂Cl₂, 5:95) to give **59g** (59%) as a brown solid; **¹H NMR** (CD₃OD, 500 MHz) 8.78 (1 H, s), 8.64 (1 H, s), 8.06-8.04 (1 H, m), 7.66 (1 H, dd, *J* = 7.9, 4.6 Hz), 7.29 (1 H, dd, *J* = 8.4, 2.0 Hz), 7.18 (1 H, d, *J* = 1.5 Hz), 7.07 (1 H, d, *J* = 8.6 Hz); HRMS (ESI-TOF) *m*/*z*: [M + H]⁺ calculated for C₁₂H₉⁷⁹BrN₃O and C₁₂H₉⁸¹BrN₃O 289.9924, 291.9904; found 289.9923, 291.9903.

### 6-Bromo-1-(pyridin-4-yl)-1H-benzo[d]imidazol-2(3H)-one (59h)

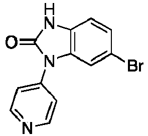

The above compound (**59h**) was prepared from **57h** following Method R and S. The crude product from first step was used in next step without further purification. The crude product was purified by column chromatography on silica gel (MeOH/CH₂Cl₂, 5:95) to give **59h** (20% over 2 steps) as a pale yellow solid; **¹H NMR** (DMSO-*d₆,* 500 MHz) 11.50 (1 H, s), 8.73 (2 H, d, *J* = 6.1 Hz), 7.66 (2 H, d, *J* = 6.1 Hz), 7.38 (1 H, d, *J* = 1.5 Hz), 7.28 (1 H, dd, *J* = 8.1, 2.0 Hz), 7.05 (1 H, d, *J* = 8.1 Hz); HRMS (ESI-TOF) *m*/*z*: [M + H]⁺ calculated for C₁₂H₉⁷⁹BrN₃O and C₁₂H₉⁸¹BrN₃O 289.9924, 291.9904; found 289.9938, 291.9920.

### 6-Bromo-1-(2-(2-methoxyethoxy)phenyl)-1H-benzo[d]imidazol-2(3H)-one (59i)

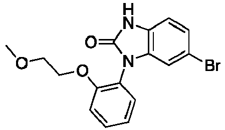

The above compound (**59i**) was prepared from **58i** following Method S and purified by column chromatography on silica gel (EtOAc/hexane, 40:60 to 60:40) to give **59i** (61%) as a white solid; **¹H NMR** (CDCl₃, 500 MHz) 10.25 (1 H, s), 7.47-7.42 (2 H, m), 7.18-7.13 (3 H, m), 6.96 (1 H, d, *J* = 8.1 Hz), 6.86 (1 H, s), 4.18-4.12 (2 H, m), 3.56 (2 H, t, *J* = 4.1 Hz), 3.18 (3 H, s); HRMS (ESI-TOF) *m*/*z*: [M + H]⁺ calculated for C₁₆H₁₆⁷⁹BrN₂O₃ and C₁₆H₁₆⁸¹BrN₂O₃ 363.0339, 365.0319; found 363.0310. 365.0292.

### 6-Bromo-1-(3-(2-methoxyethoxy)phenyl)-1H-benzo[d]imidazol-2(3H)-one (59j)

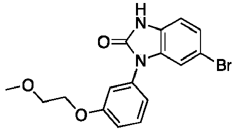

The above compound (**59j**) was prepared from **58j** following Method S and purified by column chromatography on silica gel (EtOAc/hexane, 50:50) to give **59j** (68%) as a white solid; **¹H NMR** (CD₃OD, 500 MHz) 7.48 (1 H, t, *J* = 7.6 Hz), 7.25 (1 H, dd, *J* = 8.1, 1.5 Hz), 7.11-7.03 (5 H, m), 4.18 (2 H, t, *J* = 4.6 Hz), 3.76. (2 H, t, *J* = 4.6 Hz), 3.42 (3 H, s); HRMS (ESI-TOF) *m*/*z*: [M + H]⁺ calculated for C₁₆H₁₆⁷⁹BrN₂O₃ and C₁₆H₁₆⁸¹BrN₂O₃ 363.0339, 365.0319; found 363.0034. 365.0017.

### 6-Bromo-1-(4-(2-methoxyethoxy)phenyl)-1H-benzo[d]imidazol-2(3H)-one (59k)

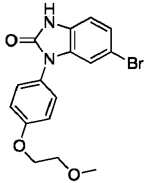

The above compound (**59k**) was prepared from **58k** following Method S and purified by column chromatography on silica gel (EtOAc/hexane, 40:60 to 60:40) to give **59k** (37%) as a pale yellow solid; **¹H NMR** (CDCl₃, 500 MHz) 9.97 (1 H, s), 7.40 (2 H, d, *J* = 8.6 Hz), 7.20 (1 H, d, *J* = 8.1 Hz), 7.11-7.08 (3 H, m) 6.99 (1 H, d, *J* = 8.1 Hz), 4.20 (2 H, t, *J* = 4.6 Hz), 3.80 (2 H, t, *J* = 4.6 Hz), 3.48 (3 H, s); HRMS (ESI-TOF) *m*/*z*: [M + H]⁺ calculated for C₁₆H₁₆⁷⁹BrN₂O₃ and C₁₆H₁₆⁸¹BrN₂O₃ 363.0339, 365.0319; found 363.0347. 365.0329.

### tert-Butyl (2-(3-(6-bromo-2-oxo-2,3-dihydro-1H-benzo[d]imidazol-1-yl)phenoxy)ethyl)carbamate (59l)

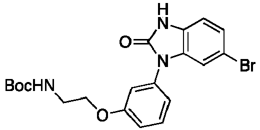

The above compound (**59l**) was prepared from **58l** following Method S. The reaction was stirred at 0°C to room temperature for 16 h and purified by column chromatography on silica gel (EtOAc/hexane, 40:60 to 60:40) to give **59l** (85%) as a light brown solid; **¹H NMR** (CDCl₃, 600 MHz) 10.55 (1 H, s), 7.47 (1 H, t, *J* = 7.3 Hz), 7.21 (1 H, d, *J* = 8.5 Hz), 7.16 (1 H, s), 7.10 (1 H, d, *J* = 7.3 Hz), 7.06 (1 H, s), 7.02-6.98 (2 H, m), 5.05 (1 H, br), 4.08 (2 H, t, *J* = 4.9 Hz), 3.57 (2 H, s), 1.46 (9 H, s); HRMS (ESI-TOF) *m*/*z:* [M + H - C₄H₈]⁺ calculated for C₁₆H₁₅⁷⁹BrN₃O₄ and C₁₆H₁₅⁸¹BrN₃O₄ 392.0241, 394.0220; found 392.0547, 394.0528

### tert-Butyl (2-(2-(3-(6-bromo-2-oxo-2,3-dihydro-1H-benzo[d]imidazol-1-yl)phenoxy)ethoxy)ethyl)carbamate (59m)

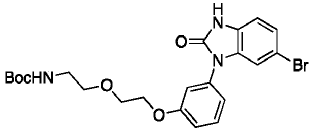

The above compound (**59m**) was prepared from **58l** following Method S. The reaction was stirred at 0°C to room temperature for 16 h and purified by column chromatography on silica gel (EtOAc/hexane, 40:60 to 60:40) to give **59l** (52%) as a yellow solid; **¹H NMR** (CDCl₃, 600 MHz) 10.58 (1 H, s), 7.4 (1 H, t, *J* = 7.3 Hz), 7.21 (1 H, d, *J* = 8.5 Hz), 7.16 (1 H, s), 7.10-7.08 (2 H, m), 7.03-6.99 (2 H, m), 5.01 (1 H, br), 4.17 (2 H, t, *J* = 4.9 Hz), 3.84 (2 H, t, *J* = 4.9 Hz), 3.62 (2 H, t, *J* = 4.9 Hz), 3.35 (2 H, br), 1.43 (9 H, s); HRMS (ESI-TOF) *m*/*z*: [M + H]⁺ calculated for C₂₂H₂₇⁷⁹BrN₃O₅ and C₂₂H₂₇⁸¹BrN₃O₅ 492.1129, 494.1109; found 492.1094, 494.1066

### 1-Ethyl-6-(4,4,5,5-tetramethyl-1,3,2-dioxaborolan-2-yl)-1H-benzo[d]imidazol-2(3H)-one (60a)

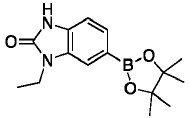

The above compound (**60a**) was prepared from **59a** following Method C and purified by column chromatography on silica gel (MeOH/CH₂Cl₂, 2.5:97.5 to 5:95) to give **60a** (86%) as a light pink solid; **¹H NMR** (CDCl₃, 500 MHz) 10.36 (1 H, s), 7.58 (1 H, d, *J* = 7.6 Hz), 7.44 (1 H, s), 7.14 (1 H, d, *J* = 7.6 Hz), 3.98 (2 H, q, *J* = 7.6 Hz), 1.46-1.29 (15 H, m); HRMS (ESI-TOF) *m*/*z*: [M + H]⁺ calculated for C₁₅H₂₂BN₂O₃ 289.1718; found 289.1728.

### 1-Isopropyl-6-(4,4,5,5-tetramethyl-1,3,2-dioxaborolan-2-yl)-1H-benzo[d]imidazol-2(3H)-one (60b)

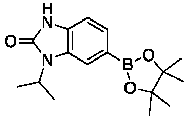

The above compound (**60b**) was prepared from **59b** following Method C and purified by column chromatography on silica gel (EtOAc/hexane, 30:70 to 40:60) to give **60b** (99%) as a light brown solid; **¹H NMR** (CDCl₃, 500 MHz) 10.40 (1 H, s), 7.56-7.54 (2 H, m), 7.12 (1 H, d, *J* = 7.6 Hz), 4.77-4.69 (1 H, m), 1.58 (6 H, d, *J* = 7.1 Hz), 1.35 (12 H, s); HRMS (ESI-TOF) *m*/*z*: [M + H]⁺ calculated for C₁₆H₂₄BN₂O₃ 303.1875; found 303.1886.

### 1-Cyclopropyl-6-(4,4,5,5-tetramethyl-1,3,2-dioxaborolan-2-yl)-1H-benzo[d]imidazol-2(3H)-one (60c)

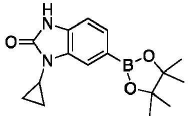

The above compound (**60c**) was prepared from **59c** following Method C and purified by column chromatography on silica gel (EtOAc/hexane, 50:50 to 60:40) to give **60c** (88%) as a yellow solid; **¹H NMR** (CDCl₃, 500 MHz) 8.86 (1 H, s), 7.61 (1 H, s), 7.57 (1 H, d, *J* = 8.1 Hz), 7.06 (1 H, d, *J* = 7.6 Hz), 2.90-2.86 (1 H, m), 1.36 (12 H, s), 1.17-1.13 (2 H, m), 1.08-1.05 (2 H, m); HRMS (ESI-TOF) *m*/*z*: [M + H]⁺ calculated for C₁₆H₂₂BN₂O₃ 301.1718; found 301.1777.

### 1-Cyclopentyl-6-(4,4,5,5-tetramethyl-1,3,2-dioxaborolan-2-yl)-1H-benzo[d]imidazol-2(3H)-one (60d)

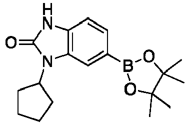

The above compound (**60d**) was prepared from **59d** following Method C and purified by column chromatography on silica gel (EtOAc/hexane, 50:50 to 60:40) to give **60d** (99%) as a white solid; **¹H NMR** (CDCl₃, 500 MHz) 9.52 (1 H, s), 7.55 (1 H, d, *J* = 7.6 Hz), 7.47 (1 H, s), 7.09 (1 H, d, *J* = 8.1 Hz), 4.80 (1 H, quin, *J* = 8.6 Hz), 2.24-2.20 (2 H, m), 2.04-1.99 (4 H, m), 1.74-1.72 (2 H, m), 1.36 (12 H, s); HRMS (ESI-TOF) *m*/*z*: [M + H]⁺ calculated for C₁₈H₂₆BN₂O₃ 329.2031; found 329.2052.

### 1-Phenyl-6-(4,4,5,5-tetramethyl-1,3,2-dioxaborolan-2-yl)-1H-benzo[d]imidazol-2(3H)-one (60e)

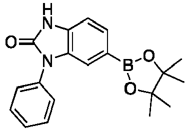

The above compound (**60e**) was prepared from **59e** following Method C and purified by column chromatography on silica gel (EtOAc/hexane, 30:70 to 40:60) to give **60e** (84%) as a light brown solid; **¹H NMR** (CDCl₃, 500 MHz) 9.38 (1 H, s), 7.61 (1 H, d, *J* = 8.6 Hz), 7.57-7.53 (5 H, m), 7.44 (1 H, s), 7.14 (1 H, d, *J* = 7.6 Hz), 1.32 (12 H, s); HRMS (ESI-TOF) *m*/*z:* [M + H]⁺ calculated for C₁₉H₂₂BN₂O₃ 337.1718; found 337.1723.

### 1-(Pyridin-2-yl)-6-(4,4,5,5-tetramethyl-1,3,2-dioxaborolan-2-yl)-1H-benzo[d]imidazol-2(3H)-one (60f)

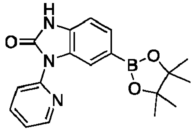

The above compound (**60f**) was prepared from **59f** following Method C and purified by column chromatography on silica gel (EtOAc/hexane, 30:70 to 35:75) to give **60f** (57%) as a white solid; **¹H NMR** (CDCl₃, 500 MHz) 9.06 (1 H, s), 8.66 (1 H, d, *J* = 5.1 Hz), 8.24 (1 H, s), 7.99 (1 H, d, *J* = 8.1 Hz), 7.88 (1 H, td, *J* = 7.4, 2.0 Hz), 7.64 (1 H, d, *J* = 8.1 Hz), 7.29-7.27 (1 H, m), 7.12 (1 H, d, *J* = 7.6 Hz), 1.34 (12 H, s); HRMS (ESI-TOF) *m*/*z*: [M + H]⁺ calculated for C₁₈H₂₁BN₃O₃ 338.1671; found 338.1678.

### 1-(Pyridin-4-yl)-6-(4,4,5,5-tetramethyl-1,3,2-dioxaborola-2-yl)-1H-benzo[d]imidazol-2(3H)-one (60h)

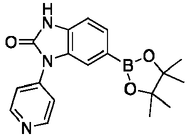

The above compound (**60h**) was prepared from **59h** following Method C and purified by column chromatography on silica gel (EtOAc/hexane, 30:70 to 35:75) to give **60h** (90%) as a yellow solid; **¹H NMR** (CDCl₃, 500 MHz) 8.81 (2 H, d, *J* = 6.1 Hz), 8.46 (1 H, s), 7.67-7.64 (3 H, m), 7.14 (1 H, d, *J* = 7.6 Hz), 1.34 (12 H, s); HRMS (ESI-TOF) *m*/*z*: [M + H]⁺ calculated for C₁₈H₂₁BN₃O₃ 338.1671; found 338.1898.

### 1-(2-(2-Methoxyethoxy)phenyl)-6-(4,4,5,5-tetramethyl-1,3,2-dioxaborolan-2-yl)-1H-benzo[d]imidazol-2(3H)-one (60i)

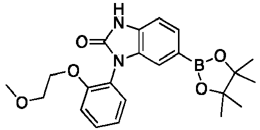

The above compound (**60i**) was prepared from **59i** following Method C and purified by column chromatography on silica gel (EtOAc/hexane, 35:75 to 70:30) to give **60i** (72%) as a pale yellow solid; **¹H NMR** (CDCl₃, 500 MHz) 8.06 (1 H, s), 7.58 (1 H, d, *J* = 7.1 Hz), 7.43-7.41 (2 H, m), 7.15-7.12 (3 H, m), 7.07 (1 H, d, *J* = 7.6 Hz), 4.14 (2 H, t, *J* = 4.6 Hz), 3.55 (2 H, t, *J* = 4.6 Hz), 3.16 (3 H, s), 1.31 (12 H, s); HRMS (ESI-TOF) *m*/*z*: [M + H]⁺ calculated for C₂₂H₂₈BN₂O₅ 411.2086; found 411.2257.

### 1-(3-(2-Methoxyethoxy)phenyl)-6-(4,4,5,5-tetramethyl-1,3,2-dioxaborolan-2-yl)-1H-benzo[d]imidazol-2(3H)-one (60j)

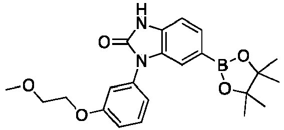

The above compound (**60j**) was prepared from **59j** following Method C and purified by column chromatography on silica gel (EtOAc/hexane, 30:70 to 35:65) to give **60j** (70%) as a yellow solid; **¹H NMR** (CDCl₃, 500 MHz) 10.17 (1 H, s), 7.60 (1 H, d, *J* = 7.6 Hz), 7.48-7.44 (2 H, m), 7.22-7.00 (4 H, m), 4.19 (2 H, d, *J* = 4.1 Hz), 3.78 (2 H, t, *J* = 4.6 Hz), 3.47 (3 H, s), 1.32 (12 H, s); HRMS (ESI-TOF) *m*/*z*: [M + H]⁺ calculated for C₂₂H₂₈BN₂O₅ 411.2086; found 411.2093.

### 1-(4-(2 -Methoxyethoxy)phenyl)-6-(4,4,5,5-tetramethyl-1,3,2-dioxaborolan-2-yl)-1H-benzol[d]imidazol-2(3H)-one (60k)

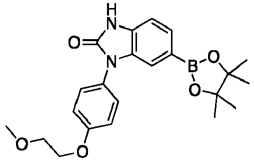

The above compound (**60k**) was prepared from **59k** following Method C and purified by column chromatography on silica gel (EtOAc/hexane, 50:50 to 70:30) to give **60k** (60%) as a pale yellow solid; **¹H NMR** (CDCl₃, 500 MHz) 8.39 (1 H, s), 7.60 (1 H, d, *J* = 8.1 Hz), 7.43-7.41 (2 H, m), 7.36 (1 H, s), 7.11-7.08 (3 H, m), 4.20 (2 H, d, *J* = 4.6 Hz), 3.80 (2 H, t, *J* = 5.1 Hz), 3.48 (3 H, s), 1.32 (12 H, s); HRMS (ESI-TOF) *m*/*z*: [M + H]⁺ calculated for C₂₂H₂₈BN₂O₅ 411.2086; found 411.2073.

### tert-Butyl (2-(3-(2-oxo-6-(4,4,5,5-tetramethyl-1,3,2-dioxaborolan-2-yl)-2,3-dihydro-1H-benzo[d]imidazol-1-yl)phenoxy)ethyl)carbamate (60l)

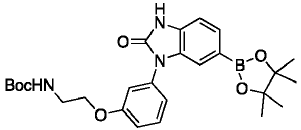

The above compound (**60l**) was prepared from **59l** following Method C and purified by column chromatography on silica gel (EtOAc/hexane, 70:30) to give **60l** (92%) as a yellow oil; **¹H NMR** (CDCl₃, 600 MHz) 7.61 (1 H, d, *J* = 8.5 Hz), 7.46 (1 H, t, *J* = 7.3 Hz), 7.43 (1 H, s), 7.13 (1 H, d, *J* = 8.1 Hz), 7.07 (1 H, s), 6.97 (1 H, dd, *J* = 8.7, 2.6 Hz), 4.07 (2 H, t, *J* = 4.6 Hz), 3.57 (2 H, br), 1.45 (9 H, s), 1.32 (12 H, s); HRMS (ESI-TOF) *m*/*z*: [M + H - C₄H₈]⁺ calculated for C₂₂H₂₇BN₃O₆ 440.1988; found 440.1996.

### tert-Butyl (2-(2-(3-(2-oxo-6-(4,4,5,5-tetramethyl-1,3,2-dioxaborolan-2-yl)-2,3-dihydro-1H-benzo[d]imidazol-1-yl)phenoxy)ethoxy)ethyl)carbamate (60m)

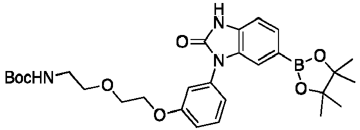

The above compound (**60m**) was prepared from **59m** following Method C and purified by column chromatography on silica gel (EtOAc/hexane, 70:30) to give **60m** (84%) as a yellow oil; **¹H NMR** (CDCl₃, 600 MHz) 10.17 (1 H, br), 7.60 (1 H, d, *J* = 7.3 Hz), 7.46 (1 H, t, *J* = 7.3 Hz), 7.43 (1 H, s), 7.15-7.13 (2 H, m), 7.11 (1 H, s), 7.02 (1 H, dd, *J* = 9.8, 2.4 Hz), 5.03 (1 H, br), 4.18 (2 H, t, *J* = 4.9 Hz), 3.84 (2 H, t, *J* = 4.9 Hz), 3.62 (2 H, t, *J* = 4.9 Hz), 3.36 (2 H, br), 1.42 (9 H, s), 1.32 (12 H, s); HRMS (ESI-TOF) *m*/*z:* [M + H]⁺ calculated for C₂₈H₃₉BN₃O₇ 540.2876; found 540.2821.

### 6-(5-(Benzylamino)pyridin-3-yl)-1-ethyl-1H-benzo[d]imidazol-2(3H)-one (61a)

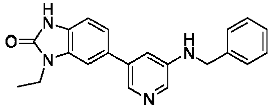

The above compound (**61a**) was prepared from **60a** and **5a** following Method E and purified by column chromatography on silica gel (MeOH/CH₂Cl₂, 5:95) to give **61a** (77%) as a light brown solid; **¹H NMR** (DMSO-*d₆,* 500 MHz) 10.87 (1 H, m), 8.04 (1 H, d, *J =* 1.5 Hz), 7.92 (1 H, d, *J =* 2.5 Hz), 7.40 (2 H, d, *J* = 7.6 Hz), 7.33 (2 H, t, *J* = 7.1 Hz), 7.28 (1 H, s), 7.23 (1 H, t, *J* = 7.6 Hz), 7.18 (1 H, dd, *J* = 7.4, 1.5 Hz), 7.08 (1 H, t, *J* = 2.0 Hz), 7.02 (1 H, d, *J* = 7.6 Hz), 6.56 (1 H, t, *J* = 6.1 Hz), 4.38 (2 H, d, *J* = 6.1 Hz), 3.37 (2 H, q, *J* = 7.1 Hz), 1.08 (3 H, t, *J* = 7.1 Hz); HRMS (ESI-TOF) *m*/*z:* [M + H]⁺ calculated for C₂₁H₂₁N₄O 345.1710; found 345.1702.

### 6-(5-(Benzylamino)pyridin-3-yl)-1-isopropyl-1H-benzo[d]imidazol-2(3H)-one (61b)

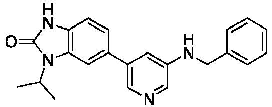

The above compound (**61b**) was prepared from **60b** and **5a** following Method E and purified by column chromatography on silica gel (MeOH/CH₂Cl₂, 2.5:97.5 to 5:95) to give **61b** (71%) as a pale yellow solid; **¹H NMR** (DMSO-*d₆,* 500 MHz) 10.86 (1 H, m), 8.02 (1 H, s), 7.93 (1 H, d, *J* = 2.5 Hz), 7.40 (2 H, d, *J* = 7.6 Hz), 7.33 (2 H, t, *J* = 7.1 Hz), 7.27 (1 H, s), 7.23 (1 H, t, *J* = 7.1 Hz), 7.17 (1 H, d, *J* = 8.1 Hz), 7.05 (1 H, s), 7.01 (1 H, d, *J* = 7.6 Hz), 6.57 (1 H, t, *J* = 6.1 Hz), 4.64-4.56 (1 H, m), 4.38 (2 H, d, *J* = 6.1 Hz), 1.44 (6 H, d, *J* = 6.6 Hz); HRMS (ESI-TOF) *m*/*z*: [M + H]⁺ calculated for C₂₂H₂₃N₄O 359.1867; found 359.1925.

### 6-(5-(Benzylamino)pyridin-3-yl)-1-cyclopropyl-1H-benzo[d]imidazol-2(3H)-one (61c)

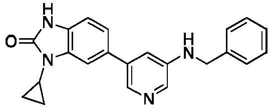

The above compound (**61c**) was prepared from **60c** and **5a** following Method E and purified by column chromatography on silica gel (MeOH/CH₂Cl₂, 2.5:97.5 to 5:95) to give **61c** (72%) as a yellow solid; **¹H NMR** (DMSO-*d₆,* 500 MHz) 10.78 (1 H, s), 8.00 (1 H, s), 7.93 (1 H, d, *J* = 2.0 Hz), 7.40 (2 H, d, *J* = 7.6 Hz), 7.33 (2 H, t, *J* = 7.6 Hz), 7.25-7.22 (2 H, m), 7.18 (1 H, d, *J* = 8.1 Hz), 7.06 (1 H, s), 6.99 (1 H, d, *J* = 7.6 Hz), 6.60 (1 H, t, *J* = 5.6 Hz), 4.37 (2 H, d, *J =* 6.1 Hz), 2.86-2.84 (1 H, m), 1.03-0.99 (2 H, m), 0.89-0.87 (2 H, m); HRMS (ESI-TOF) *m*/*z:* [M + H]⁺ calculated for C₂₂H₂₁N₄O 357.1710; found 357.1704.

### 6-(5-(Benzylamino)pyridin-3-yl)-1-cyclopentyl-1H-benzo[d]imidazol-2(3H)-one (61d)

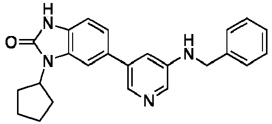

The above compound (**61d**) was prepared from **60d** and **5a** following Method E and purified by column chromatography on silica gel (EtOAc/hexane, 40:60 to 80:20) to give **61d** (47%) as a yellow solid; **¹H NMR** (DMSO-*d₆,* 500 MHz) 10.89 (1 H, s), 8.01 (1 H, s), 7.93 (1 H, s), 7.39 (2 H, d, *J* = 7.1 Hz), 7.33 (2 H, t, *J* = 7.6 Hz), 7.24-7.21 (2 H, m), 7.17 (1 H, d, *J* = 8.1 Hz), 7.04-7.01 (1 H, m), 6.57 (1 H, t, *J* = 5.6 Hz), 4.72 (1 H, quin, *J* = 9.2 Hz), 4.37 (2 H, d, *J* = 5.6 Hz), 2.06-1.88 (6 H, m), 1.66 (2 H, br); HRMS (ESI-TOF) *m*/*z*: [M + H]⁺ calculated for C₂₄H₂₅N₄O 385.2023; found 385.2133.

### 6-(5-(Benzylamino)pyridin-3-yl)-1-phenyl-1H-benzo[d]imidazol-2(3H)-one (61e)

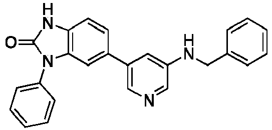

The above compound (**61e**) was prepared from **60e** and **5a** following Method E and purified by column chromatography on silica gel (MeOH/CH₂Cl₂, 2.5:97.5 to 5:95) to give **61e** (70%) as an off-white solid; **¹H NMR** (DMSO-*d₆,* 500 MHz) 11.23 (1 H, s), 7.94 (1 H, d, *J* = 1.5 Hz), 7.89 (1 H, d, *J* = 2.0 Hz), 7.60-7.55 (4 H, m), 7.47-7.45 (1 H, m), 7.35 (2 H, d, *J* = 7.1 Hz), 7.28-7.24 (3 H, m), 7.19 (1 H, t, *J* = 7.1 Hz), 7.13 (1 H, d, *J* = 8.1 Hz), 7.02 (1 H, s), 6.99 (1 H, s), 6.56 (1 H, t, *J* = 6.1 Hz), 4.32 (2 H, d, *J* = 5.6 Hz); HRMS (ESI-TOF) *m*/*z*: [M + H]⁺ calculated for C₂₅H₂₁N₄O 393.1710; found 393.1715.

### 6-(5-(Benzylamino)pyridin-3-yl)-1-(pyridin-2-yl)-1H-benzo[d]imidazol-2(3H)-one (61f)

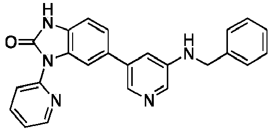

The above compound (**61f**) was prepared from **60f** and **5a** following Method E and purified by column chromatography on silica gel (MeOH/CH₂Cl₂, 2.5:97.5) to give **61f** (87%) as a white solid; **¹H NMR** (DMSO-*d₆,* 500 MHz) 11.37 (1 H, s), 8.59-8.58 (1 H, m), 8.05-8.04 (2 H, m), 8.02-7.96 (2 H, m), 7.93 (1 H, d, *J* = 2.5 Hz), 7.39-7.37 (3 H, m), 7.33-7.29 (3 H, m), 7.22 (1 H, t, *J* = 7.1 Hz), 7.14 (1 H, d, *J* = 8.1 Hz), 7.03 (1 H, t, *J* = 2.0 Hz), 6.60 (1 H, t, *J* = 6.1 Hz), 4.36 (2 H, d, *J* = 6.1 Hz); HRMS (ESI-TOF) *m*/*z:* [M + H]⁺ calculated for C₂₄H₂₀N₅O 394.1663; found 394.1661.

### 6-(5-(Benzylamino)pyridin-3-yl)-1-(pyridin-3-yl)-1H-benzo[d]imidazol-2(3H)-one (61g)

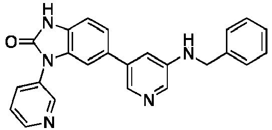

The above compound (**61g**) was prepared from **59g** following Method C and purified by column chromatography on silica gel (EtOAc/hexane, 30:70 to 35:75) to give a mixture of intermediate **60g** as a brown solid. The mixture was used in next step, Method E, and purified by column chromatography on silica gel (MeOH/CH₂Cl₂, 2.5:97.5 to 5:95) to give **61g** (34%) as a gray solid; **¹H NMR** (DMSO-*d₆,* 500 MHz) 11.34 (1 H, s), 8.83 (1 H, d, *J* = 2.5 Hz), 8.65 (1 H, d, *J* = 5.1 Hz), 8.05 (1 H, d, *J* = 8.1 Hz), 7.97 (1 H, s), 7.90 (1 H, d, *J* = 2.0 Hz), 7.62 (1 H, dd, *J* = 8.1, 4.6 Hz), 7.36 (2 H, d, *J* = 7.6 Hz), 7.31-7.25 (3 H, m), 7.20-7.15 (2 H, m), 7.08 (1 H, s), 7.02 (1 H, s), 6.55 (1 H, t, *J* = 5.6 Hz), 4.33 (2 H, d, *J* = 5.6 Hz); HRMS (ESI-TOF) *m*/*z*: [M + H]⁺ calculated for C₂₄H₂₀N₅O 394.1663; found 394.1693.

### 6-(5-(Benzylamino)pyridin-3-yl)-1-(3-(2-methoxyethoxy)phenyl)-1H-benzo[d]imidazol-2(3H)-one (61j)

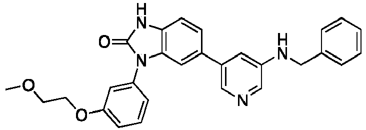

The above compound (**61j**) was prepared from **60j** and **5a** following Method E and purified by column chromatography on silica gel (MeOH/CH₂Cl₂, 2.5:97.5 to 5:95) to give **61j** (65%) as a pale yellow solid; **¹H NMR** (DMSO-*d₆,* 500 MHz) 11.22 (1 H, s), 7.94 (1 H, d, *J* = 1.5 Hz), 7.90 (1 H, d, *J* = 2.5 Hz), 7.47 (1 H, t, *J* = 8.1 Hz), 7.35 (2 H, d, *J* = 7.1 Hz), 7.28-7.25 (3 H, m), 7.19 (1 H, t, *J* = 7.1 Hz), 7.13-7.12 (3 H, m), 7.05-7.033 (2 H, m), 6.99 (1 H, t, *J* = 2.0 Hz), 6.56 (1 H, t, *J* = 6.1 Hz), 4.32 (2 H, d, *J* = 5.6 Hz), 4.15-4.13 (2 H, m), 3.67-3.65 (2 H, m), 3.27 (3 H, s); HRMS (ESI-TOF) *m*/*z*: [M + H]⁺ calculated for C₂₈H₂₇N₄O₃ 467.2078; found 467.2090.

### 6-(5-(Benzylamino)pyridin-3-yl)-1-(4-(2-methoxyethoxy)phenyl)-1H-benzo[d]imidazol-2(3H)-one (61k)

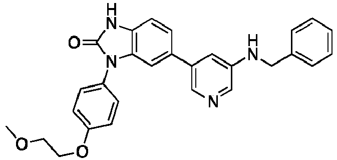

The above compound (**61k**) was prepared from **60k** and **5a** following Method E and purified by column chromatography on silica gel (MeOH/CH₂Cl₂, 2.5:97.5 to 5:95) to give **61j** (89%) as a pale yellow solid; **¹H NMR** (DMSO-*d₆,* 500 MHz) 11.15 (1 H, s), 7.93 (1 H, s), 7.89 (1 H, d, *J* = 2.0 Hz), 7.44 (2 H, d, *J* = 8.6 Hz), 7.35 (2 H, d, *J* = 7.6 Hz), 7.28-7.24 (3 H, m), 7.19 (1 H, t, *J* = 7.6 Hz), 7.12-7.10 (3 H, m), 6.98 (1 H, t, *J* = 2.0 Hz), 6.92 (1 H, s), 6.55 (1 H, t, *J* = 5.6 Hz), 4.32 (2 H, d, *J* = 5.6 Hz), 4.17 (2 H, t, *J* = 4.6 Hz), 3.70-3.69 (2 H, m), 3.32 (3 H, s); HRMS (ESI-TOF) *m*/*z*: [M + H]⁺ calculated for C₂₈H₂₇N₄O₃ 467.2078; found 467.2091.

### tert-Butyl (2-(3-(6-(5-(benzylamino)pyridin-3-yl)-2-oxo-2,3-dihydro-1H-benzo[d]imidazol-1-yl)phenoxy)ethyl)carbamate (61l)

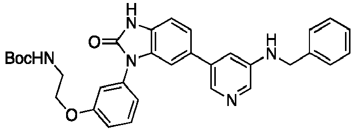

The above compound (**61l**) was prepared from **60l** and **5a** following Method E and purified by column chromatography on silica gel (MeOH/CH₂Cl₂, 5:95) to give **61l** (62%) as a yellow solid; **¹H NMR** (DMSO-*d₆,* 600 MHz) 11.24 (1 H, s), 7.93 (1 H, s), 7.90 (1 H, s), 7.47 (1 H, t, *J* = 7.3 Hz), 7.35 (2 H, d, *J* = 7.3 Hz), 7.27-7.25 (3 H, m), 7.19 (1 H, t, *J* = 7.3 Hz), 7.13-7.10 (3 H, m), 7.05-7.02 (2 H, m), 6.98 (1 H, s), 6.58 (1 H, s), 6.45 (1 H, s), 4.32 (2 H, d, *J* = 6.1 Hz), 4.13 (2 H, t, *J* = 4.9 Hz), 3.73-3.73 (2 H, m), 3.45-3.43 (2 H, m), 3.09-3.06 (2 H, m), 1.33 (9 H, s); HRMS (ESI-TOF) *m*/*z*: [M + H]⁺ calculated for C₃₂H₃₄N₅O₄ 552.2606; found 552.2556.

### tert-Butyl (2-(2-(3-(6-(5-(benzylamino)pyridin-3-yl)-2-oxo-2,3-dihydro-1H-benzo[d]imidazol-1-yl)phenoxy)ethoxy)ethyl)carbamate (61m)

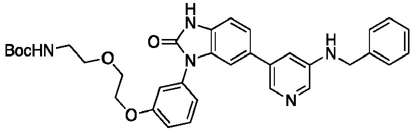

The above compound (**61m**) was prepared from **60m** and **5a** following Method E and purified by column chromatography on silica gel (MeOH/CH₂Cl₂, 5:95) to give **61m** (53%) as a brown solid; **¹H NMR** (DMSO-*d₆,* 600 MHz) 11.23 (1 H, s), 7.93 (1 H, s), 7.89 (1 H, d, *J* = 2.4 Hz), 7.47 (1 H, t, *J* = 8.5 Hz), 7.34 (2 H, d, *J* = 8.5 Hz), 7.27-7.25 (3 H, m), 7.19 (1 H, t, *J* = 7.3 Hz), 7.13-7.11 (3 H, m), 7.03-7.02 (2 H, m), 6.98 (1 H, s), 6.58 (1 H, t, *J* = 4.9 Hz), 6.45 (1 H, s), 4.32 (2 H, d, *J* = 6.1 Hz), 4.00 (2 H, t, *J* = 4.9 Hz), 3.31-3.29 (2 H, m), 1.34 (9 H, s); HRMS (ESI-TOF) *m*/*z*: [M + H]⁺ calculated for C₃₄H₃₈N₅O₅ 596.2868; found 596.2782.

### Example 21 - General Procedure for the Preparation of 1-(3-(2-Aminoalkoxy)phenyl)-6-(5-(benzylamino)pyridin-3-yl)-1H-benzo[d]imidazol-2(3H)-one hydrochloride-Method T

### 1-(3-(2-Aminoethoxy)phenyl)-6-(5-(benzylamino)pyridin-3-yl)-1H-benzo[d]imidazol-2(3H)-one hydrochloride (61n)

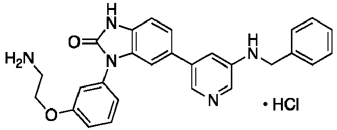

To a solution of **61l** (14.5 mg, 0.03 mmol) in 1,4-dioxane (1.5 mL) was added 4 N HCl in 1,4-dioxane (0.5 mL). The reaction was stirred at room temperature for 16 h the solvent was removed under reduced pressure. Toluene (2 mL) was added and removed *in vacuo.* The crude product was washed with Et₂O to give **61n** (12.6 mg, 91%) as a pale yellow solid; **¹H NMR** (DMSO-*d₆,* 600 MHz) 11.47 (1 H, s), 8.27 (1 H, s), 8.18 (2 H, br), 7.97 (1 H, s), 7.76 (1 H, s), 7.52 (1 H, t, *J* = 7.3 Hz), 7.43 (1 H, d, *J* = 8.5 Hz), 7.39-7.38 (2 H, m), 7.33-7.30 (2 H, m), 7.26-7.21 (4 H, m), 7.12-7.03 (2 H, m), 6.47 (1 H, t, *J* = 8.5 Hz), 4.47 (2 H, s), 4.24 (2 H, t, *J* = 4.9 Hz), 3.23-3.22 (2 H, m); HRMS (ESI-TOF) *m*/*z*: [M + H]⁺ calculated for C₂₇H₂₆N₅O₂ 452.2082; found 452.2028.

### 1-(3-(2-(2-Aminoethoxy)ethoxy)phenyl)-6-(5-(benzylamino)pyridin-3-yl)-1H-benzo[d]imidazol-2(3H)-one hydrochloride (61o)

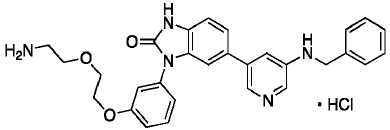

The above compound (**61o**) was prepared from **60m** following Method T to give **61o** (99%) as a light brown solid; **¹H NMR** (DMSO-*d₆,* 600 MHz) 11.44 (1 H, s), 8.24 (1 H, s), 7.97 (1 H, s), 7.92-7.87 (3 H, m), 7.49 (1 H, t, *J* = 7.3 Hz), 7.42-7.37 (3 H, m), 7.32-7.30 (2 H, m), 7.26-7.20 (3 H, m), 7.16-7.14 (2 H, m), 7.07-7.06 (2 H, m), 4.46 (2 H, br), 4.19 (2 H, br), 3.81-3.80 (2 H, m), 3.67-3.66 (2 H, m), 3.00-2.97 (2 H, m); HRMS (ESI-TOF) *m*/*z*: [M + H]⁺ calculated for C₂₉H₃₀N₅O₃ 496.2344; found 496.2344.

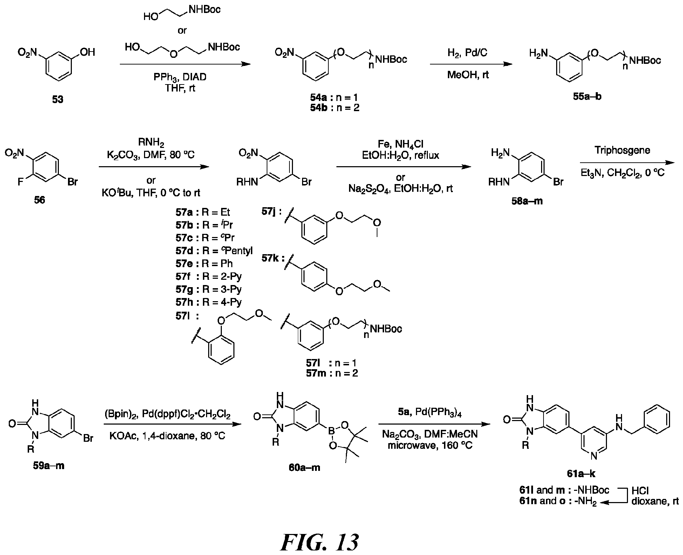

The synthetic schemes for compounds **61a-o,** discussed *supra,* are shown in Figure 13.

### (R,E)-N-((5-Bromopyridin-3-yl)methylene)-2-methylpropane-2-sulfinamide (62a)

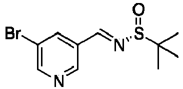

To a solution of **34** (100 mg, 0.54 mmol) in anhydrous THF (2.0 mL) was added titaniumethoxide (0.56 mL, 2.69 mmol). The reaction mixture was purged with nitrogen then (*R*)-(+)-2-methyl-2-propanesulfinamide (78.2 mg, 0.64 mmol). The reaction was stirred at room temperature for 5 h. Brine (2 mL) was added to the mixture and stirred for 10 min. The aqueous layer was extracted with EtOAc (2 × 20 mL). The combined organic extracts were washed with brine, dried over anhydrous Na₂SO₄, filtered, and concentrated. The crude product (145.0 mg, 93%)was used in next step without further purification; **¹H NMR** (CDCl₃, 500 MHz) 8.90 (1 H, s), 8.78 (1 H, s), 8.60 (1 H, s), 8.31 (1 H, s), 1.27 (9 H, s); HRMS (ESI-TOF) *m*/*z*: [M + H]⁺ calculated for C₁₀H₁₄⁷⁹BrN₂OS and C₁₀H₁₄⁸¹BrN₂OS 289.0005, 290.9985; found 289.0012, 290.9996

### (S,E)-N-((5-Bromopyridin-3-yl)methylene)-2-methylpropane-2-sulfinamide (62b)

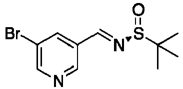

To a solution of **34** (200 mg, 1.08 mmol) in anhydrous 1,2-dichloroethane (4.0 mL) was added (*S*)-(-)-2-methyl-2-propanesulfinamide (143 mg, 1.18 mmol) and copper (II) sulfate (343 mg, 2.15 mmol). The reaction mixture was purged with nitrogen and stirred at 50°C for 42 h. The reaction was filtered through a Celite pad. The solvent was removed *in vacuo* and purified by column chromatography on silica gel (EtOAc/hexane₂, 30:70) to give **62b** (84%) as a pale yellow solid; **¹H NMR** (CDCl₃, 500 MHz) 8.91 (1 H, d, *J* = 1.5 Hz), 8.80 (1 H, d, *J* = 1.8 Hz), 8.60 (1 H, s), 8.32 (1 H, t, *J* = 2.0 Hz), 1.28 (9 H, s); HRMS (ESI-TOF) *m*/*z*: [M + H]⁺ calculated for C₁₀H₁₄⁷⁹BrN₂OS and C₁₀H₁₄⁸¹BrN₂OS 289.0005, 290.9985; found 289.0032, 291.0015.

### Example 22 - General Procedure for the Preparation of N-(1-(5-Bromopyridin-3-yl)-2-phenylethyl)-2-methylpropane-2-sulfinamide-Method U

### (R)-N-((R)-1-(5-Bromopyridin-3-yl)-2-phenylethyl)-2-methylpropane-2-sulfinamide (63a)

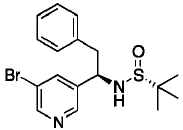

To a solution of **62a** (145 mg, 0.50 mmol) in anhydrous THF (2.0 mL) was slowly added 2 M benzylmagnesium chloride (0.4 mL, 0.75 mmol) at -40°C under nitrogen. The resulting mixture was stirred at -40°C for 5 h. After being quenched with saturated ammonium chloride (3 mL), the aqueous layer was extracted with EtOAc (2 × 10 mL). The combined organic extracts were washed with brine, dried over anhydrous Na₂SO₄, filtered, and concentrated. The residue was and purified by column chromatography on silica gel (EtOAc/hexane, 50:50 to 80:20) to give **63a** (63%) as a yellow solid; **¹H NMR** (CDCl₃, 500 MHz) 8.58 (1 H, s), 8.36 (1 H, s), 7.71 (1 H, s), 7.25-7.21 (3 H, m), 7.01 (2 H, d, *J* = 7.1 Hz), 4.64-4.60 (1 H, m), 3.55 (2 H, d, *J* = 4.6 Hz), 3.34-3.30 (1 H, m), 3.03-2.99 (1 H, m), 1.16 (9 H, s); HRMS (ESI-TOF) *m*/*z*: [M + H]⁺ calculated for C₁₇H₂₂⁷⁹BrN₂OS and C₁₇H₂₂⁸¹BrN₂OS 381.0631, 383.0611; found 381.1, 383.1.

### (S)-N-((S)-1-(5-Bromopyridin-3-yl)-2-phenylethyl)-2-methylpropane-2-sulfinamide (63b)

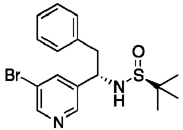

The above compound (**63b**) was prepared from **62b** following Method U and purified by column chromatography on silica gel (EtOAc/hexane, 50:50 to 80:20) to give **63b** (77%) as a yellow solid; **¹H NMR** (CDCl₃, 500 MHz) 8.58 (1 H, s), 8.36 (1 H, s), 7.71 (1 H, s), 7.62-7.21 (3 H, m), 7.01 (2 H, d, *J* = 6.6 Hz), 4.62 (1 H, q, *J* = 5.1 Hz), 3.58 (1 H, d, *J* = 4.6 Hz), 3.31 (1 H, dd, *J* = 13.5, 7.1 Hz), 3.01 (1 H, dd, *J* = 13.2, 7.6 Hz), 1.16 (9 H, s); HRMS (ESI-TOF) *m*/*z*: [M + H]⁺ calculated for C₁₇H₂₂⁷⁹BrN₂OS and C₁₇H₂₂⁸¹BrN₂OS 381.0631, 383.0611; found 381.1, 383.12.

### Example 23 - General Procedure for the Preparation of 1-(5-Bromopyridin-3-yl)-2-phenylethanamine-Method V

### (S)-1-(5-Bromopyridin-3-yl)-2-phenylethanamine (64b)

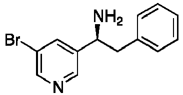

To a solution of **63b** (64.2 mg, 0.17 mmol) in anhydrous MeOH (3.0 mL) was added 4 N HCl in 1,4-dioxane (0.2 mL, 0.67 mmol). The resulting mixture was stirred at room temperature for 4 h. After being quenched with saturated NaHCO₃ (2 mL), the aqueous layer was extracted with EtOAc (2 × 10 mL). The combined organic extracts were washed with brine, dried over anhydrous Na₂SO₄, filtered, and concentrated. The residue was and purified by column chromatography on silica gel (MeOH/CH₂Cl₂, 5:95) to give **64b** (39.6 mg, 85%) as a clear oil; **¹H NMR** (CDCl₃, 500 MHz) 8.5 (1 H, d, *J* = 2.0 Hz), 8.45 (1 H, s), 7.90 (1 H, s), 7.31 (2 H, t, *J* = 7.1 Hz), 7.25 (1 H, t, *J* = 7.1 Hz), 7.15 (2 H, d, *J* = 7.6 Hz), 4.24 (1 H, dd, *J* = 8.6, 5.1 Hz), 2.98 (1 H, dd, *J* = 13.2, 5.1 Hz), 2.82 (1 H, dd, *J* = 9.2, 13.5 Hz), 1.48 (2 H, br); HRMS (ESI-TOF) *m*/*z*: [M + H]⁺ calculated for C₁₃H₁₄⁷⁹BrN₂ and C₁₃H₁₄⁸¹BrN₂ 277.0335, 279.1671; found 277.0379, 279.0358.

### (R)-1-(5-bromopyridin-3-yl)-2-phenylethanamine (64a)

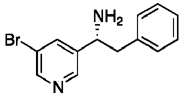

The above compound (**64a**) was prepared from **63a** following Method V and purified by column chromatography on silica gel (MeOH/CH₂Cl₂, 5:95) to give **64a** (98%) as a yellow oil; **¹H NMR** (CDCl₃, 500 MHz) 8.55 (1 H, d, *J* = 2.0 Hz), 8.44 (1 H, d, *J* = 2.0 Hz), 7.89 (1 H, s), 7.30 (2 H, t, *J* = 7.1 Hz), 7.27-7.23 (1 H, m), 7.14 (2 H, d, *J* = 7.1 Hz), 4.23 (1 H, dd, *J* = 8.9, 5.1 Hz), 2.97 (1 H, dd, *J* = 13.7, 5.1 Hz), 2.81 (1 H, dd, *J* = 13.2, 8.6 Hz), 1.46 (2 H, br); HRMS (ESI-TOF) *m*/*z*: [M + H]⁺ calculated for C₁₃H₁₄⁷⁹BrN₂ and C₁₃H₁₄⁸¹BrN₂ 277.0335, 279.1671; found 277.0334, 279.0316.

### (R)-5-(5-(1-Amino-2-phenylethyl)pyridin-3-yl)-1H-benzo[d]imidazol-2(3H)-one (65a)

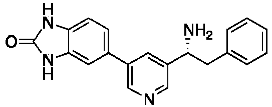

The above compound (**65a**) was prepared from **64a** and **8b** following Method E and purified by column chromatography on silica gel (MeOH/CH₂Cl₂, 10:90 to 15:85) to give **65a** (27%) as a light brown solid; **¹H NMR** (DMSO-*d₆,* 500 MHz) 10.76 (1 H, s), 10.73 (1 H, s), 8.62 (1 H, d, *J* = 2.5 Hz), 8.37 (1 H, d, *J* = 2.0 Hz), 7.92 (1 H, t, *J* = 2.0 Hz), 7.27-7.24 (3 H, m), 7.20-7.15 (4 H, m), 7.03 (1 H, d, *J* = 7.6 Hz), 4.18 (1 H, t, *J* = 7.6 Hz), 2.92 (2 H, d, *J* = 7.1 Hz); HRMS (ESI-TOF) *m*/*z*: [M + H]⁺ calculated for C₂₀H₁₉N₄O 331.1554; found 331.1586.

### (S)-5-(5-(1-Amino-2-phenylethyl)pyridin-3-yl)-1H-benzo[d]imidazol-2(3H)-one (65b)

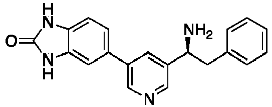

The above compound (**65b**) was prepared from **64b** and **8b** following Method E and purified by column chromatography on silica gel (MeOH/CH₂Cl₂, 5:95 to 10:90) to give **65b** (23%) as a pale yellow solid; **¹H NMR** (DMSO-*d₆,* 500 MHz) 10.74 (1 H, s), 10.71 (1 H, s), 8.61 (1 H, d, *J* = 2.0 Hz), 8.36 (1 H, d, *J* = 1.5 Hz), 7.90 (1 H, t, *J* = 2.0 Hz), 7.25-7.22 (3 H, m), 7.18-7.13 (4 H, m), 7.01 (1 H, d, *J* = 8.1 Hz), 4.18 (1 H, t, *J* = 7.1 Hz), 2.92 (2 H, d, *J* = 6.1 Hz); HRMS (ESI-TOF) *m*/*z*: [M + H]⁺ calculated for C₂₀H₁₉N₄O 331.1554; found 331.1547.

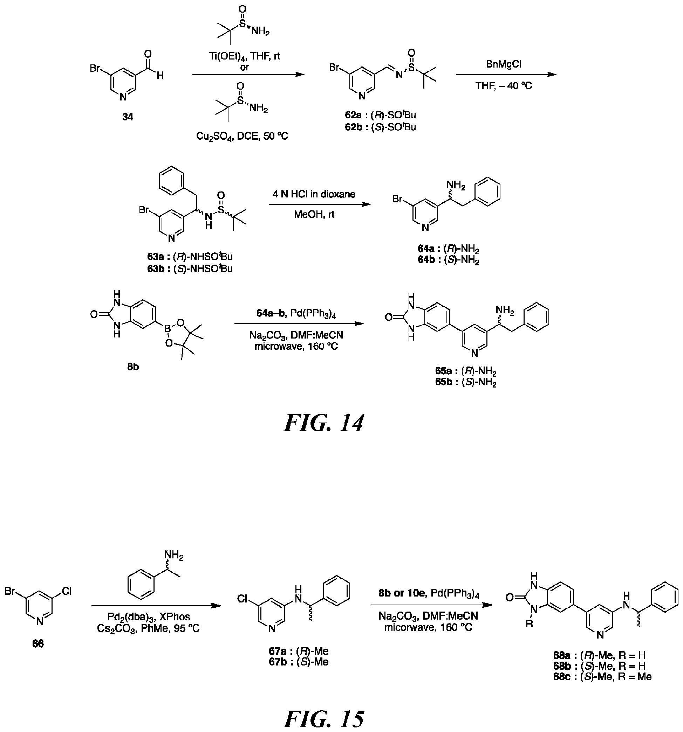

The synthetic schemes for compounds **65a-b,** discussed *supra,* are shown in Figure 14.

### Example 24 - General Procedure for the Preparation of 5-Chloro-N-(1-phenylethyl)pyridin-3-amine-Method W

### (R)-5-Chloro-N-(1-phenylethyl)pyridin-3-amine (67a)

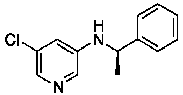

To a solution of **66** (250 mg, 1.3 mmol) in anhydrous toluene (5.0 mL) was added (R)-(+)-1-phenylethylamine (0.2 mL, 1.56 mmol), XPhos (60.6 mg, 0.13 mmol), Pd₂(dba)₃ (119 mg, 0.13 mmol) and Cs₂CO₃ (1.27 g, 3.90 mmol). The resulting mixture was purged with nitrogen and stirred at 95°C for 20 h. After being quenched with water (5 mL), the aqueous layer was extracted with EtOAc (2 × 20 mL). The combined organic extracts were washed with brine, dried over anhydrous Na₂SO₄, filtered, and concentrated. The residue was and purified by column chromatography on silica gel (EtOAc/hexane, 30:70 to 40:60) to give **67a** (61.6 mg, 20%) as an orange solid; **¹H NMR** (CDCl₃, 500 MHz) 7.85 (1 H, d, *J* = 2.5 Hz), 7.84 (1 H, d, *J* = 1.5 Hz), 7.36-7.32 (4 H, m), 7.26 (1 H, m), 6.70 (1 H, t, *J* = 2.0 Hz), 4.46 (1 H, quin, *J* = 5.6 Hz), 4.19 (1 H, br), 1.55 (3 H, d, *J* = 7.1 Hz); HRMS (ESI-TOF) *m*/*z*: [M + H]⁺ calculated for C₁₃H₁₄ClN₂ 233.0841; found 233.0944.

### (S)-5-Chloro-N-(1-phenylethyl)pyridin-3-amine (67b)

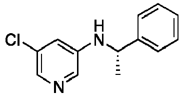

The above compound (**67b**) was prepared from **66** and (*S*)-(-)-1-phenylethylamine following Method W and purified by column chromatography on silica gel (EtOAc/hexane, 20:80 to 30:70) to give **67b** (46%) as a light brown solid; **¹H NMR** (CDCl₃, 500 MHz) 7.84 (1 H, d, *J* = 2.5 Hz), 7.83 (1 H, d, *J* = 2.0 Hz), 7.35-7.31 (4 H, m), 7.27-7.24 (1 H, m), 6.70 (1 H, t, *J* = 2.0 Hz), 4.45 (1 H, quin, *J* = 6.1 Hz), 4.26 (1 H, br), 1.54 (3 H, d, *J* = 7.1 Hz); HRMS (ESI-TOF) *m*/*z:* [M + H]⁺ calculated for C₁₃H₁₄ClN₂ 233.0841; found 233.0873.

### (R)-5-(5-((1-Phenylethyl)amino)pyridin-3-yl)-1H-benzo[d]imidazol-2(3H)-one (68a)

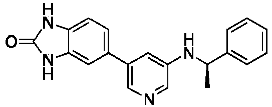

The above compound (**68a**) was prepared from **67a** and **8b** following Method E and purified by column chromatography on silica gel (MeOH/CH₂Cl₂, 5:95 to 10:90) to give **68a**(18%) as a yellow solid; ¹H NMR (DMSO-*d₆,* 500 MHz) 10.68 (1 H, s), 10.65 (1 H, s), 7.90 (1 H, s), 7.82 (1 H, d, *J* = 2.0 Hz), 7.40 (2 H, d, *J* = 7.6 Hz), 7.30 (2 H, t, *J* = 7.6 Hz), 7.18 (1 H, t, *J* = 6.6 Hz), 7.05 (1 H, t, *J* = 8.1 Hz), 6.98-6.94 (3 H, m), 6.48 (1 H, d, *J* = 7.1 Hz), 4.60 (1 H, quin, *J* = 7.1 Hz), 1.44 (3 H, d, *J* = 6.6 Hz); HRMS (ESI-TOF) *m*/*z*: [M + H]⁺ calculated for C₂₀H₁₉N₄O 331.1553; found 331.1549.

### (S)-5-(5-((1-Phenylethyl)amino)pyridin-3-yl)-1H-benzo[d]imidazol-2(3H)-one (68b)

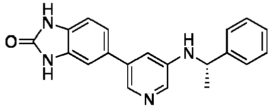

The above compound (**68b**) was prepared from **67b** and **8b** following Method E and purified by column chromatography on silica gel (MeOH/CH₂Cl₂, 5:95 to 10:90) to give **68b** (16%) as a yellow solid; ¹H NMR (DMSO-*d₆,* 500 MHz) 10.68 (1 H, s), 10.65 (1 H, s), 7.90 (1 H, s), 7.82 (1 H, d, *J* = 2.5 Hz), 7.40 (2 H, d, *J* = 7.1 Hz), 7.30 (2 H, t, *J* = 7.6 Hz), 7.18 (1 H, t, *J* = 7.1 Hz), 7.05 (1 H, t, *J* = 7.1 Hz), 6.98-6.94 (3 H, m), 6.48 (1 H, d, *J* = 7.1 Hz), 4.60 (1 H, quin, *J* = 6.6 Hz), 1.44 (3 H, d, *J* = 6.6 Hz); HRMS (ESI-TOF) *m*/*z*: [M + H]⁺ calculated for C₂₀H₁₉N₄O 331.1553; found 331.1922.

### (S)-1-Methyl-6-(5-((1-phenylethyl)amino)pyridin-3-yl)-1H-benzo[d]imidazol-2(3H)-one (68c)

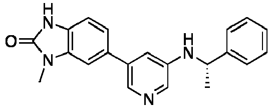

The above compound (**68c**) was prepared from **67b** and **10e** following Method E and purified by column chromatography on silica gel (MeOH/CH₂Cl₂, 5:95 to 10:90) to give **68c** (15%) as a pale yellow solid; ¹H NMR (DMSO-*d₆,* 500 MHz) 10.87 (1 H, s), 7.98 (1 H, d, *J* = 1.5 Hz), 7.85 (1 H, d, *J* = 2.0 Hz), 7.41 (2 H, d, *J* = 7.6 Hz), 7.30 (2 H, t, *J* = 7.6 Hz), 7.20-7.17 (2 H, m), 7.11 (1 H, dd, *J* = 8.6, 1.0 Hz), 7.00-6.99 (2 H, m), 6.49 (1 H, d, *J* = 7.1 Hz), 4.61 (1 H, quin, *J* = 6.6 Hz), 1.45 (3 H, d, *J* = 6.6 Hz); HRMS (ESI-TOF) *m*/*z*: [M + H]⁺ calculated for C₂₁H₂₁N₄O 345.1710; found 345.1697.

The synthetic schemes for compounds **68a-c,** discussed *supra,* are shown in Figure 15.

### Example 25 - General Procedure for the Preparation of 3-(Benzylamino)-5-(2-oxo-2,3-dihydro-1H-benzo[d]imidazol-5-yl)pyridine 1-oxide-Method X

### 3-(Benzylamino)-5-(3-methyl-2-oxo-2,3-dihydro-1H-benzo[d]imidazol-5-yl)pyridine 1-oxide (69a)

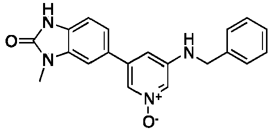

To a solution of **12c** (8.2 mg, 0.025 mmol) in 1,2-dichloroethane (1 mL) and MeOH (0.5 mL) was added *m*-CPBA (17 mg, 0.10 mmol) at 0°C. The reaction mixture was stirred at 0°C for 1 h then 80°C for 20 h. After being quenched with saturated NaS₂O₃ (1 mL), the aqueous layer was extracted with EtOAc (2 × 10 mL). The combined organic extracts were washed with brine, dried over anhydrous Na₂SO₄, filtered, and concentrated. The residue was and purified by column chromatography on silica gel (MeOH/CH₂Cl₂, 10:90 to 15:85) to give **69a** (3.2 mg, 37%) as a yellow solid; **¹H NMR** (CD₃OD, 500 MHz) 7.86 (1 H, s), 7.64 (1 H, s), 7.41-7.39 (2 H, m), 7.35 (2 H, t, *J* = 8.1 Hz), 7.28-7.25 (4 H, m), 7.13 (1 H, d, *J* = 8.1 Hz), 7.10 (1 H, s), 4.42 (2 H, s), 3.42 (3 H, s); HRMS (ESI-TOF) *m*/*z*: [M + H]⁺ calculated for C₂₀H₁₉N₄O₂ 347.1503; found 347.1513.

### 3-(Benzylamino)-5-(2-oxo-2,3-dihydro-1H-benzo[d]imidazol-5-yl)pyridine 1-oxide (69b)

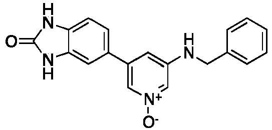

The above compound (**69b**) was prepared from **13a** following Method X and purified by column chromatography on silica gel (MeOH/CH₂Cl₂, 5:95 to 10:90) to give **69b** (31%) as a yellow solid; **¹H NMR** (CD₃OD, 500 MHz) 7.81 (1 H, s), 7.63 (1 H, s), 7.40-7.34 (4 H, m), 7.28-7.20 (3 H, m), 7.11 (1 H, d, *J* = 8.1 Hz), 7.06 (1 H, s), 4.12 (2 H, s); HRMS (ESI-TOF) *m*/*z*: [M + H]⁺ calculated for C₁₉H₁₇N₄O₂ 333.1347; found 333.1586.

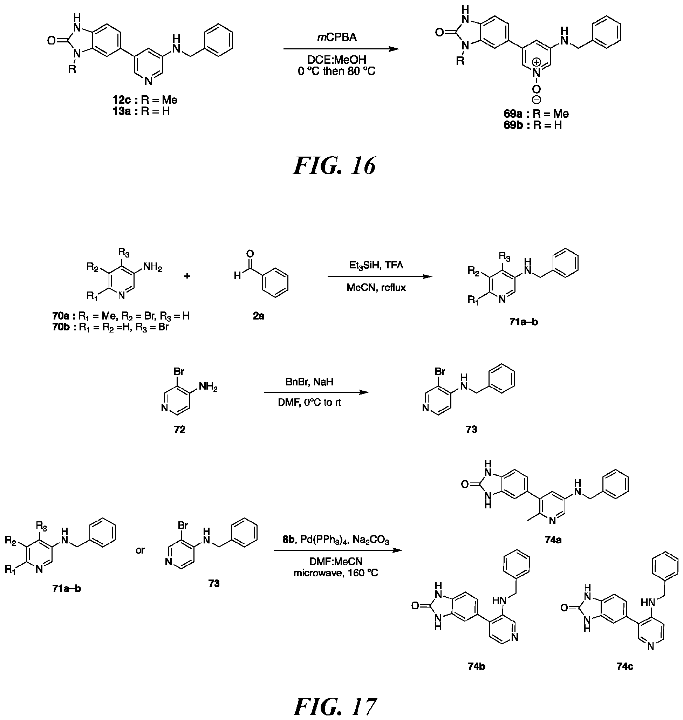

The synthetic schemes for compounds **69a-b,** discussed *supra,* are shown in Figure 16.

### N-Benzyl-5-bromo-6-methylpyridin-3-aminee (71a)

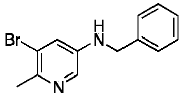

The above compound (**71a**) was prepared from **70a** and **2a** following Method B and purified by column chromatography on silica gel (MeOH/CH₂Cl₂, 5:95) to give **71a** (24%) as a pale yellow solid; **¹H NMR** (CDCl₃, 600 MHz) 7.90 (1 H, d, *J* = 2.4 Hz), 7.37-7.33 (4 H, m), 7.30 (1 H, t, *J* = 7.3 Hz), 7.08 (1 H, s), 4.30 (2 H, d, *J* = 4.9 Hz), 4.03 (1 H, br); HRMS (ESI-TOF) *m*/*z*: [M + H]⁺ calculated for C₁₃H₁₄⁷⁹BrN₂ and C₁₃H₁₄⁸¹BrN₂ 277.0335, 279.0315; found 277.0251, 279.0231.

### N-Benzyl-4-bromopyridin-3-amine (71b)

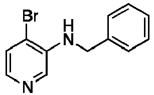

The above compound (**71b**) was prepared from **70b** and **2a** following Method B and purified by column chromatography on silica gel (MeOH/CH₂Cl₂, 5:95) to give **71b** (44%) as a pale yellow solid; **¹H NMR** (CDCl₃, 600 MHz) 7.96 (1 H, s), 7.79 (1 H, d, *J* = 4.9 Hz), 7.37-7.36 (5 H, m), 7.32-7.30 (1 H, m), 7.08 (1 H, s), 4.65 (1 H, br), 4.47 (2 H, d, *J* = 4.9 Hz); HRMS (ESI-TOF) *m*/*z*: [M + H]⁺ calculated for C₁₂H₁₂⁷⁹BrN₂ and C₁₂H₁₂⁸¹BrN₂ 263.0179, 265.0158; found 263.0160, 265.0140.

### N-Benzyl-3-bromopyridin-4-amine (73)

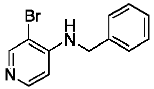

To a solution **72** (200.0 mg, 1.16 mmol) in anhydrous DMF (5 mL) was added 60% NaH (46.0 mg, 1.16 mmol) at 0°C under nitrogen and stirred for 30 min. Benzylbromide (0.14 mL, 1.16 mmol) was added. The reaction was stirred at 0°C and slowly increased to room temperature for 3 h. After being quenched by the addition of water, the aqueous layer was extracted with EtOAc (2 × 20 mL). The combined organic extracts were washed with brine, dried over anhydrous Na₂SO₄, filtered, and concentrated. The residue was purified by column chromatography on silica gel (EtOAc/hexane, 50:50 to 30:70) to afford **73** (76.6 mg, 25%) as a yellow oil; **¹H NMR** (CDCl₃, 600 MHz) 8.39 (1 H, s), 8.13 (1 H, d, *J* = 4.9 Hz), 7.39-7.37 (2 H, m), 7.33-7.30 (3 H, m), 6.47 (1 H, d, *J* = 4.9 Hz), 5.21 (1 H, br), 4.45 (2 H, d, *J* = 6.1 Hz); HRMS (ESI-TOF) *m*/*z*: [M + H]⁺ calculated for C₁₂H₁₂⁷⁹BrN₂ and C₁₂H₁₂⁸¹BrN₂ 263.0179, 265.0158; found 263.0189, 265.0168.

### 5-(5-(Benzylamino)-2-methylpyridin-3-yl)-1H-benzo[d]imidazol-2(3H)-one (74a)

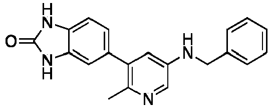

The above compound (**74a**) was prepared from **71a** and **8b** following Method E and purified by column chromatography on silica gel (MeOH/CH₂Cl₂, 5:95 to 10:90) to give **74a** (41%) as a brown solid; ¹H NMR (DMSO-*d₆,* 500 MHz) 10.64 (1 H, s), 10.62 (1 H, s), 7.83 (1 H, d, *J* = 2.4 Hz), 7.35 (2 H, d, *J* = 6.1 Hz), 7.31 (2 H, t, *J* = 7.3 Hz), 7.22 (1 H, t, *J* = 7.3 Hz), 6.93 (1 H, d, *J* = 8.5 Hz), 6.82 (1 H, d, *J* = 8.5 Hz), 6.78 (1 H, s), 6.72 (1 H, d, *J* = 2.4 Hz), 6.31 (1 H, t, *J* = 6.1 Hz), 4.29 (2 H, d, *J* = 6.1 Hz), 2.21 (3 H, s); HRMS (ESI-TOF) *m*/*z*: [M + H]⁺ calculated for C₂₀H₁₉N₄O 331.1554; found 331.1514.

### 5-(3-(Benzylamino)pyridin-4-yl)-1H-benzo[d]imidazol-2(3H)-one (74b)

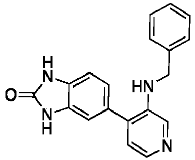

The above compound (**74b**) was prepared from **71b** and **8b** following Method E and purified by column chromatography on silica gel (MeOH/CH₂Cl₂, 5:95 to 10:90) to give **74b** (42%) as a brown solid; ¹H NMR (DMSO-*d₆,* 500 MHz) 10.73 (2 H, s), 7.81 (1 H, d, *J* = 4.9 Hz), 7.78 (1 H, s), 7.34-7.30 (4 H, m), 7.21 (1 H, t, *J* = 6.1 Hz), 7.06-7.02 (3 H, m), 6.95 (1 H, d, *J* = 4.9 Hz), 5.67 (1 H, t, *J* = 6.1 Hz), 4.34 (2 H, d, *J* = 6.1 Hz); HRMS (ESI-TOF) *m*/*z*: [M + H]⁺ calculated for C₁₉H₁₇N₄O 317.1397; found 317.1391.

### 5-(4-(Benzylamino)pyridin-3-yl)-1H-benzo[d]imidazol-2(3H)-one (74c)

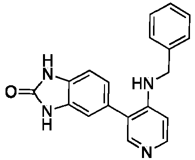

The above compound (**74c**) was prepared from **73** and **8b** following Method E and purified by column chromatography on silica gel (1 N NH₃ in MeOH/CH₂Cl₂, 15:85) to give **74c** (36%) as a brown solid; ¹H NMR (DMSO-*d₆,* 500 MHz) 10.67 (2 H, s), 7.96 (1 H, d, *J* = 4.9 Hz), 7.87 (1 H, s), 7.32-7.29 (4 H, m), 7.20 (1 H, t, *J* = 6.1 Hz), 7.04 (1 H, d, *J* = 7.3 Hz), 6.96-6.95 (2 H, m), 6.38 (1 H, d, *J* = 4.9 Hz), 6.27 (1 H, t, *J* = 6.1 Hz), 4.33 (2 H, d, *J* = 6.1 Hz); HRMS (ESI-TOF) *m*/*z*: [M + H]⁺ calculated for C₁₉H₁₇N₄O 317.1397; found 317.1391.

The synthetic schemes for compounds **74a-c,** discussed *supra,* are shown in Figure 17.

### Example 26 - DYRK1A Binding Assays/IC50 Assay

Compounds were tested for DYRK1A binding activity at DiscoverX. DiscoverX uses proprietary KINOMEscan^{®} Assay (Fabian et al., "A Small Molecule-kinase Interaction Map for Clinical Kinase Inhibitors," Nat. Biotechnol. 23(3):329-336 (2005)). Compounds were screened for DYRK1A activity at a single concentration of 3 µM in duplicates. Similarly, the dissociation constant K_{d} of the hit compounds from the initial screening was determined at DiscoverX using their proprietary KINOMEscan^{®} Assay. K_{d} values are determined using eleven serial three fold dilutions with the highest concentration of 60 µM. The results of the binding assay are displayed in Table 1.

Some compounds were also tested for IC50 (nM) activity using the DYRK1/DYRK1A assay from Reaction Biology Corp., the results of which are also displayed in Table 1.

**Table 1. DYRK1A Binding Activity**

| ***Compound*** | ***STRUCTURE*** | ***Kd (nM)*** | ***IC50 (nM)*** |
|---|---|---|---|
| **11a** | 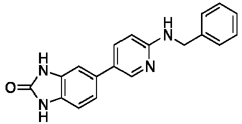 | 300 | - |
| **11b** | 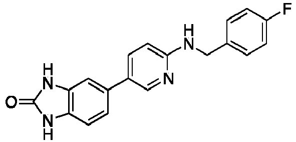 | 580, 510 | - |
| **11c** | 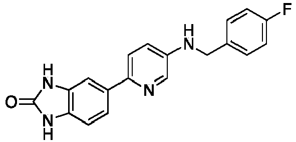 | 6000 | - |
| **12b** | 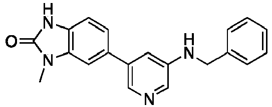 | 0.82, 1.6 | - |
| **12c** | 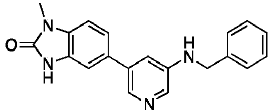 | 2000 | - |
| **13a** | 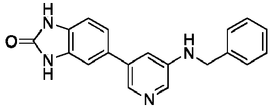 | 2.3, 2.9, 1.1 | - |
| **13b** | 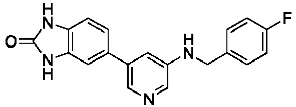 | 2.9, 1.8 | - |
| **13c** | 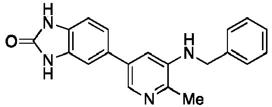 | 140 | - |
| **13d** | 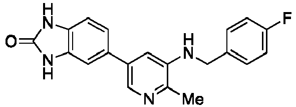 | 240 | - |
| **13e** | 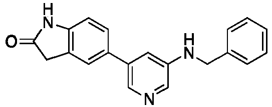 | 1.3, 1.9 | - |
| **30a** | 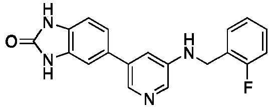 | 1.2 | - |
| **30b** | 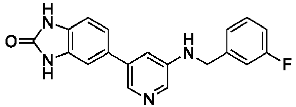 | 1.2 | - |
| **30c** | 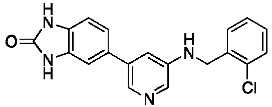 | 1.3 | - |
| **30d** | 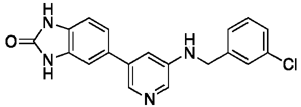 | 1.3 | - |
| **30e** | 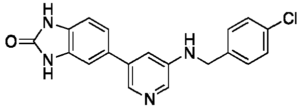 | 23 | - |
| **30f** | 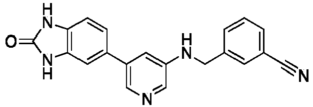 | 5.8 | - |
| **30g** | 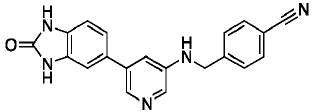 | 31 | - |
| **30h** | 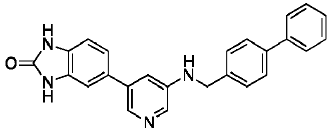 | 1200 | - |
| **30i** | 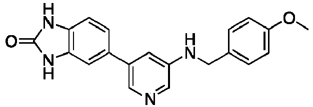 | TBD | - |
| **30j** | 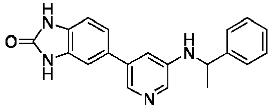 | 0.71, 1.1 | - |
| **30k** | 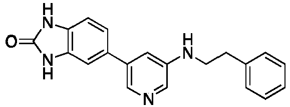 | 4.3 | - |
| **30l** | 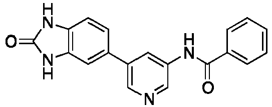 | TBD | - |
| **30m** | 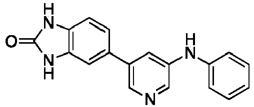 | TBD | - |
| **30n** | 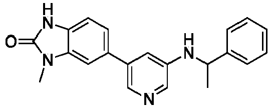 | 0.81 | - |
| **30o** | 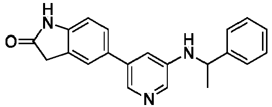 | 0.81 | - |
| **30p** | 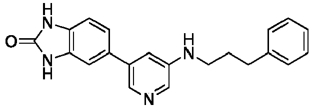 | 15 | 1.83 |
| **30q** | 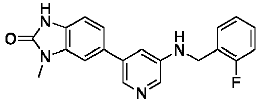 | 0.86 | 0.3 |
| **30r** | 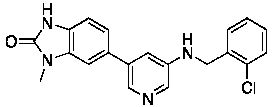 | 1.3 | 0.1 |
| **31a** | 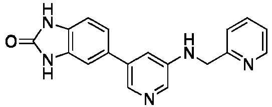 | 1.2 | - |
| **31b** | 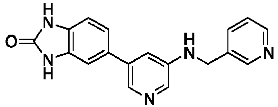 | 2.1 | - |
| **31c** | 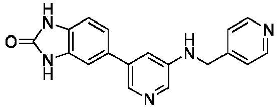 | 3.9 | - |
| **31d** | 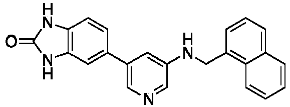 | 28 | - |
| **31e** | 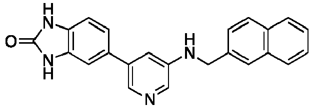 | 100 | - |
| **31f** | 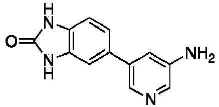 | TBD | - |
| **33a** | 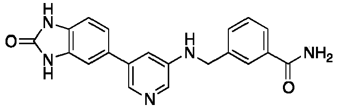 | 9.9 | - |
| **33b** | 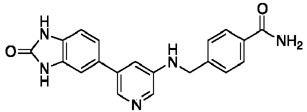 | 14 | - |
| **39a** | 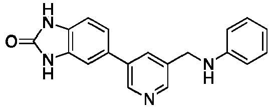 | TBD | - |
| **39b** | 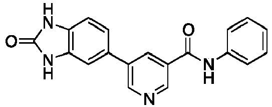 | TBD | - |
| **45a** | 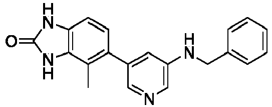 | TBD | - |
| **45b** | 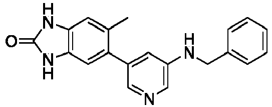 | TBD | - |
| **46a** | 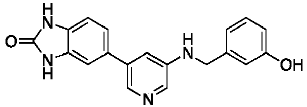 | TBD | - |
| **46b** | 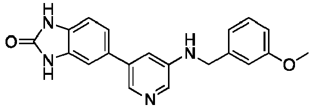 | TBD | - |
| **46c** | 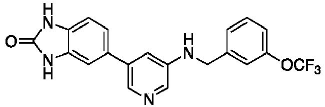 | TBD | - |
| **49a** | 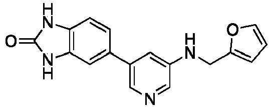 | - | 1.74 |
| **49b** | 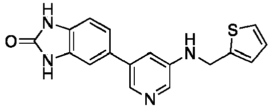 | - | 0.48 |
| **49c** | 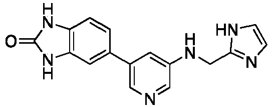 | - | 4.91 |
| **49d** | 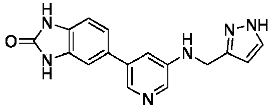 | - | 15.2 |
| **49e** | 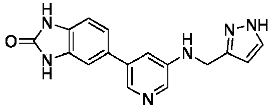 | - | 3.27 |
| **49f** | 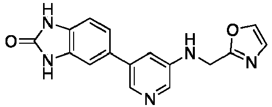 | - | 4.03 |
| **52a** | 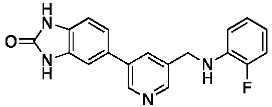 | - | 3.67 |
| **52b** | 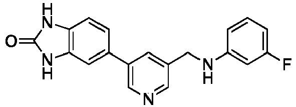 | - | 1.31 |
| **52c** | 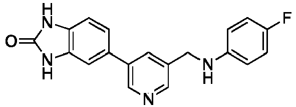 | - | 6.11 |
| **52d** | 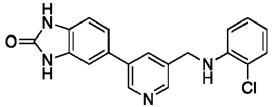 | - | 5.05 |
| **52f** | 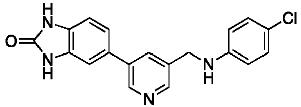 | - | TBD |
| **52g** | 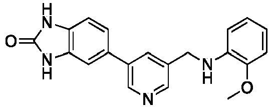 | - | 2.72 |
| **52h** | 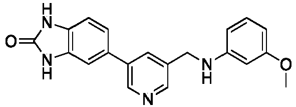 | - | 2.26 |
| **52i** | 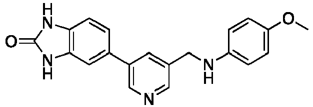 | - | 2.2 |
| **61a** | 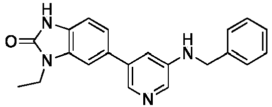 | - | 1.41 |
| **61b** | 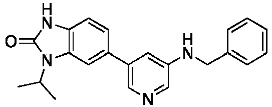 | - | 13.8 |
| **61c** | 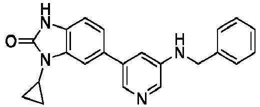 | - | 3.25 |
| **61d** | 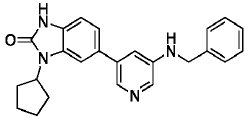 | - | 24 |
| **61e** | 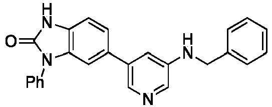 | - | 3.8 |
| **61f** | 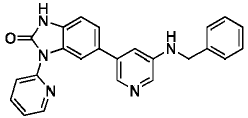 | - | 0.52 |
| **61g** | 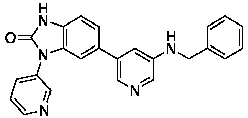 | - | 6.47 |
| **61j** | 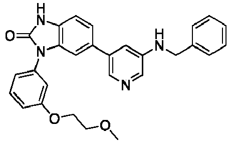 | - | 3.47 |
| **61k** | 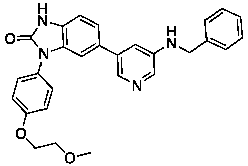 | - | 38 |
| **61l** | 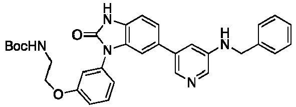 | - | 103 |
| **61m** | 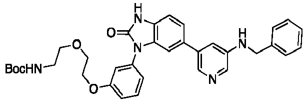 | - | 42.8 |
| **61n** | 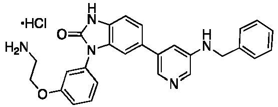 | - | 7.12 |
| **61o** | 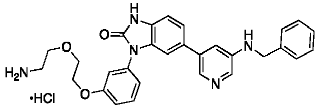 | - | 3.9 |
| **65a** | 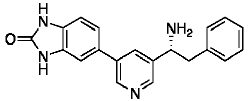 | 3.2 | 3.98 |
| **65b** | 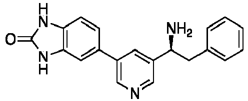 | 15 | 23.2 |
| **68a** | 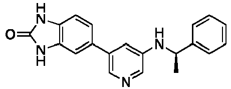 | 0.59 | 0.21 |
| **68b** | 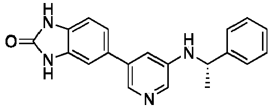 | 7.5 | 2.18 |
| **69c** | 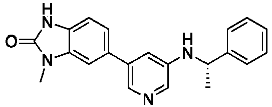 | - | 0.286 |
| **69a** | 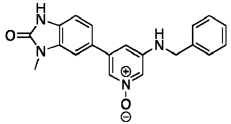 | - | 99.9 |
| **69b** | 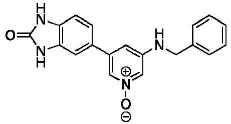 | - | 45.2 |
| 74a | 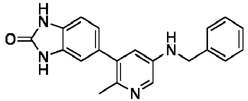 | - | 2100 |
| **74b** | 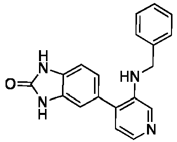 | - | 8580 |
| **74c** | 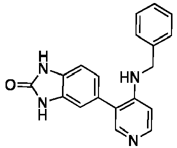 | - | 1690 |

| | | | |
|---|---|---|---|
| *TBD: To be determined* | | | |

## Claims

1. A compound of formula (I) having the following structure: 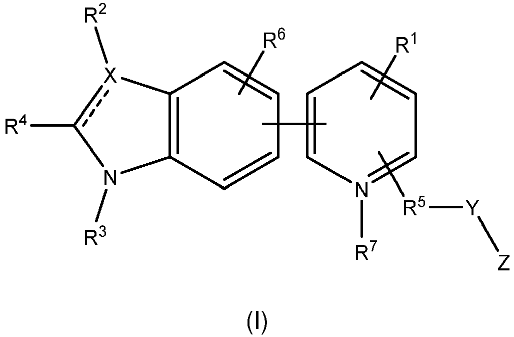 or a stereoisomer, pharmaceutically acceptable salt, oxide, or solvate thereof, wherein
R¹ and R⁶ are independently optionally present, and when present, each is independently a substituted or unsubstituted C₁-C₆ alkyl, halogen, -CF₃, or -OCF₃;
R² is H;
R³ is H;
R⁴ is an oxygen that forms a carbonyl;
R⁵ is NH;
R⁷ is optionally present, and when present is an oxygen forming a pyridine N-oxide;
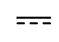 is a single bond;
X is N;
Y is selected from a bond, CH₂, CH(CH₃), CH₂CH₂, CH₂CH(CH₃), and CH(CH₃)CH₂; and
Z is an unsubstituted phenyl ring or a phenyl ring substituted with a hydroxyl, -OCF₃, halogen, a nitrile, a benzene ring, C₁-C₆ alkoxy, or -CONH₂.

2. The compound according to claim 1, wherein
Y is CH₂; and
Z is a phenyl ring.

3. The compound according to claim 1, selected from the group consisting of

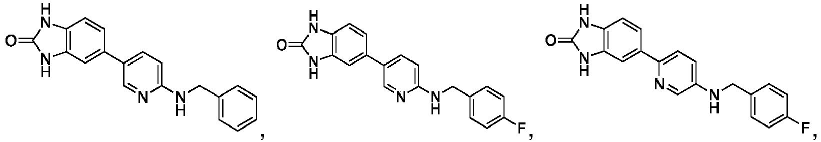

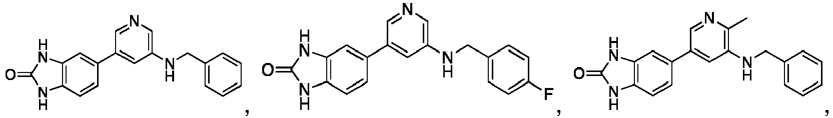

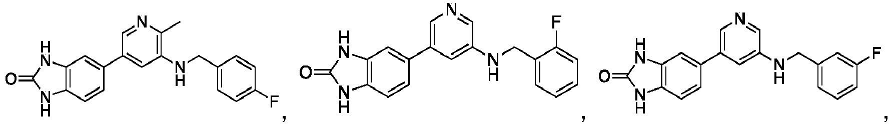

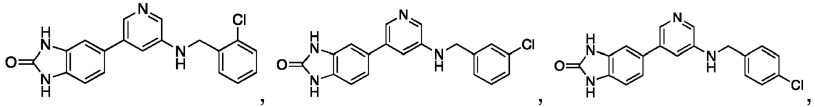

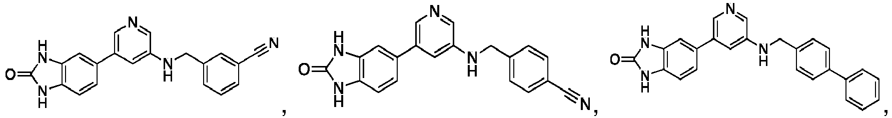

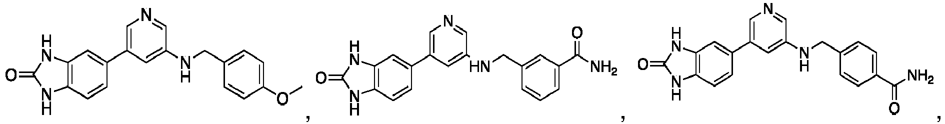

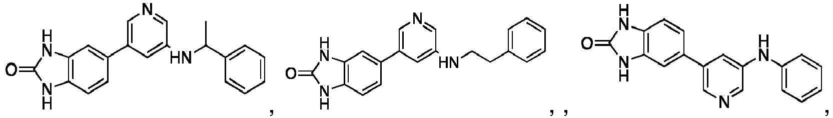

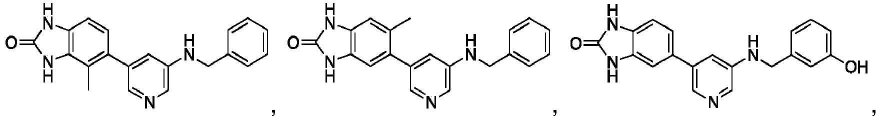

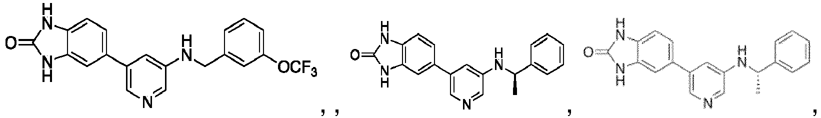

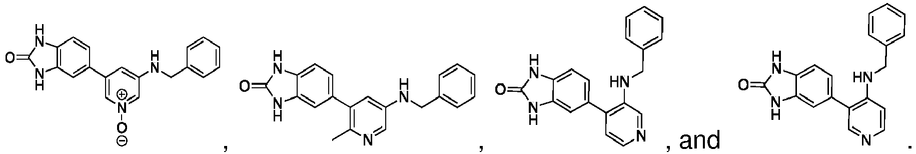

4. A composition comprising:
a compound according to claim 1 and
a carrier.

5. The composition according to claim 4 further comprising:
a) a transforming growth factor beta (TGFβ) superfamily signaling pathway inhibitor; and/or
b) a glucagon-like peptide-1 receptor (GLP1R) agonist, a Dipeptidyl Peptidase IV (DDP4) inhibitor, or a combination thereof.

6. A compound of claim 1 for use in the treatment of a condition associated with insufficient insulin secretion, said treatment comprising:
administering to a subject in need of treatment for a condition associated with an insufficient level of insulin secretion a compound of claim 1 under conditions effective to treat the subject for the condition.

7. The compound for use as claimed in claim 6, wherein the subject in need of treatment has been diagnosed as having one or more of type I diabetes (T1D), type II diabetes (T2D), gestational diabetes, congenital diabetes, maturity onset diabetes (MODY), cystic fibrosis-related diabetes, hemochromatosis-related diabetes, drug-induced diabetes, monogenic diabetes, metabolic syndrome or insulin resistance or wherein the subject has had a pancreatectomy, pancreas transplantation, or pancreatic islet transplantation.

8. A compound according to claim 1 for use in the treatment of a neurological disorder, said treatment comprising:
administering to a subject in need of treatment for a neurological disorder a compound of claim 1 under conditions effective to treat the subject for the condition.

9. The compound for use as claimed in claim 6 or claim 8, wherein said treatment further comprises:
a) administering a transforming growth factor beta (TGFβ) superfamily signaling pathway inhibitor; and/or
b) administering a glucagon-like peptide-1 receptor (GLP1R) agonist, a Dipeptidyl Peptidase IV (DDP4) inhibitor, or a combination thereof.

10. The compound for use as claimed in any one of claims 8-9, wherein the subject has been diagnosed as having one or more of diabetes, Down's Syndrome, or a neurodegenerative disease.

## Patentansprüche

1. Verbindung der Formel (I) mit der folgenden Struktur: 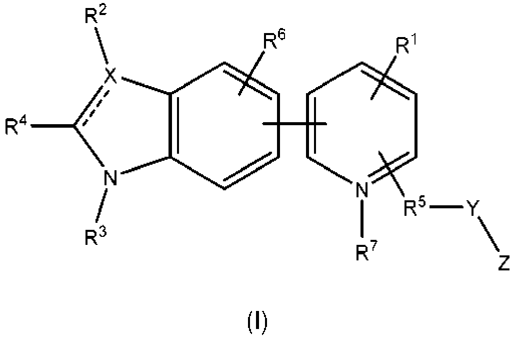 oder ein Stereoisomer, pharmazeutisch verträgliches Salz, Oxid oder Solvat davon, wobei
R¹ und R⁶ unabhängig optional vorhanden sind und, wenn vorhanden, jeweils unabhängig ein substituiertes oder unsubstituiertes C₁-C₆-Alkyl, Halogen, -CF₃ oder -OCF₃ sind;
R² H ist;
R³ H ist;
R⁴ ein Sauerstoff ist, der ein Carbonyl bildet;
R⁵ NH ist;
R⁷ optional vorhanden ist und, wenn vorhanden, ein Sauerstoff ist, der ein Pyridin-N-oxid bildet;
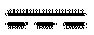 eine Einfachbindung ist;
X N ist;
Y aus einer Bindung, CH₂, CH(CH₃), CH₂CH₂, CH₂CH(CH₃) und CH(CH₃)CH₂ ausgewählt ist; und
Z ein unsubstituierter Phenylring oder ein Phenylring ist, der mit einem Hydroxyl, -OCF₃, Halogen, einem Nitril, einem Benzolring, C₁-C₆-Alkoxy oder -CONH₂ substituiert ist.

2. Verbindung nach Anspruch 1, wobei
Y CH₂ ist; und
Z ein Phenylring ist.

3. Verbindung nach Anspruch 1, ausgewählt aus der Gruppe bestehend aus

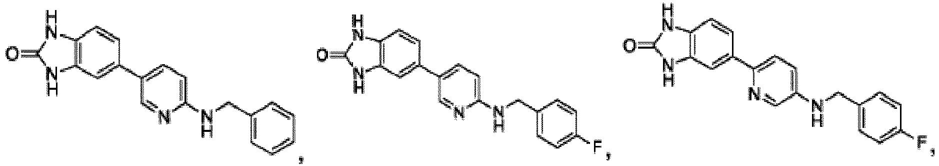

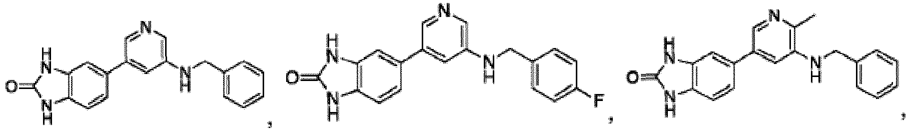

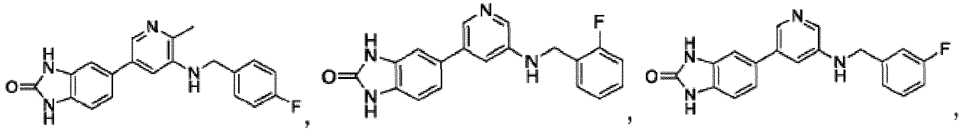

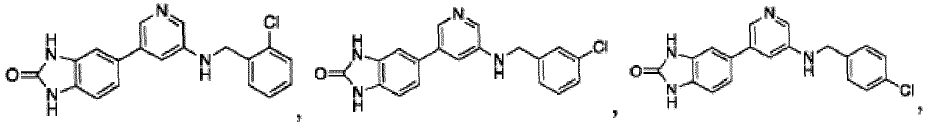

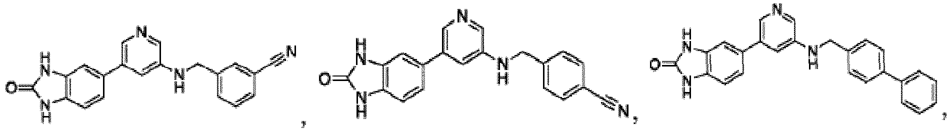

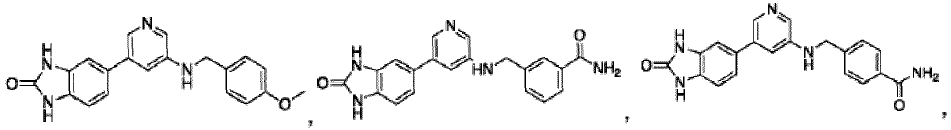

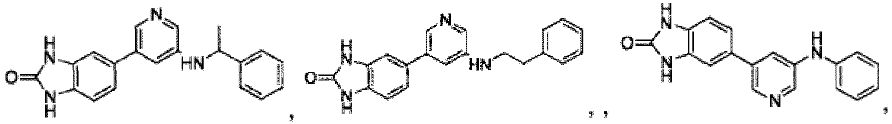

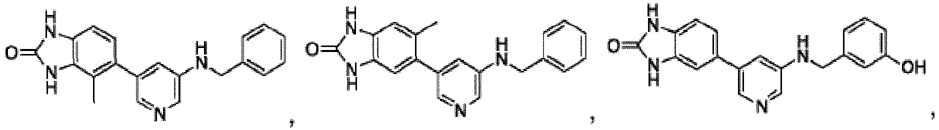

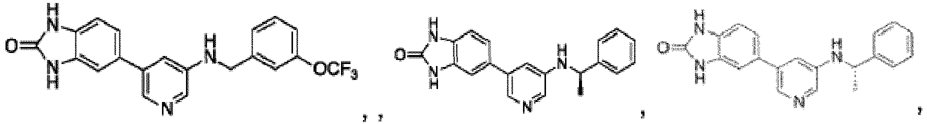

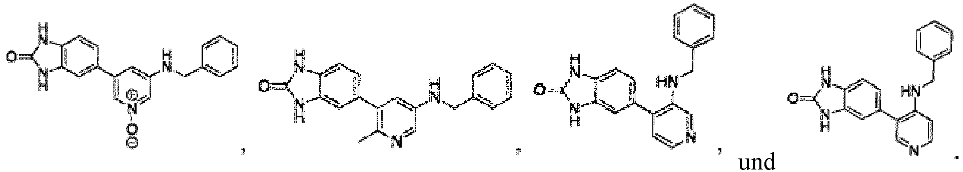

4. Zusammensetzung, umfassend:
eine Verbindung nach Anspruch 1 und
einen Träger.

5. Zusammensetzung nach Anspruch 4, ferner umfassend:
a) einen Signalweginhibitor der Transforming Growth Factor Beta(TGF β)-Superfamilie; und/oder
b) einen Glucagon-ähnlicher Peptid-1-Rezeptor(GLP1R)-Agonisten, einen Dipeptidylpeptidase IV(DDP4)-Inhibitor oder eine Kombination davon.

6. Verbindung nach Anspruch 1 zur Verwendung bei der Behandlung eines Zustands, der mit unzureichender Insulinsekretion einhergeht, wobei die Behandlung Folgendes umfasst:
Verabreichen einer Verbindung nach Anspruch 1 an ein Subjekt, das einer Behandlung für einen Zustand bedarf, der mit einer unzureichenden Insulinsekretion einhergeht, unter Bedingungen, die zum Behandeln des Subjekts für den Zustand wirksam sind.

7. Verbindung zur Verwendung nach Anspruch 6, wobei bei dem Subjekt, das einer Behandlung bedarf, eines oder mehrere von Diabetes Typ I (T1D), Diabetes Typ II (T2D), Schwangerschaftsdiabetes, angeborener Diabetes und Erwachsenendiabetes (MODY), Diabetes bei Mukoviszidose, Hämochromatose-bedingter Diabetes, medikamenteninduzierter Diabetes, monogener Diabetes, metabolisches Syndrom oder Insulinresistenz diagnostiziert wurde oder wobei sich das Subjekt einer Pankreatektomie, Pankreastransplantation oder Pankreasinseltransplantation unterzogen hat.

8. Verbindung nach Anspruch 1 zur Verwendung bei der Behandlung einer neurologischen Störung, wobei die Behandlung Folgendes umfasst:
Verabreichen einer Verbindung nach Anspruch 1 an ein Subjekt, das einer Behandlung einer neurologischen Störung bedarf, unter Bedingungen, die zum Behandeln des Subjekts für den Zustand wirksam sind.

9. Verbindung zur Verwendung nach Anspruch 6 oder Anspruch 8, wobei die Behandlung ferner Folgendes umfasst:
a) Verabreichen eines Signalweginhibitors der Transforming Growth Factor Beta(TGF β)-Superfamilie, und/oder
b) Verabreichen eines Glucagon-ähnlicher Peptid-1-Rezeptor(GLP1R)-Agonisten, eines Dipeptidylpeptidase IV(DDP4)-Inhibitors oder einer Kombination davon.

10. Verbindung zur Verwendung nach einem der Ansprüche 8 bis 9, wobei bei dem Subjekt eines oder mehrere von Diabetes, Down-Syndrom oder einer neurodegenerativen Erkrankung diagnostiziert wurden.

## Revendications

1. Composé de formule (I) comportant la structure suivante : 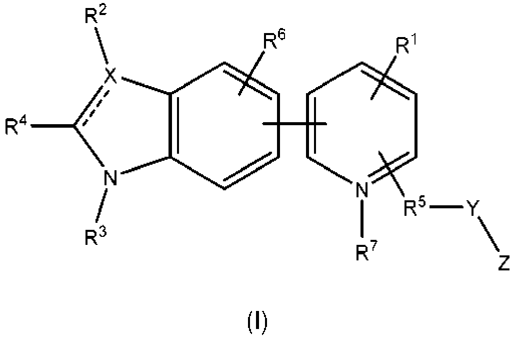 ou un stéréoisomère, un sel pharmaceutiquement acceptable, un oxyde ou un solvant de celui-ci, dans lequel
R¹ et R⁶ sont indépendamment éventuellement présents, et lorsqu'ils sont présent, chacun représente indépendamment un alkyle en C₁-C₆ substitué ou non substitué, halogène, -CF₃, ou -OCF₃ ;
R² représente H ;
R³ représente H ;
R⁴ représente un oxygène qui forme un carbonyle ;
R⁵ représente NH ;
R⁷ est éventuellement présent, et lorsqu'il est présent, représente un oxygène formant un N-oxyde de pyridine ;
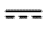 représente une liaison simple ;
X représente N ;
Y est choisi parmi une liaison, CH₂, CH(CH₃), CH₂CH₂, CH₂CH(CH₃), et CH(CH₃)CH₂ ; et
Z représente un cycle phényle non substitué ou un cycle phényle substitué par un hydroxyle, -OCF₃, halogène, un nitrile, un cycle benzénique, alcoxy en C₁-C₆, ou -CONH₂.

2. Composé selon la revendication 1, dans lequel
Y représente CH₂; et
Z représente un cycle phényle.

3. Composé selon la revendication 1, choisi dans le groupe constitué par

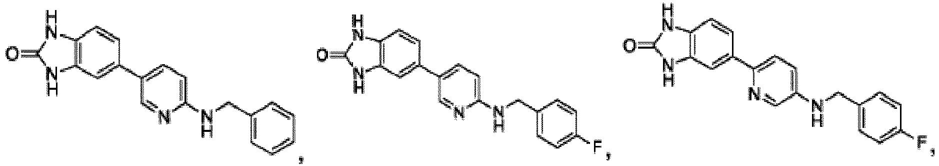

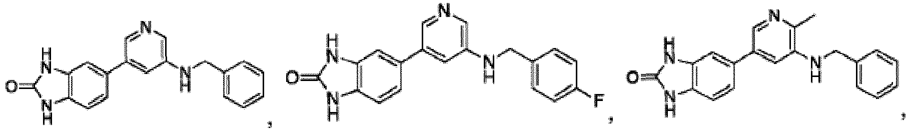

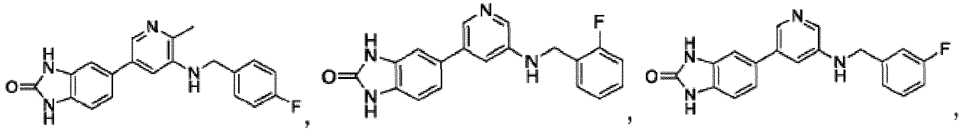

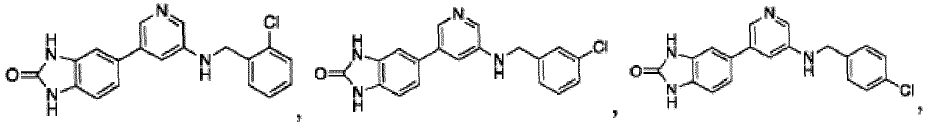

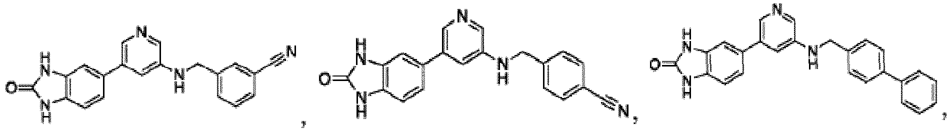

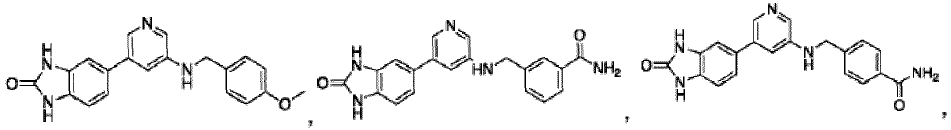

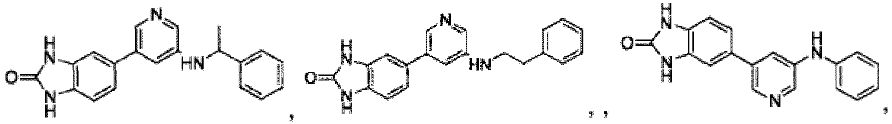

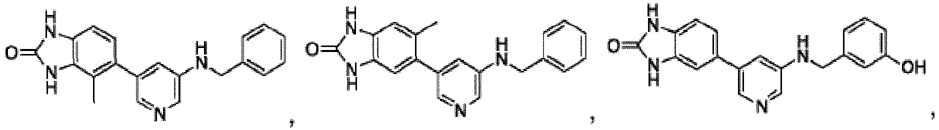

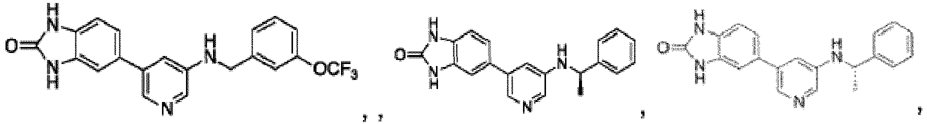

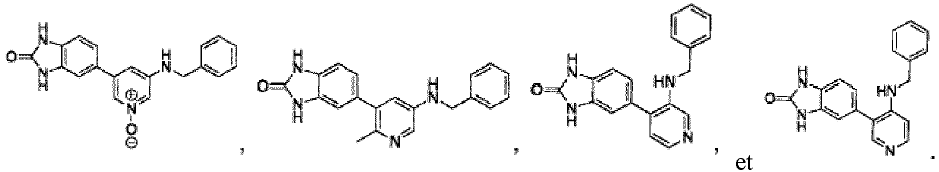

4. Composition comprenant :
un composé selon la revendication 1 et
un véhicule.

5. Composition selon la revendication 4 comprenant en outre :
a) un inhibiteur de la voie de signalisation de la superfamille du facteur de croissance transformant bêta (TGFβ) ; et/ou
b) un agoniste du récepteur du peptide-1 de type glucagon (GLP1R), un inhibiteur de la Dipeptidyl Peptidase IV (DDP4) ou une combinaison de ceux-ci.

6. Composé selon la revendication 1 destiné à être utilisé dans le traitement d'une condition associée à une sécrétion insuffisante d'insuline, ledit traitement comprenant :
l'administration à un sujet nécessitant un traitement pour une condition associée à un niveau insuffisant de sécrétion d'insuline d'un composé selon la revendication 1 dans des conditions efficaces pour traiter le sujet pour la condition.

7. Composé destiné à être utilisé selon la revendication 6, dans lequel on a diagnostiqué chez le sujet nécessitant un traitement un ou plusieurs des types suivants : le diabète de type I (T1D), le diabète de type II (T2D), le diabète gestationnel, le diabète congénital, le diabète de la maturité (MODY), le diabète lié à la fibrose kystique, le diabète lié à l'hémochromatose, le diabète induit par les médicaments, le diabète monogénique, le syndrome métabolique ou la résistance à l'insuline, ou dans lequel le sujet a subi une pancréatectomie, une transplantation de pancréas ou une transplantation d'îlots de Langerhans pancréatiques.

8. Composé selon la revendication 1 destiné à être utilisé dans le traitement d'un trouble neurologique, ledit traitement comprenant :
l'administration à un sujet nécessitant un traitement pour un trouble neurologique d'un composé selon la revendication 1 dans des conditions permettant de traiter le sujet pour ce trouble.

9. Composé destiné à être utilisé selon la revendication 6 ou la revendication 8, dans lequel ledit traitement comprend en outre :
a) l'administration d'un inhibiteur de la voie de signalisation de la superfamille du facteur de croissance transformant bêta (TGFβ) ; et/ou
b) l'administration d'un agoniste du récepteur du peptide-1 de type glucagon (GLP1R), d'un inhibiteur de la Dipeptidyl Peptidase IV (DDP4) ou d'une combinaison de ceux-ci.

10. Composé destiné à être utilisé selon l'une quelconque des revendications 8 à 9, dans lequel le sujet a été diagnostiqué comme étant atteint d'un ou de plusieurs parmi le diabète, le syndrome de Down ou une maladie neurodégénérative.
